# EUROPEAN PATENT APPLICATION

(11) **EP 4 628 151 A2**
(43) Date of publication of application: **08.10.2025**
(21) Application number: 25181864.7
(22) Date of filing: 31.08.2021
(51) Int. Cl.: A61N 1/05

(54) **DEVICE FOR THERAPEUTIC SINO-NASAL TREATMENT**

(30) Priority: 31.08.2020 US 202063072352 P; 05.05.2021 US 202163184377 P; 05.05.2021 US 202163184373 P; 05.05.2021 US 202163184383 P
(62) Divisional of application: 21807235.3
(71) Applicant: Neurent Medical Limited, Oranmore, Galway (IE)
(72) Inventor: TOWNLEY, David, County Clare (IE); MCLAUGHLIN, Cathal, Galway (IE); DE GANTE, Sofia Iniguez, Galway (IE); HORAN, Steven, Galway (IE)
(74) Representative: HGF

(57) **Abstract**

The invention generally relates to systems and methods for targeting of specific tissue(s) of interest in a sino-nasal region of a patient for the treatment of a rhinosinusitis condition. A device of the present invention includes an end effector including one or more flexible printed circuit board (PCB) members for delivering energy to one or more target sites within the sino-nasal cavity of the patient while minimizing or avoiding collateral damage to surrounding or adjacent non-targeted tissue, such as blood vessels, bone, and non-targeted neural tissue.

## Description

### Field of the Invention

The invention generally relates systems for treating medical conditions, and, more particularly, to a device for the treatment of a rhinosinusitis condition.

### Background

Rhinitis is an inflammatory disease of the nose and is reported to affect up to 40% of the population. It is the fifth most common chronic disease in the United States. The most common and impactful symptoms of rhinitis are congestion and rhinorrhea. Allergic rhinitis accounts for up to 65% of all rhinitis patients. Allergic rhinitis is an immune response to an exposure to allergens, such as airborne plant pollens, pet dander or dust. Non-allergic rhinitis is the occurrence of common rhinitis symptoms of congestion and rhinorrhea. As non-allergic rhinitis is not an immune response, its symptoms are not normally seasonal and are often more persistent. The symptoms of rhinitis include a runny nose, sneezing, and sino-nasal itching and congestion.

Current treatment devices generally provide therapeutic treatment using various modalities, such as cryotherapeutic cooling, ultrasound energy (e.g., high intensity focused ultrasound ("HIFU") energy), microwave energy (e.g., via a microwave antenna), direct heating, high and/or low power laser energy, mechanical vibration, and/or optical power. Delivery of such therapeutic treatment is provided via one or more discrete elements (e.g., electrodes, transducers, etc.) provided on some form of an end effector.

### Summary

The invention is a new and unique end effector that takes advantage of the benefits of flexible printed circuit boards (PCBs), as well as various manufacturing techniques, to provide an improved device for the treatment of a rhinosinusitis condition. In particular, the end effector includes one or more retractable and expandable segments, each of which is comprised of a framework of support elements having elastic properties. Each retractable and expandable segment further includes one or more flexible printed circuit board (PCB) members provided thereon. The flexible PCB members are composed of a flexible material capable of moving (e.g., bending, twisting, folding, etc.) between various positions in correspondence with movement of the underlying retractable and expandable segment to which it is attached. Each flexible PCB member further includes one or more energy delivering elements (e.g., electrodes) provided thereon and configured to deliver energy to tissue associated with one or more target sites in the sino-nasal cavity. Once delivered within the sino-nasal cavity, the one or more segments can expand to a specific shape and/or size corresponding to anatomical structures within the sino-nasal cavity and associated with target sites to undergo delivery of therapeutic energy for treatment of a condition (i.e., rhinosinusitis or the like). As such, once deployed, the flexible PCBs of the first and second segments contact and conform to a shape of the respective locations, including conforming to and complementing shapes of the one or more anatomical structures, thereby accurately positioning the electrodes for focused application of energy to targeted tissue at the one or more target sites.

The present invention utilizes the many benefits of flexible PCBs, as well as certain manufacturing techniques, to provide an end effector that is capable of highly conforming to anatomical variations within a sino-nasal cavity so that an operator can perform an accurate, minimally invasive, and localized application of energy to one or more target sites within the sino-nasal cavity of the patient to thereby treat a sino-nasal condition.

In particular, the underlying design of the end effector is unique. In a preferred embodiment, the end effector is multi-segmented and includes a proximal segment and a distal segment. The proximal and distal segments are constructed from single, unitary work pieces having elastic properties. More specifically, a single piece of shape memory material, such as nitinol, may be used to construct one or more portions of the proximal segment and further construct the distal segment in its entirety. For example, in one embodiment, the proximal segment is composed of a pair of interlocking members, while the distal segment is composed of a single member. The pair of interlocking members of the proximal segment include a first member providing a first set of support elements and a second member providing a second set of support members. As such, each of the first member and the second member may be constructed from a single workpiece and subsequently interlocked within one another to form the proximal segment, while the distal segment comprises a single component (as opposed to interlocking components), and is thus formed from a single workpiece.

The single workpiece may initially be in the form of a tube or a flat plate and can be laser cut to form the desired framework of support elements of the proximal and distal segments. In addition to reducing time, cost, and complexity, the use of laser machining allows a greater amount of design freedom for the manufacturer, in turn leading to a more tailored geometry and mechanical properties of a given segment of the end effector. For example, laser machining allows greater control over mechanical properties of the support elements, including tailoring the stiffness of a specific one of, or a given group of, support elements for a given segment, thereby allowing for tailoring of the tissue apposition profile when the given segment is in an expanded, deployed configuration. Furthermore, utilizing a stock workpiece in the form of a tube or flat plate results in support elements having a relatively flat surface upon which a corresponding flexible PCB member is affixed, thereby improving apposition of the PCB member to tissue within the sino-nasal cavity.

Furthermore, the use of flexible PCB members results in a greater amount of usable surface area than what is otherwise available with existing end effectors. The increase in surface area allows for a greater number of energy delivery elements to be introduced and utilized in a given procedure and further expands the possible number of patterns of such energy delivery elements. As a result, the contact surface increases substantially, thereby allowing for the end effector of the present invention to deliver treatment to certain areas within the sino-nasal cavity that may have been previously unreachable or untreatable with current treatment devices, or that previously required a surgeon to reposition a given device to reach such areas. The use of flexible PCB members also reduces the overall complexity with regard to manufacturing the end effector of the present invention. In particular, any given flexible PCB member (including an overall PCB assembly, which includes multiple PCB members) is constructed separately from the end effector, which includes constructing the overall electrode design and placement on a given PCB member. Once a PCB assembly is complete, PCB members are then attached to respective portions of a given segment of the end effector as a separate manufacturing step, thereby reducing the complexity that is otherwise associated with placing electrodes directly on the end effector, which is a common practice.

The invention provides improved manufacturing techniques for bonding the PCB members to support elements of a given proximal or distal segment. In particular, the present invention contemplates the use of bonding, thermal, and mechanical processes for joining PCB members to respective support elements, which may include one or more of adhesion, mechanical, lamination, polymer reflow, induction heating, spot welding, and laser welding processes. For example, in one embodiment, attaching a PCB member to a respective support element includes a reflow process in which a flexible PCB member is positioned relative to a respective support element, one or more polymer layers are then disposed around the flexible PCB member, and heat is then applied resulting in the one or more polymer layers encasing the flexible PCB member and subsequently affixing the flexible PCB member to the underlying support element of the end effector segment. In another embodiment, polymer sleeves may be secured to the support elements, thereby providing a substrate upon which a flexible PCB member may be positioned and subsequently attached. It should be noted that the process of securing the polymer sleeve and bonding the flexible PCB member to the support element may occur simultaneously via the application of pressure and heat.

In some embodiments, one or more of the support elements of the underlying proximal and distal segments of the end effector may further include fixation points that facilitate the attachment and alignment of the polymer sleeve and/or flexible PCB member thereto. For example, one or more of the support elements may include a recess, hole, notch, groove, etching, or the like to thereby increase surface area to receive an adhesive or pooling of melted polymer and mechanically secure the sleeve and/or PCB member in place ensuring alignment and simplifying assembly.

Accordingly, the end effector of the present invention addresses drawbacks of current treatment devices. In particular, the invention recognizes that current treatment devices may have limitations regarding the delivery of therapeutic energy to tissues of interest, as the delivery of energy may be limited by the architecture of a given end effector. More specifically, many current devices include an end effector having a number of energy delivering elements discretely placed along portions thereof, which results in a surgeon having to reposition the end effector and deliver energy multiple times to a single target site to ensure that the tissue of interest receives adequate treatment. Accordingly, such devices may result in incomplete treatment of the targeted tissue and/or collateral damage to surrounding tissue, organs, bone, or the like.

One aspect of the present invention provides a device for treating a condition in a sino-nasal cavity. The device includes an end effector dimensioned for insertion into a sino-nasal cavity, the end effector comprising at least one segment that is a unitary single piece of material comprising a plurality of individual struts. The device further includes a flexible printed circuit board (PCB) member attached to at least one of the struts. The flexible PCB member is configured to deliver energy to one or more target sites within the sino-nasal cavity.

In some embodiments, the plurality of individual struts are transformable between a retracted configuration and a deployed configuration. In some embodiments, at least when in the deployed configuration, a portion of the plurality of individual struts are in a coaxial configuration with respect to a longitudinal axis of the device. In other embodiments, at least when in the deployed configuration, at least a portion of the plurality of individual struts are in an annular configuration with respect to a longitudinal axis of the device. In some embodiments, at least a portion of the plurality of individual struts comprises a free distal end. For example, the at least one segment includes a distal segment, in which each of the plurality of individual struts of the distal segment comprises a free, distal end. In some embodiments, at least a portion of the plurality of individual struts comprise connectors that connect different ones of the struts.

The end effector is multi-segmented and includes at least two segments, including a distal segment and a proximal segment. In some embodiments, the distal segment comprises a proximal end, generally in the form of a collar or the like, which is shaped and/or sized to be received by a portion of the proximal segment. In particular, the collar of the distal segment may be fitted within a corresponding collar of the proximal segment, such that the proximal and distal segments are coaxially aligned and share a common longitudinal axis.

In some embodiments, the proximal segment comprises at least one pair of struts, each of the struts comprising a first end and a second end that are each connected to a portion of the proximal segment. In other words, the pair of struts do not include a free, independent end (i.e., an end unconnected to anything). In some embodiments, the distal segment comprises struts that are deployable into a coaxial configuration with respect to a longitudinal axis of the device.

In some embodiments, at least a portion of the plurality of individual struts comprise one or more articulation sites that improve flexibility of the struts. For example, the one or more articulation sites comprise an area of reduced material. The area of reduced material may form an S-shaped configuration, for example.

In some embodiments, a portion of the plurality of individual struts are arranged around a circumference of a distal end of the end effector. In such an embodiment, at least one of the plurality of individual struts comprises a stiffness that is different than a stiffness of a second strut.

In some embodiments, the plurality of individual struts comprise at least two substantially flat faces opposite of one another.

In some embodiments, each of the plurality of struts comprises a corresponding flexible PCB member. It should be noted, however, that in some embodiments, some of the plurality of struts may be devoid of a corresponding flexible PCB member.

In some embodiments, the device further comprises a soft polymer disposed between the flexible PCB member and the at least one of the plurality of individual struts. In other embodiments, a soft polymer may be disposed over the flexible PCB member and the at least one of the plurality of individual struts.

Another aspect of the present invention provides a method of constructing a device for treating a condition in a sino-nasal cavity. The method includes providing an end effector dimensioned to be at least partially deployed inside a sino-nasal cavity of a patient and attaching a flexible printed circuit board (PCB) member to the end effector, the flexible PCB configured to deliver energy to a target site within the sino-nasal cavity.

In some embodiments, the attaching step involves a thermal process. The thermal process may comprise one of a reflow process, induction heating, spot welding, or laser welding. The reflow process may include polymer reflow, for example.

In some embodiments, the thermal process comprises positioning the flexible PCB member on the effector, disposing one or more polymer layers around the flexible PCB member, and applying at least heat.

In other embodiments, prior to attaching the flexible PCB member, a polymer sleeve is secured, via polymer reform, to a portion of the end effector to thereby provide a substrate onto which the flexible PCB member is attached.

In some embodiments, a portion of the end effector comprises one or more fixation points to facilitate the attachment of the flexible PCB to the end effector. The one or more fixation points comprise a recess, a hole, a notch, or a groove, etched into the end effector. The one or more fixation points may be disposed at a distal portion of the end effector.

In some embodiments, at least a portion of the flexible PCB member is attached to the end effector by an adhesive. The adhesive is applied to one or more fixation points disposed on the end effector prior to attaching the flexible PCB member.

In some embodiments, the end effector may include one or more deployable struts to which the flexible PCB member is attached. In some embodiments, at least a portion of the deployable struts comprise a free distal end. The deployable struts may include opposing surfaces and the flexible member is attached to the opposing surfaces. In some embodiments, the flexible PCB member is machined from a single sheet of flexible PCB material.

Another aspect of the present invention provides a method of constructing a device for treating a condition in a sino-nasal cavity. The method includes providing a single piece of metal and cutting the single piece of metal to form an end effector dimensioned for insertion into a sino-nasal cavity of a subject. The cutting involves laser machining.

The single piece of metal comprises at least one of a tube and a plate. The end effector generally includes one or more deployable struts, at least a portion of the deployable struts comprising a free distal end. In some embodiments, a proximal portion of the at least a portion of the struts comprises one or more articulation sites that facilitate flexibility.

Another aspect of the present invention provides a medical device including an end effector dimensioned for insertion into a sino-nasal cavity, the end effector comprising a plurality of struts extending from a distal end of the effector in a radial configuration, wherein at least two of the struts are connected by a cross member.

In some embodiments, the cross member comprises a flexible printed circuit board (PCB). In some embodiments, the cross member connects two immediately adjacent struts. In some embodiments, the plurality of struts are connected by a plurality of cross members. In some embodiments, substantially every other one of the plurality of the struts are connected by a corresponding cross member. In some the plurality of struts are connected by a plurality of cross members in non-uniform locations along a length of the plurality of struts. In some embodiments, the cross member comprises one or more electrodes. In some embodiments, the plurality of struts and the cross member are transformable between a retracted configuration and a deployed configuration. In some embodiments, when in a deployed configuration, the cross member achieves a locked state inhibiting retraction of the plurality of struts.

In some embodiments, the cross member comprises an articulation site. In some embodiments, the cross member comprises an arcuate shape. In some embodiments, the cross member comprises a chevron shape. In some embodiments, the cross member is configured to influence a radial stiffness of the at least two struts.

In some embodiments, each one of the plurality of struts comprises a flexible PCB member configured to deliver energy to one or more target sites within the sino-nasal cavity.

In some embodiments, at least two of the flexible PCB members are configured to deliver different energy profiles from each other.

### Brief Description of the Drawings

FIGS. 1A and 1B are diagrammatic illustrations of a system for treating a condition of a patient using a handheld device according to some embodiments of the present disclosure.
FIG. 2 is a diagrammatic illustration of the console coupled to the handheld device consistent with the present disclosure, further illustrating one embodiment of an end effector of the handheld device for delivering energy to tissue at one or more target sites.
FIG. 3A is a cut-away side view illustrating the anatomy of a lateral sino-nasal wall.
FIG. 3B is an enlarged side view of the nerves of the lateral sino-nasal wall of FIG. 1A.
FIG. 3C is a front view of a left palatine bone illustrating geometry of microforamina in the left palatine bone.
FIG. 4 is a side view of one embodiment of a handheld device for providing therapeutic treatment consistent with the present disclosure.
FIGS. 5A-5F are various views of a multi-segment end effector consistent with the present disclosure.

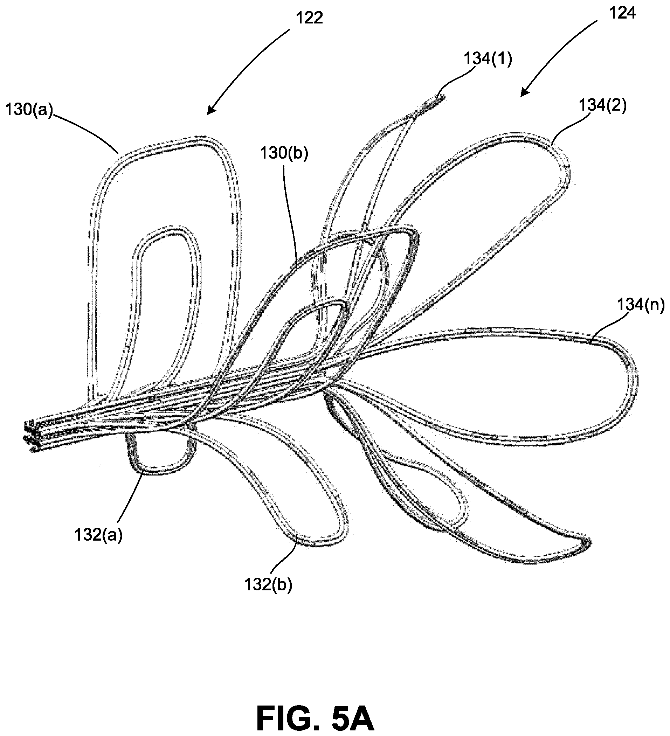

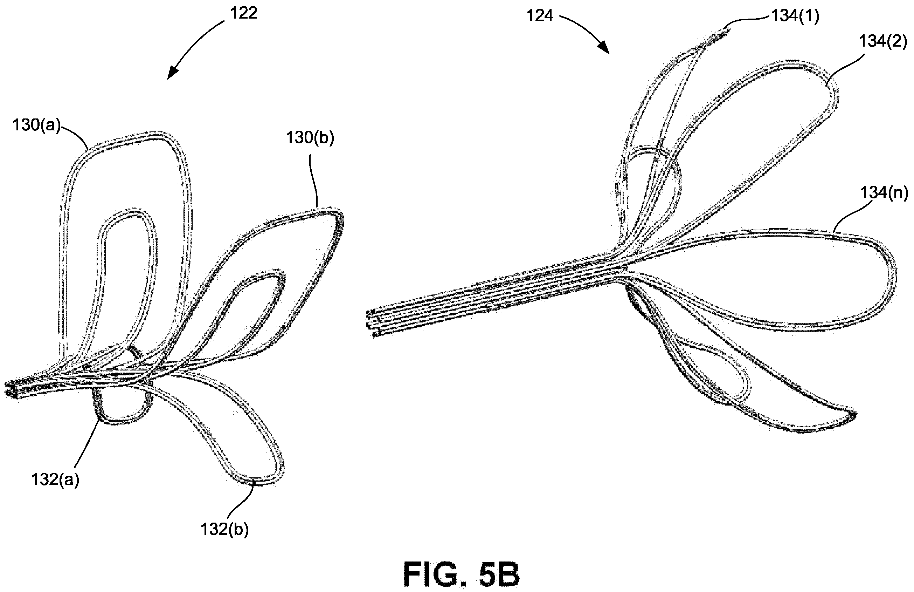

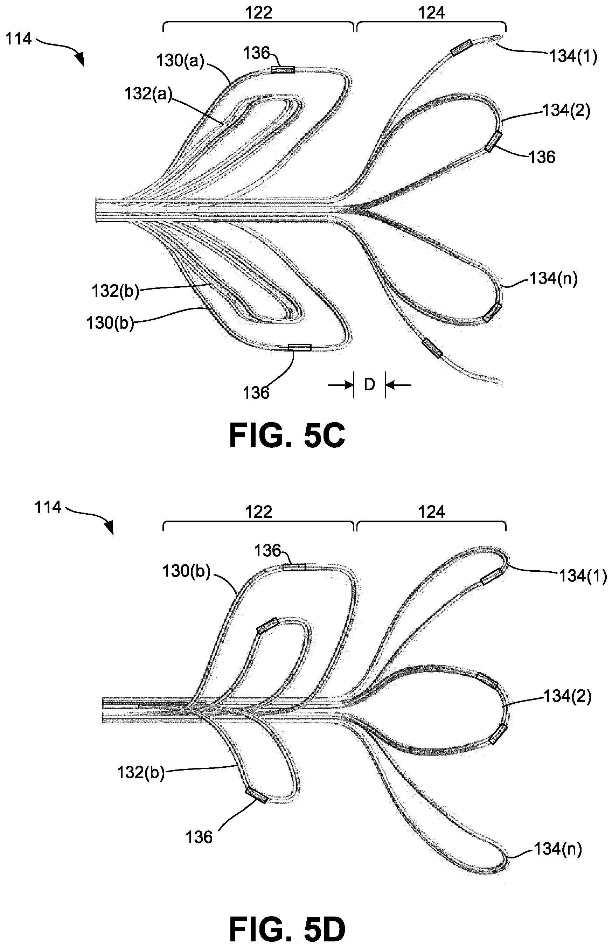

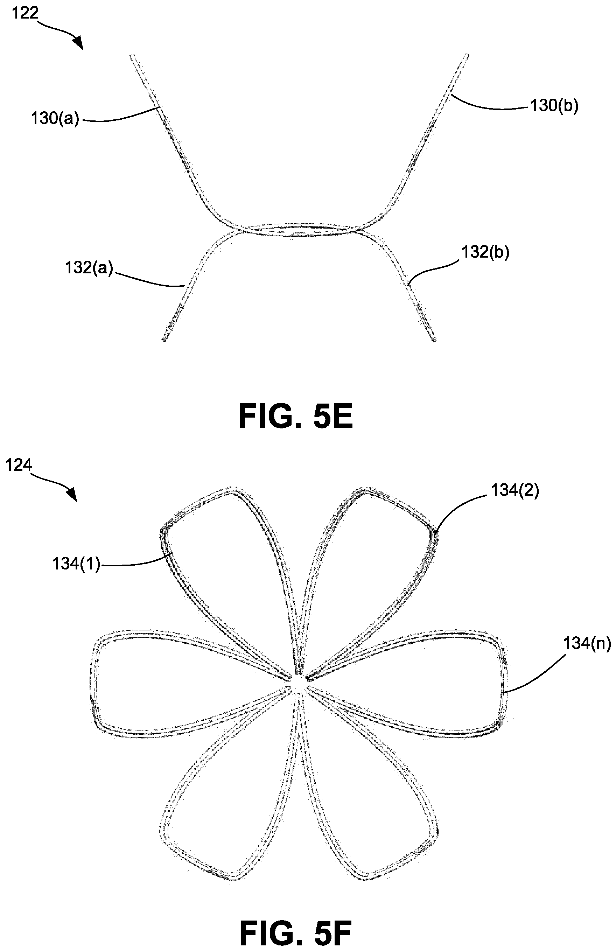

FIG. 5A is an enlarged, perspective view of the multi-segment end effector illustrating the first (proximal) segment and second (distal) segment. FIG. 5B is an exploded, perspective view of the multi-segment end effector. FIG. 5C is an enlarged, top view of the multi-segment end effector. FIG. 5D is an enlarged, side view of the multi-segment end effector. FIG. 5E is an enlarged, front (proximal facing) view of the first (proximal) segment of the multi-segment end effector. FIG. 5F is an enlarged, front (proximal facing) view of the second (distal) segment of the multi-segment end effector.

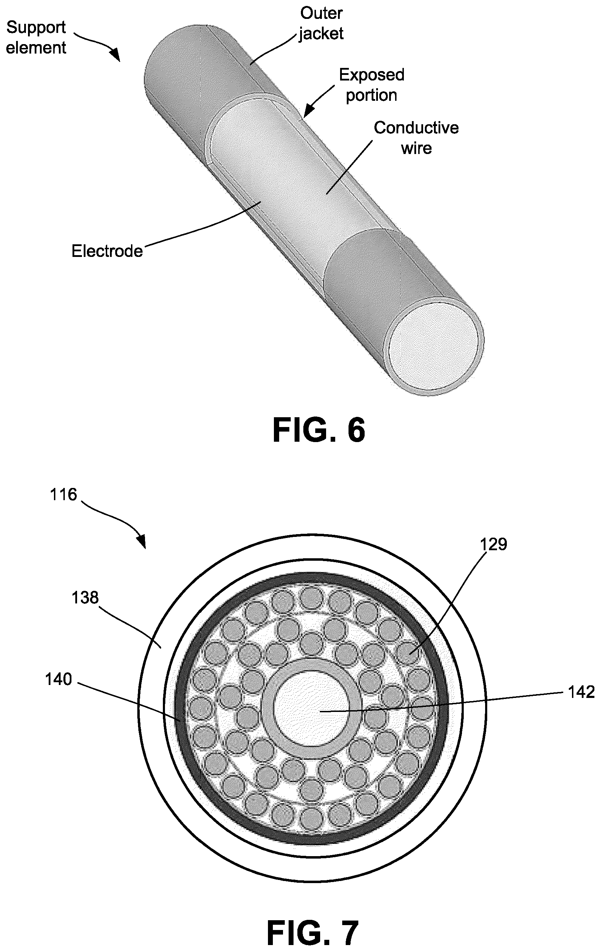

FIG. 6 is a perspective view, partly in section, of a portion of a support element illustrating an exposed conductive wire serving as an energy delivery element or electrode element.

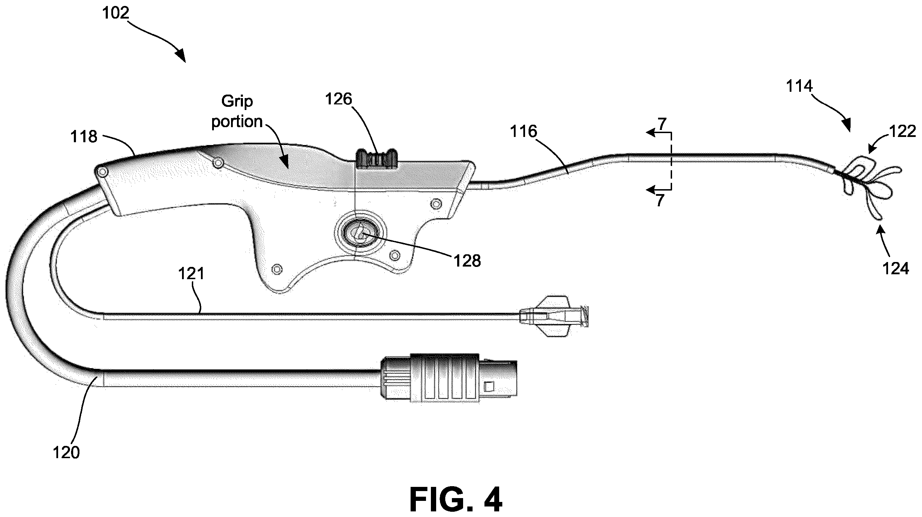

FIG. 7 is a cross-sectional view of a portion of the shaft of the handheld device taken along lines 7-7 of FIG. 4.

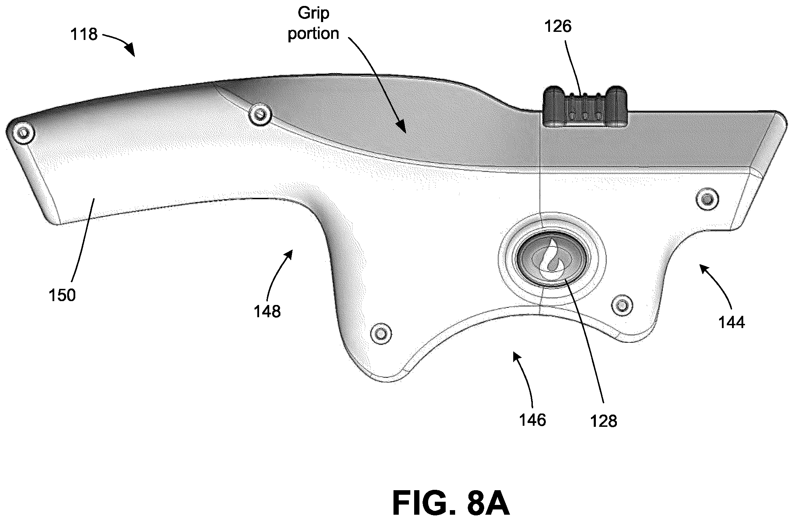

FIG. 8A is a side view of the handle of the handheld device.

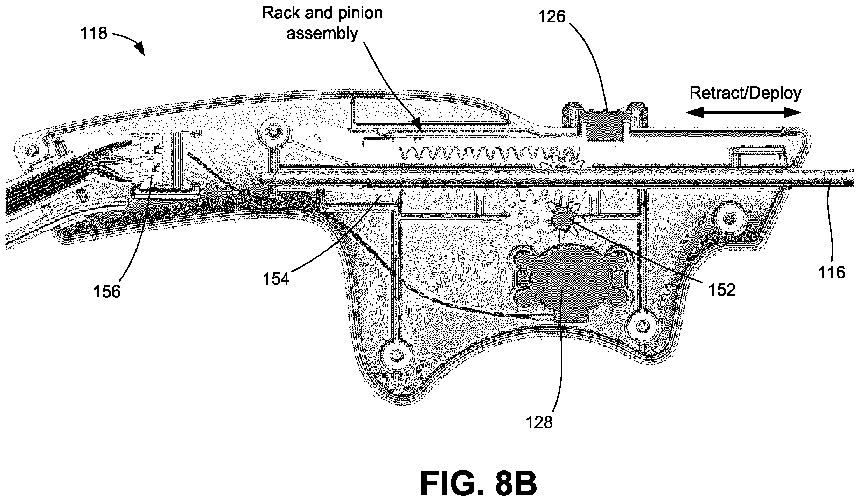

FIG. 8B is a side view of the handle illustrating internal components enclosed within.

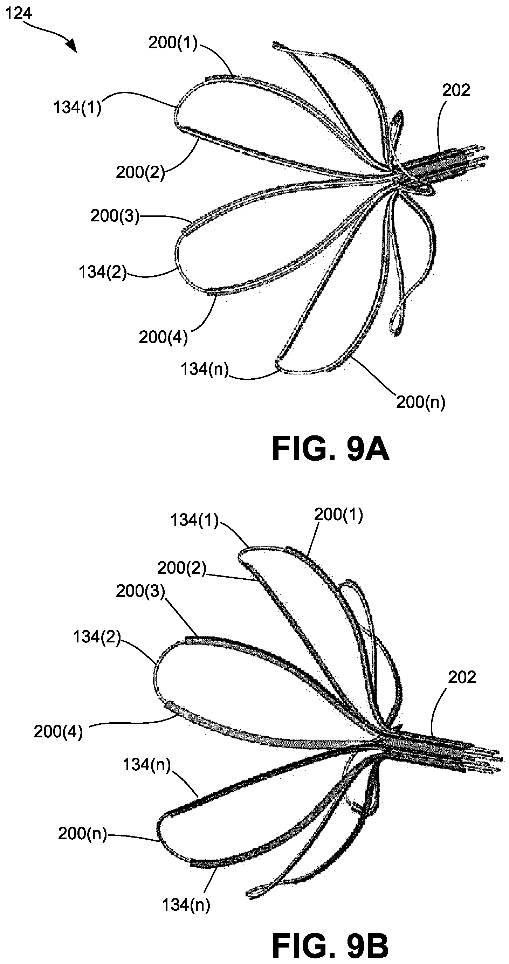

FIGS. 9A and 9B are enlarged perspective views of the second (distal) segment of the multi-segment end effector including a flexible printed circuit board (PCB) assembly operably associated therewith.

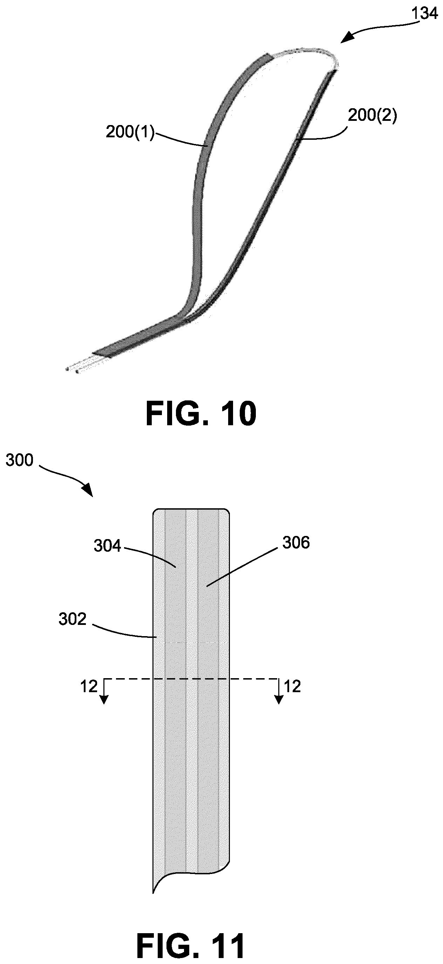

FIG. 10 is an enlarged perspective view of a single looped strut or support element of the second (distal) segment, illustrating individual PCB members fixed to portions thereof.
FIG. 11 is an enlarged plan view of one embodiment of a flexible PCB member consistent with the present disclosure.

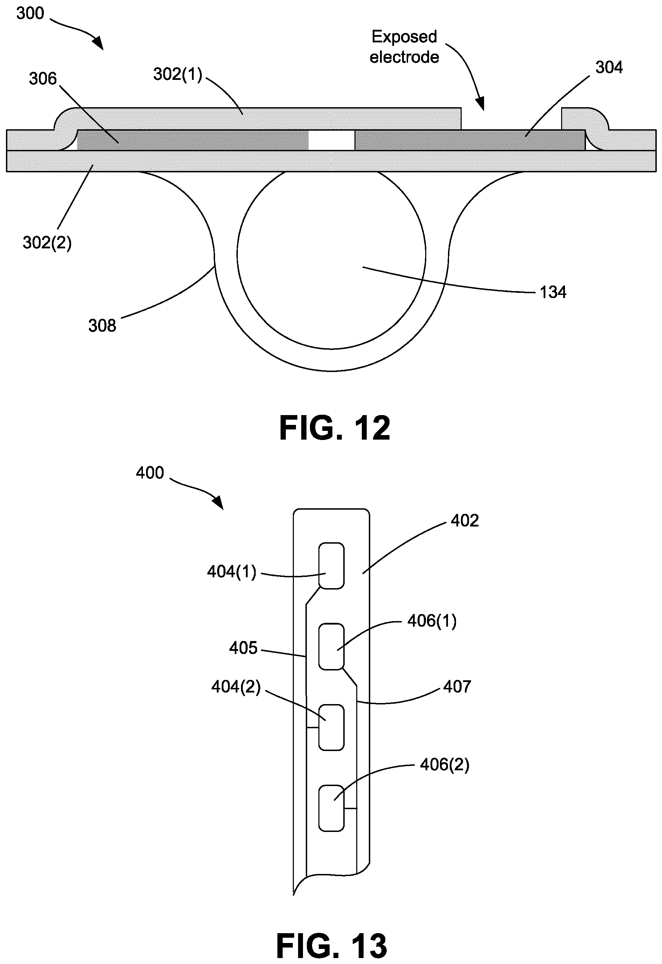

FIG. 12 is a cross-sectional view of a portion of the flexible PCB member taken along lines 12-12 of FIG. 11.
FIG. 13 is an enlarged plan view of another embodiment of a flexible PCB member consistent with the present disclosure.

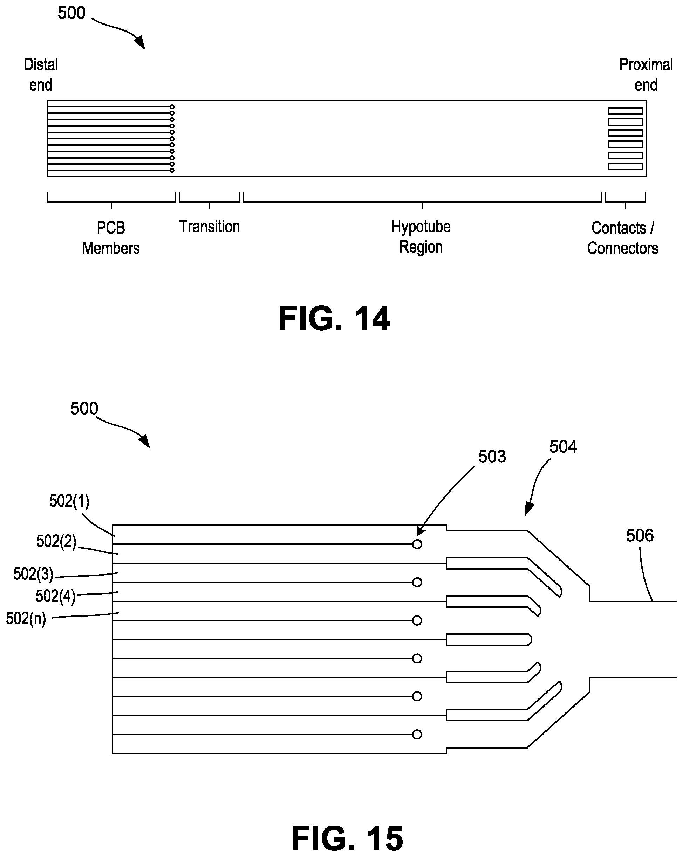

FIG. 14 is a plan view of a flexible PCB assembly consistent with the present disclosure, illustrating various portions of the assembly.
FIG. 15 is an enlarged plan view of one embodiment of flexible PCB members of a flexible PCB assembly consistent with the present disclosure.

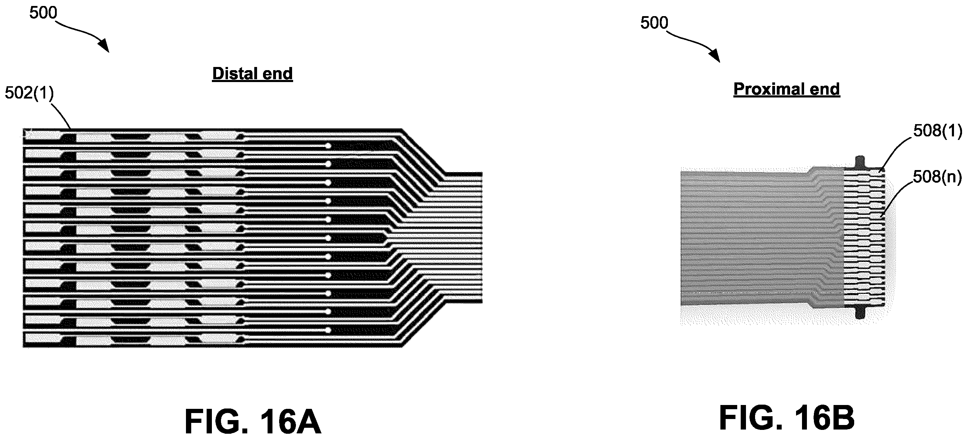

FIGS. 16A and 16B are enlarged plan views of a distal end and proximal end, respectively, of a flexible PCB assembly consistent with the present disclosure.

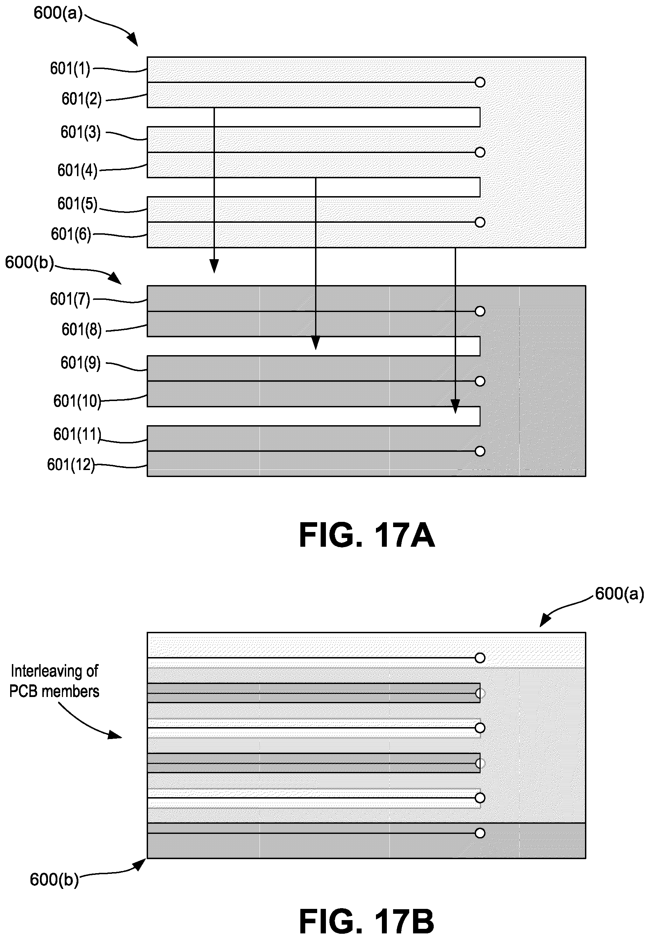

FIGS. 17A and 17B are plan views of another embodiment of a flexible PCB assembly consistent with the present disclosure, illustrating the interleaving of two separate assemblies to form a combined assembly of overlapping flexible PCB members from each assembly.

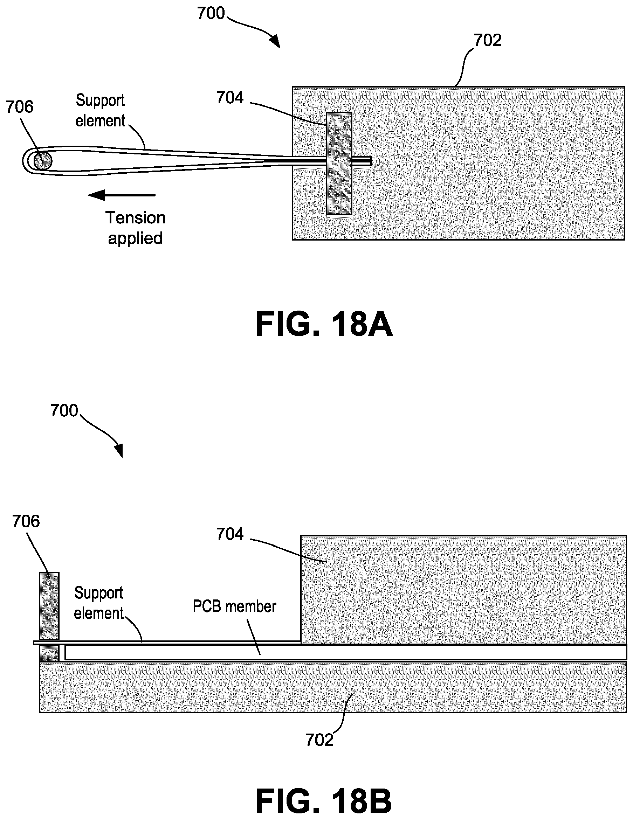

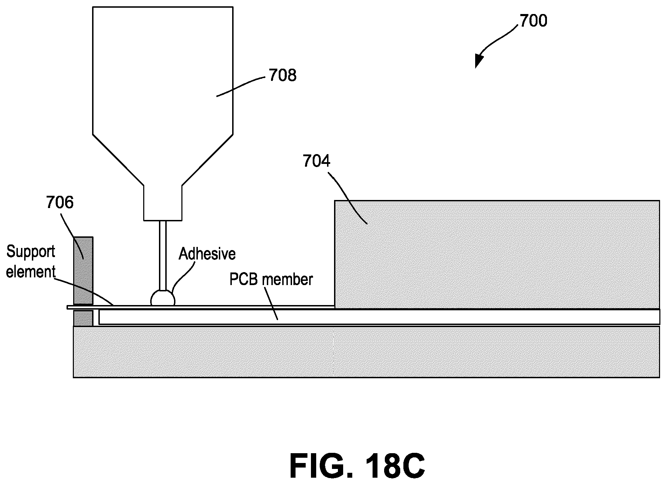

FIGS. 18A, 18B, and 18C are top and side views, partly in section, of one embodiment of a jig assembly used for attaching one or more flexible PCB members to respective support elements of the second (distal) segment of the end effector.

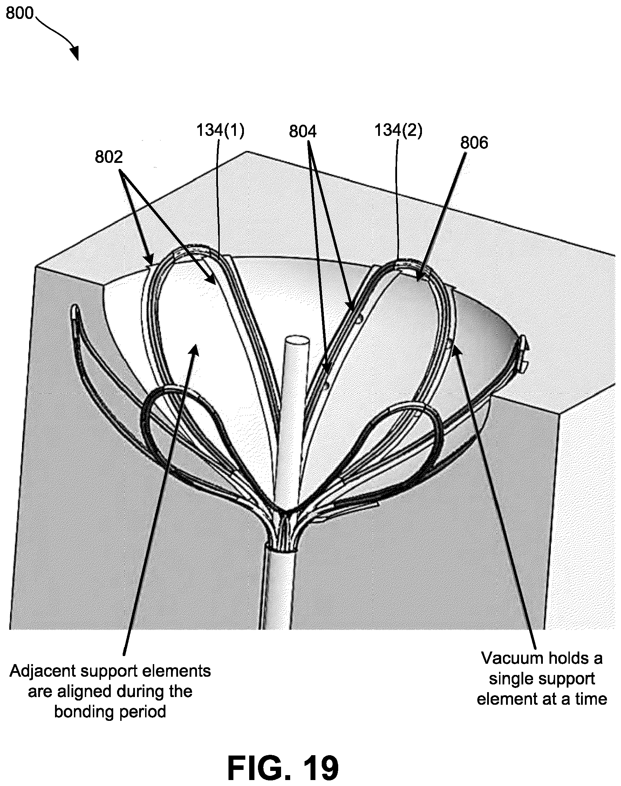

FIG. 19 is a perspective view of another embodiment of a jig assembly used for attaching one or more flexible PCB members to respective support elements of the second (distal) segment of the end effector.

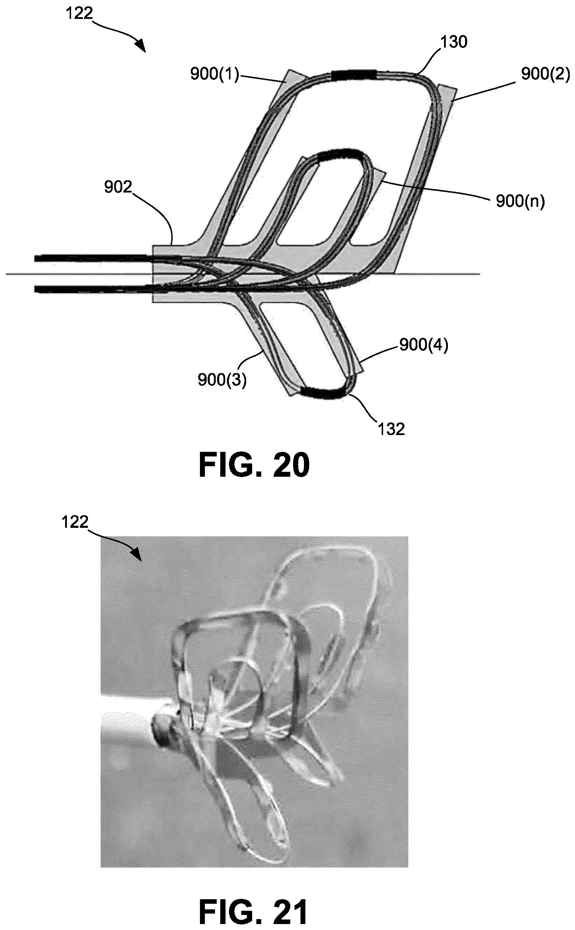

FIG. 20 is an enlarged side view of the first (proximal) segment of the multi-segment end effector illustrating placement of a flexible PCB assembly, comprised of multiple flexible PCB members, upon the various support elements of the first (proximal) segment.
FIG. 21 is an image illustrating a perspective view of the first (proximal) segment including the flexible PCB assembly attached to the support elements.

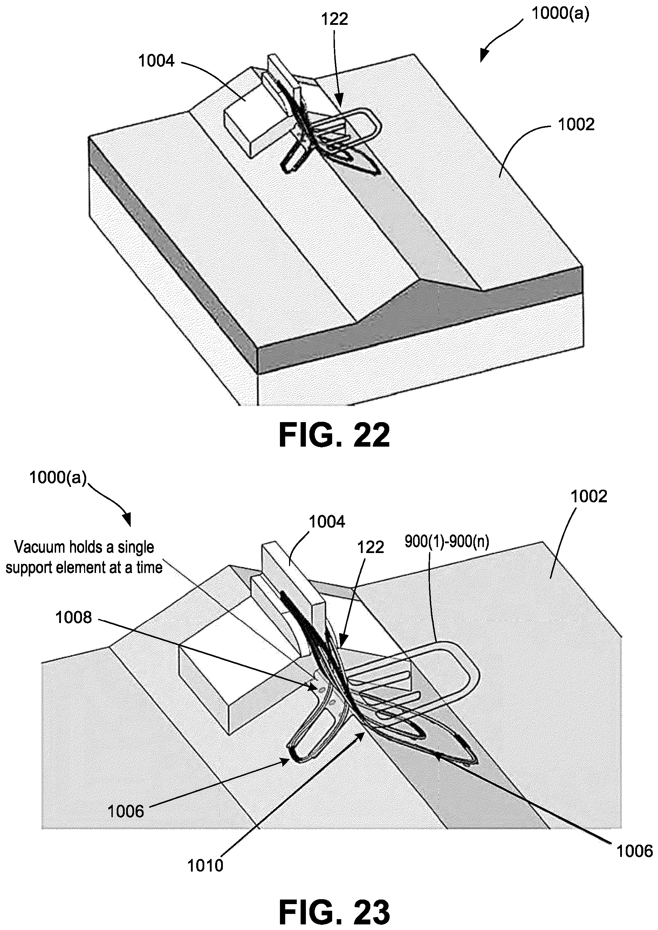

FIG. 22 is a perspective view of one embodiment of a jig assembly used for attaching one or more flexible PCB members to respective support elements of the first (distal) segment of the end effector. FIG. 23 is an enlarged view of the jig assembly of FIG. 22.

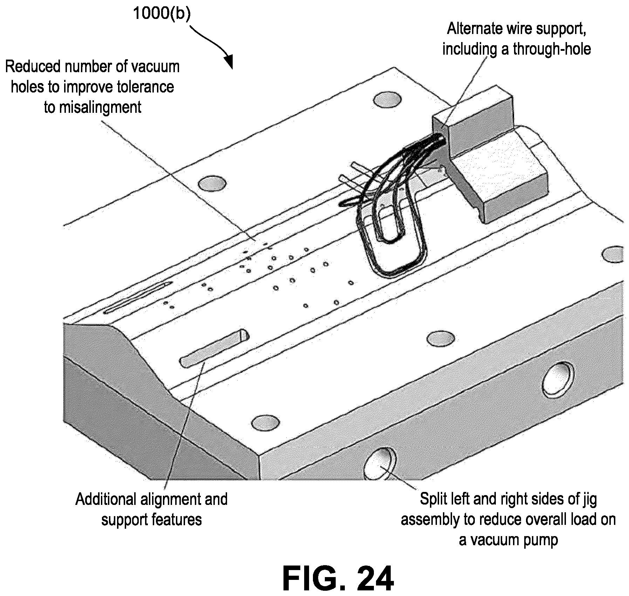

FIG. 24 is an enlarged view of an alternate embodiment of the jig assembly of FIG. 22.

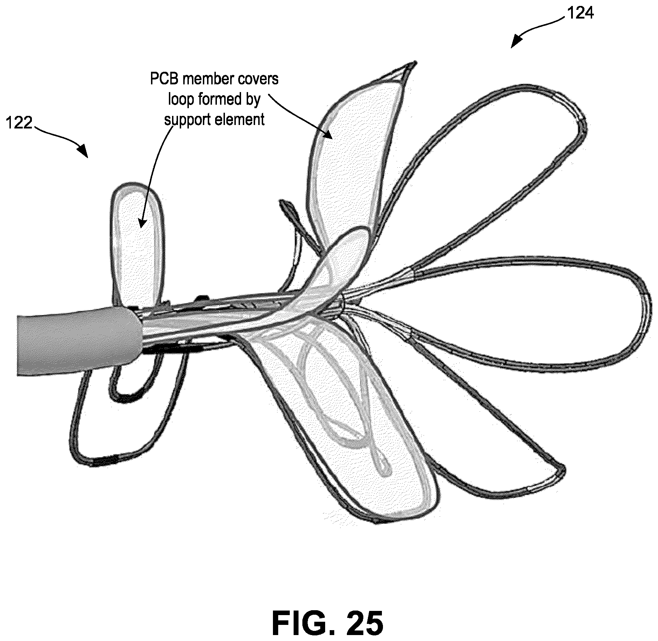

FIG. 25 is an enlarged perspective view of the multi-stage end effector illustrating flexible PCB members coupled to loop struts or support elements of each of the first (proximal) and second (distal) segments, in which the flexible PCB members substantially covers the loop-like or leaflet-like shape when in the deployed configuration.

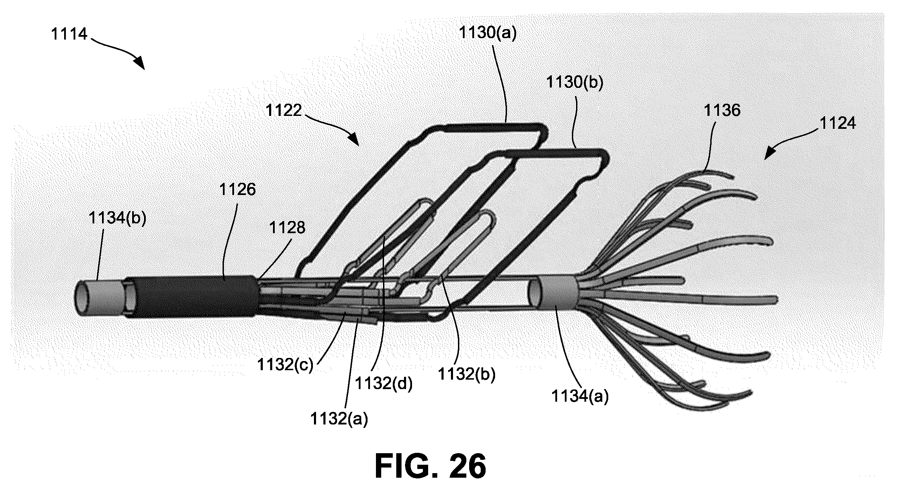

FIG. 26 is a side perspective view of another embodiment of a multi-segment end effector consistent with the present disclosure.

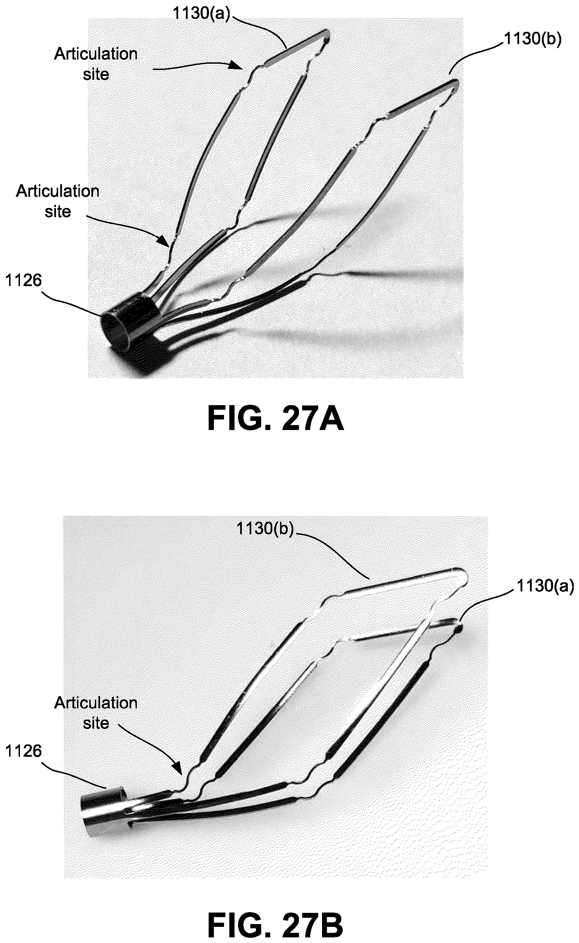

FIGS. 27A and 27B are perspective views of a portion (a first interlocking member) of the proximal segment of the end effector of FIG. 26.

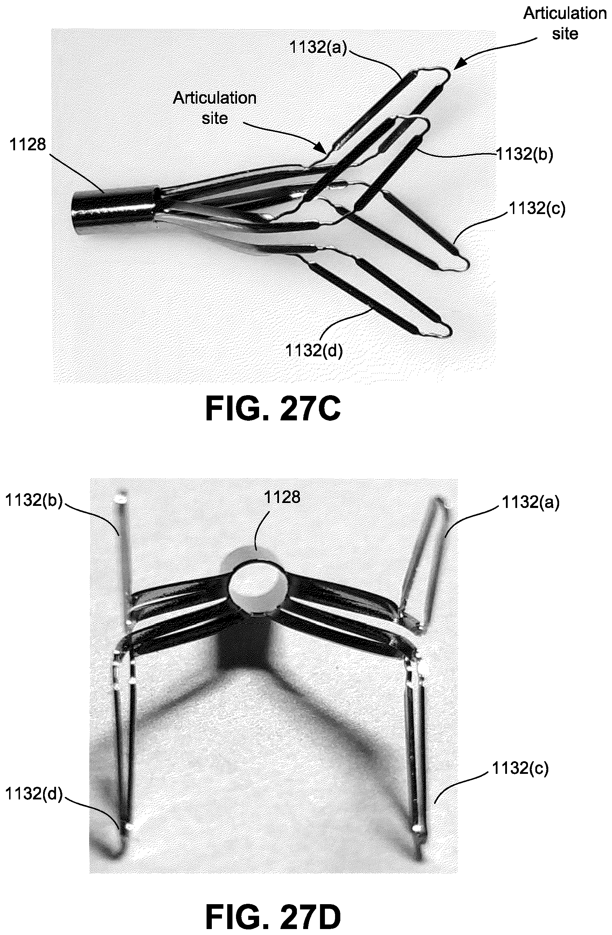

FIGS. 27C and 27D are side and front-facing perspective views of a portion (a second interlocking member) of the proximal segment of the end effector of FIG. 26.

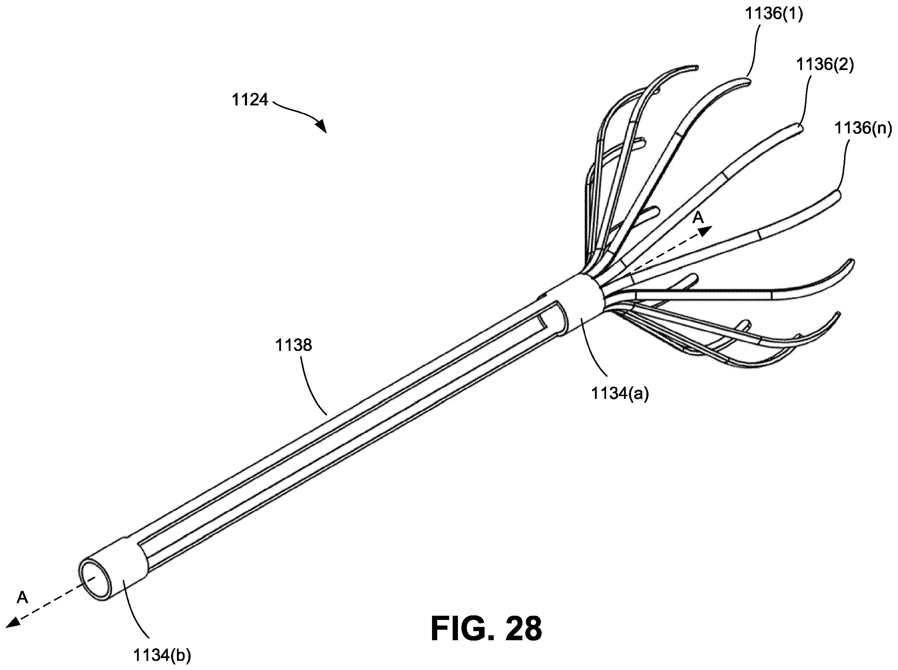

FIG. 28 is a perspective view of a distal segment of the end effector of FIG. 26.

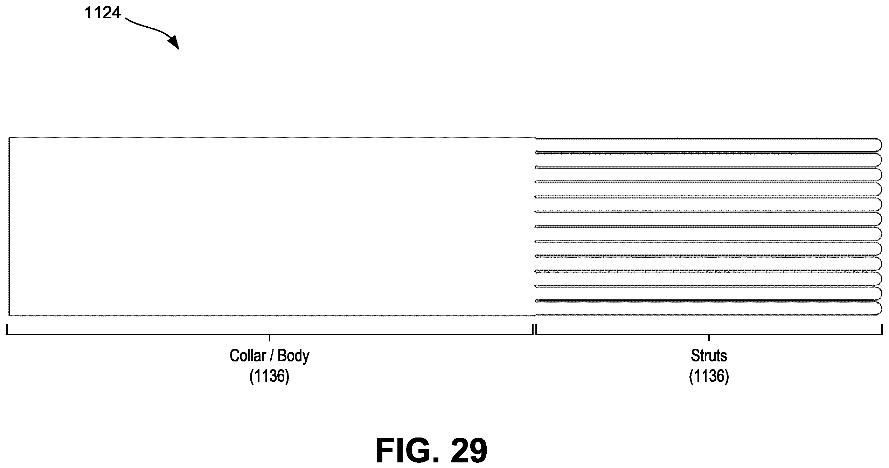

FIG. 29 is a plan view of a single workpiece of material from which the distal segment of FIG. 28, specifically the plurality of support elements / struts, are constructed via a laser machining process.

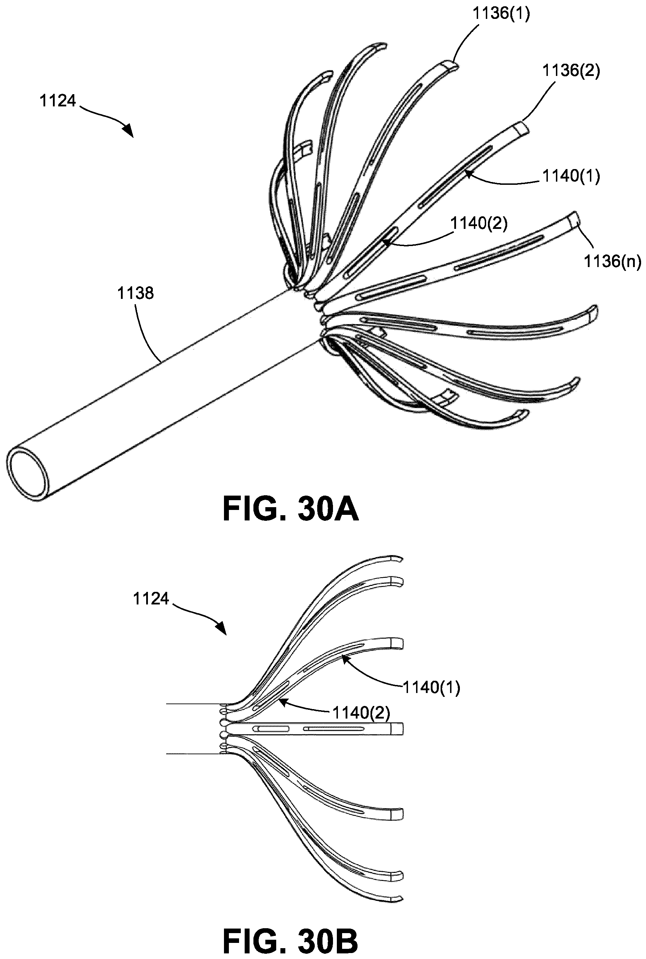

FIGS. 30A and 30B are perspective and side views of the distal segment illustrating fixation points defined on each of the plurality of support elements / struts.

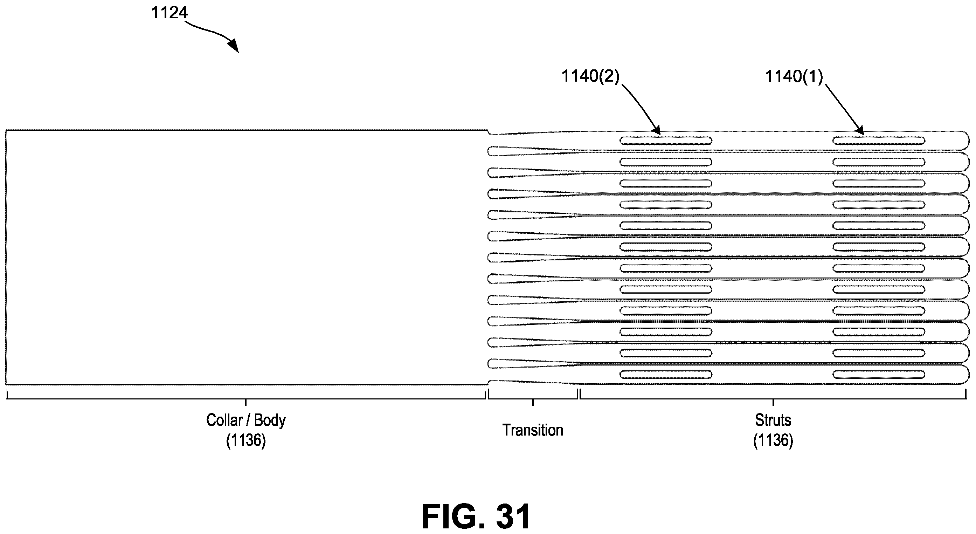

FIG. 31 is a plan view of a single workpiece of material from which the distal segment of FIGS. 30A-30B, specifically the plurality of support elements / struts and associated fixation points, are constructed via a laser machining process.

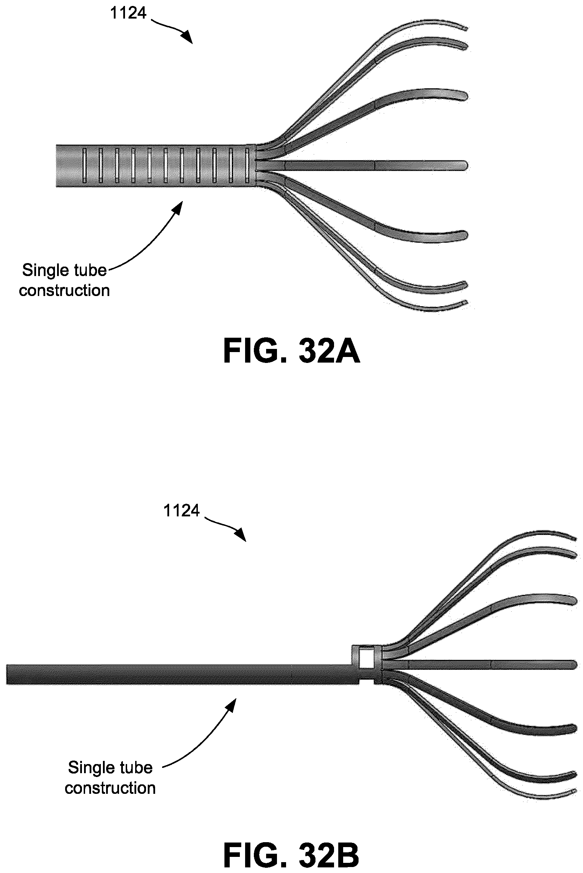

FIGS. 32A and 32B are side views of a single workpiece of material, generally in the form of a tube, from which the distal segment is constructed via a laser machining process.

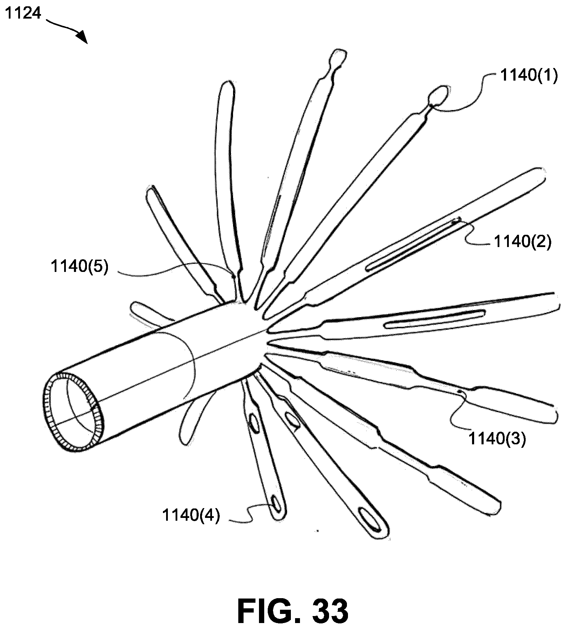

FIG. 33 is a perspective view of a distal segment illustrating various fixation point designs provided on one or more of the plurality of support elements / struts.

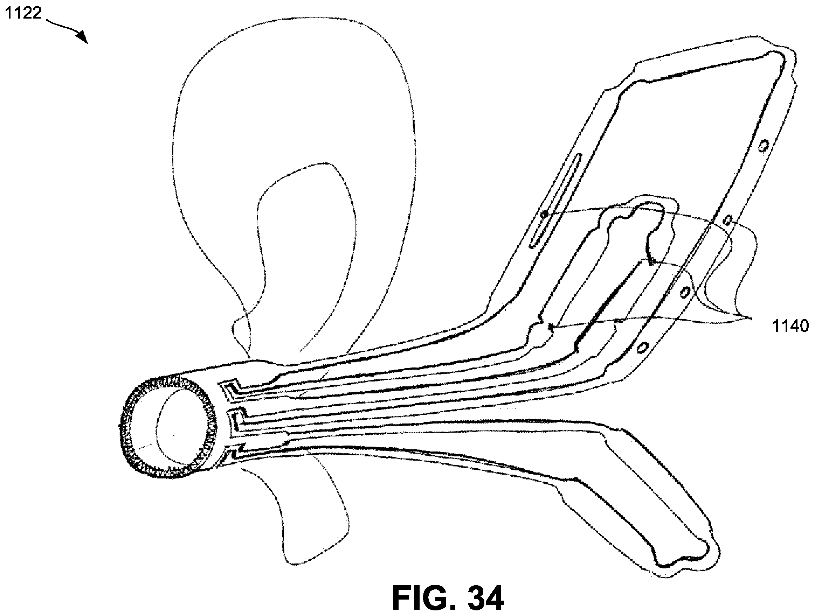

FIG. 34 is a perspective view of a proximal segment illustrating various fixation point designs provided on one or more of the plurality of support elements / struts.

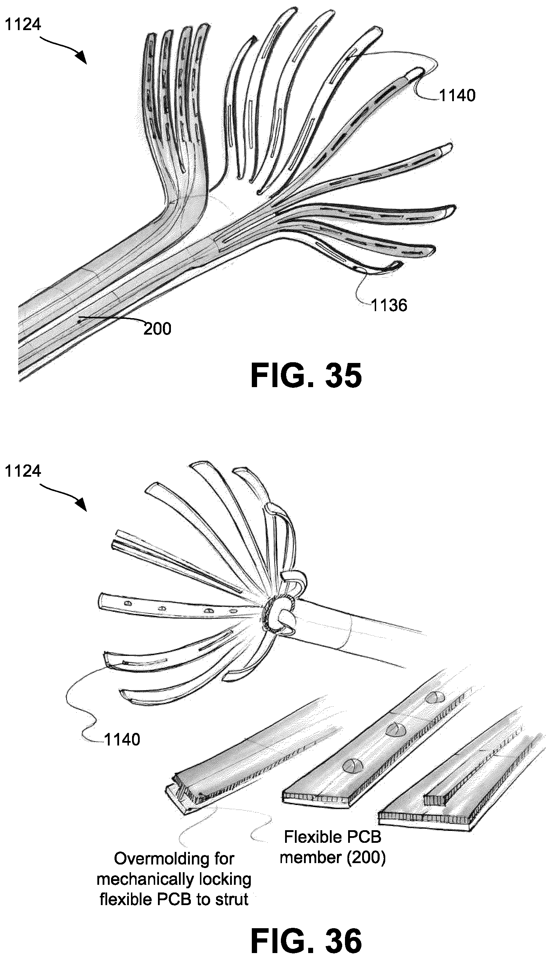

FIG. 35 is a perspective view illustrating coupling of flexible PCB members to corresponding support elements / struts of the distal segment of FIGS. 30A-30B, utilizing the fixation points.
FIG. 36 is a perspective view of the distal segment of FIGS. 30A-30B, including enlarged views illustrating the use of a polymer overlay to join a flexible PCB member to a corresponding support element / strut of the distal segment.

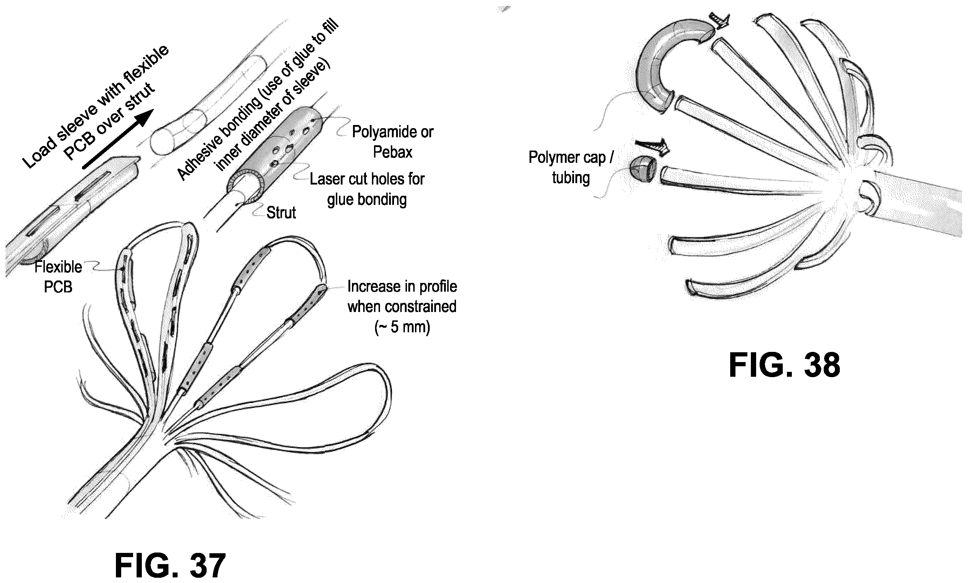

FIG. 37 is a perspective view of the distal segment of FIGS. 9A-9B, for example, illustrating loading of a polymer sleeve over a wire support element of the distal segment, in which a flexible PCB is joined to the polymer sleeve.
FIG. 38 is a perspective view of the distal segment of FIG. 28, illustrating placement of polymer caps or tubing to the free, distal ends of the support elements / struts.

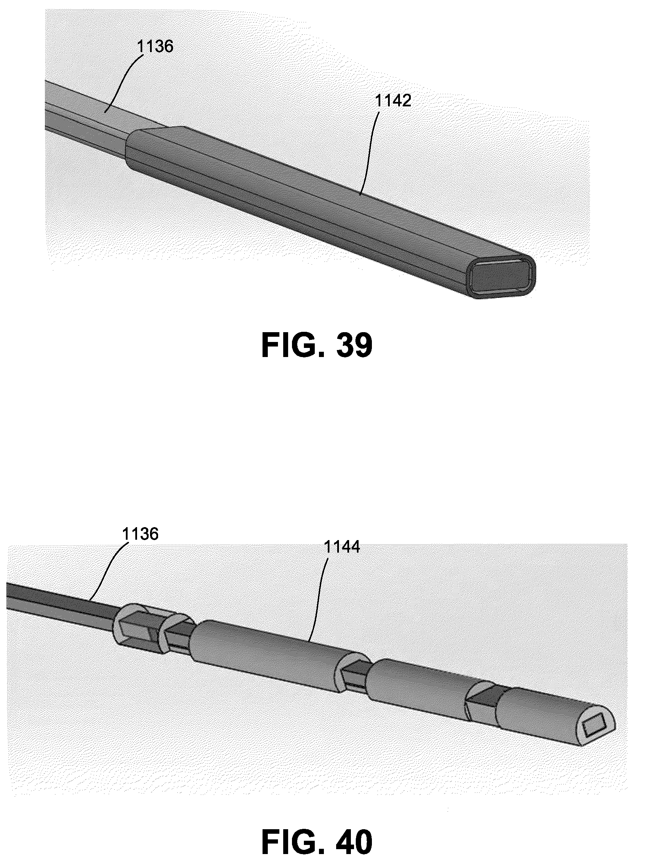

FIGS. 39 and 40 are enlarged views illustrating placement of polymer sleeves over a support element / strut of the distal segment of FIG. 29. FIG. 39 illustrates a sleeve extending along a length of the support element / strut, while FIG. 40 illustrates discrete portions of a polymer sleeve positioned along a length of the support element / strut (generally forming runners at specific locations of the support element / strut.

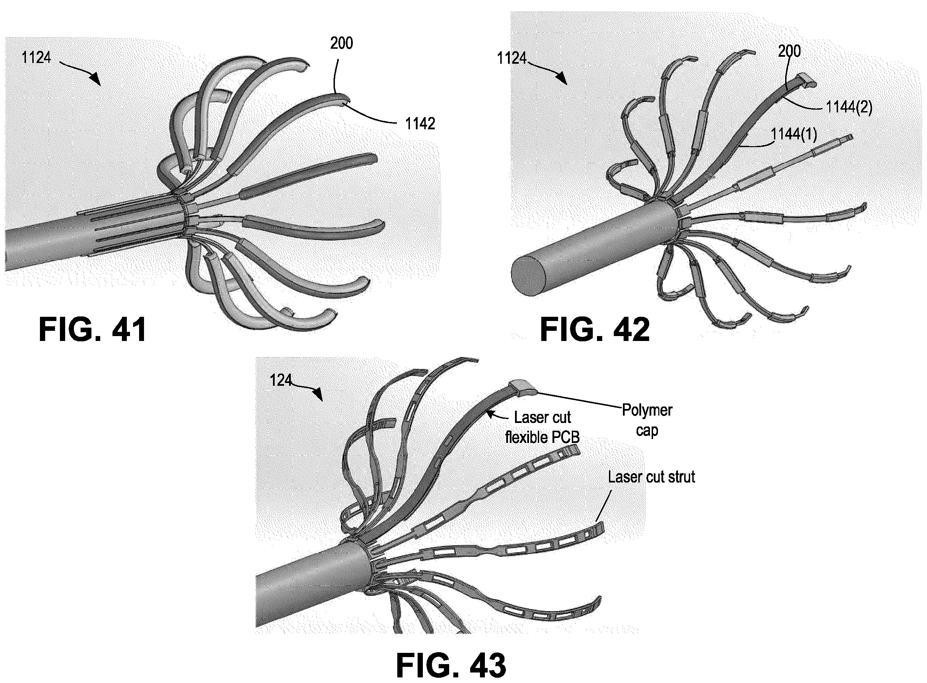

FIG. 41 is a perspective view of the distal segment in which each of the plurality of support elements / struts includes a polymer sleeve running a majority of the length and further include a flexible PCB member attached thereto.
FIG. 42 is a perspective view of the distal segment in which each of the plurality of support elements / struts includes polymer runners provided at discrete locations along a length of each and further include a flexible PCB member attached to the runners on each support element / strut.
FIG. 43 is a perspective view of a distal segment in which each of the support elements / struts has been laser cut and further includes multiple fixation points to which flexible PCB members may be attached.

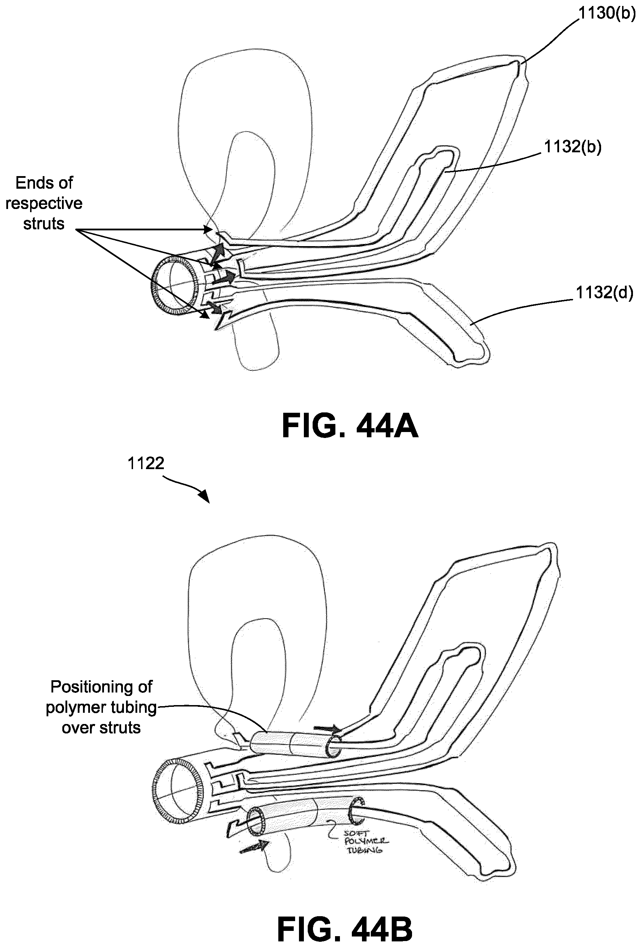

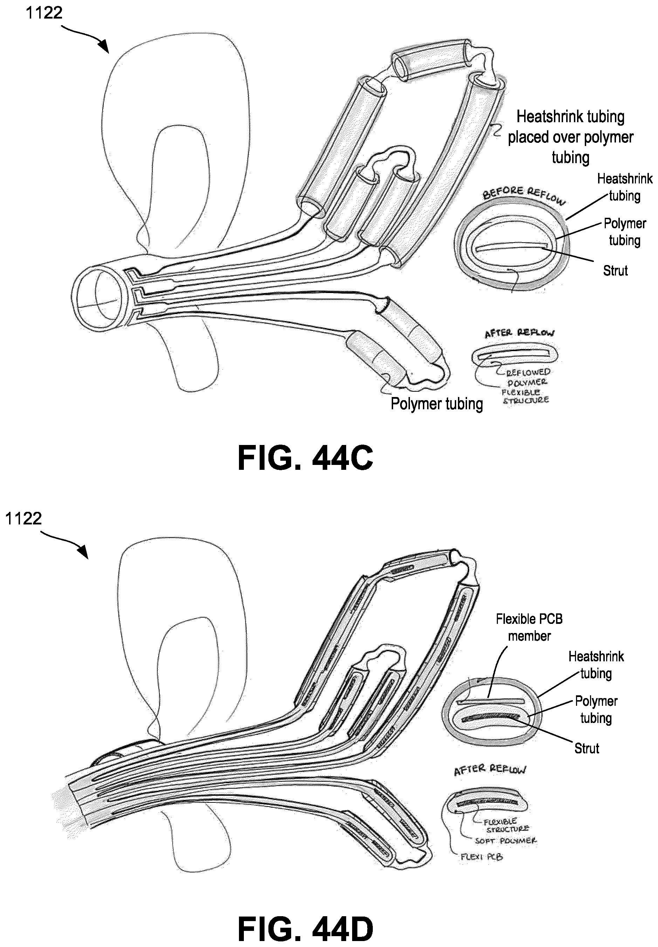

FIGS. 44A, 44B, 44C, and 44D illustrate a reflow process for positioning and affixing flexible PCB members and polymer tubing to the struts of the proximal segment.

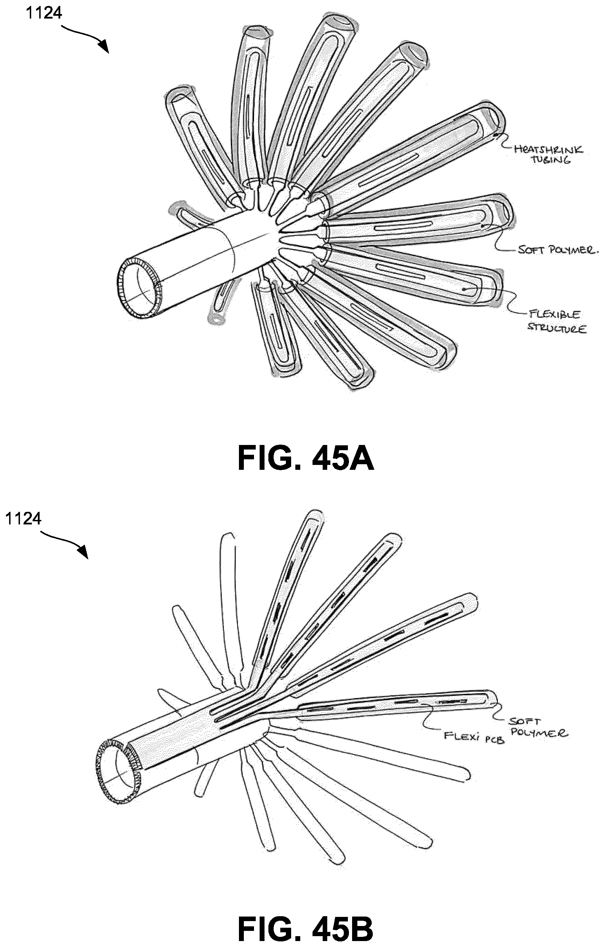

FIGS. 45A and 45B illustrate a reflow process for positioning and affixing flexible PCB members and polymer tubing to the support elements / struts of the distal segment.

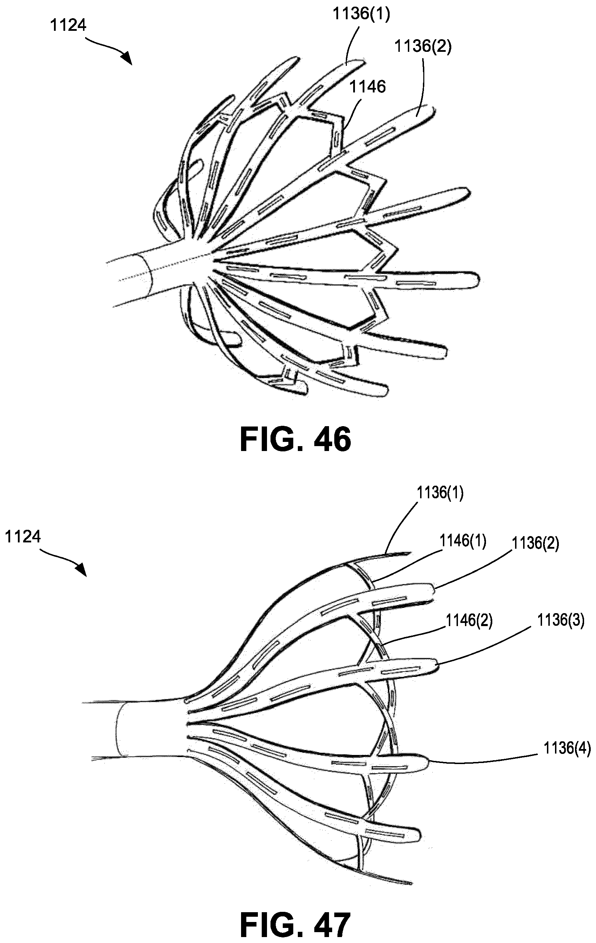

FIG. 46 is a perspective view of a distal segment in which a plurality of support elements / struts are connected by cross members in a first configuration (i.e., an s-shape or chevron-shape pattern), wherein immediately adjacent support elements / struts are connected to one another.
FIG. 47 is a perspective view of a distal segment in which a plurality of support elements / struts are connected by cross members in a second configuration, wherein every other one of the plurality of the support elements / struts are connected by a corresponding cross member.

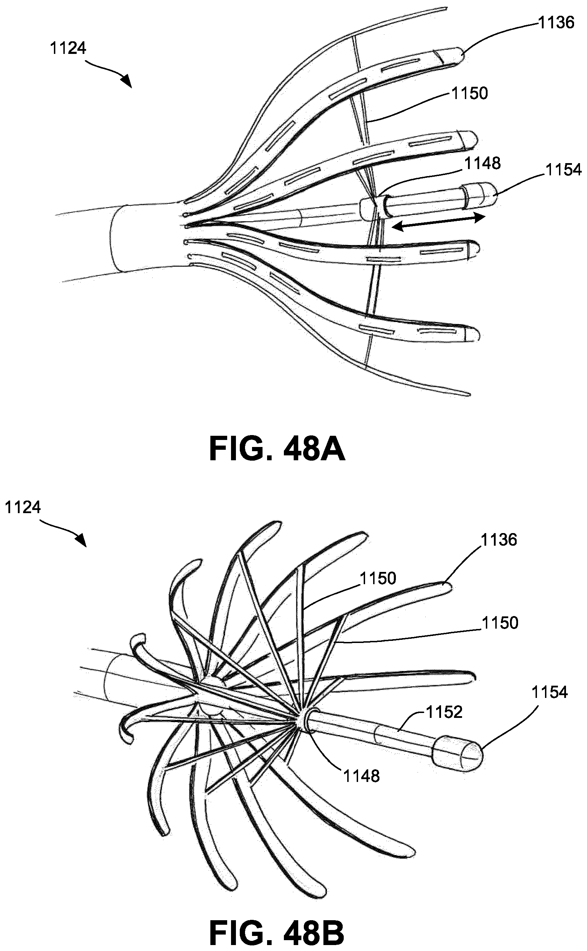

FIGS. 48A and 48B are side views illustrating a distal segment transitioning to an expanded, deployed configuration and corresponding movement of a cross member locking mechanism coupling at least two support elements / struts to one another and achieving a locked state when the segment is in a fully deployed configuration to thereby inhibit retraction of the plurality of support elements / struts.

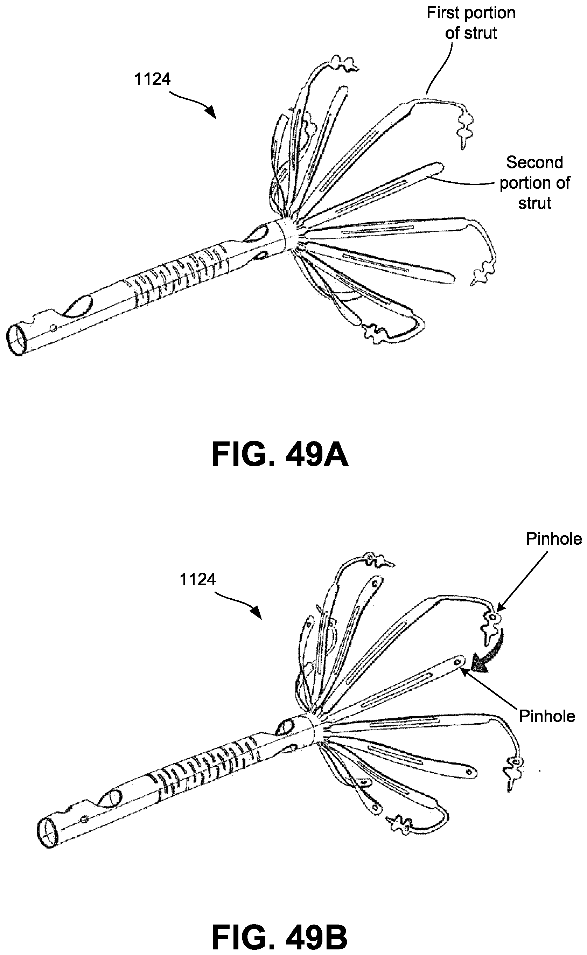

FIGS. 49A-49C are perspective views of a distal segment including one embodiment of looped struts, each of which comprises two portions that couple to one another to cooperatively form the looped strut.

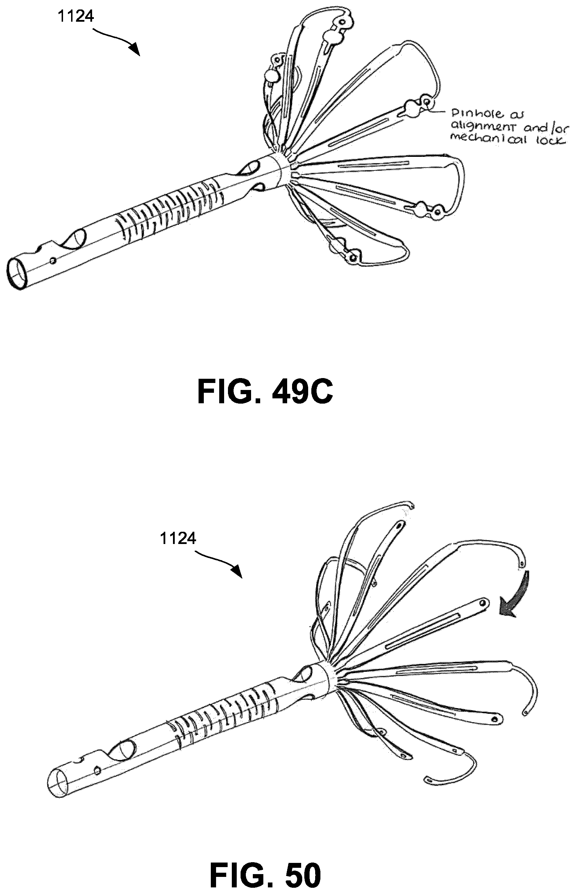

FIG. 50 is a perspective view of a distal segment including another embodiment of looped struts, each of which comprises two portions that couple to one another to cooperatively form the looped strut

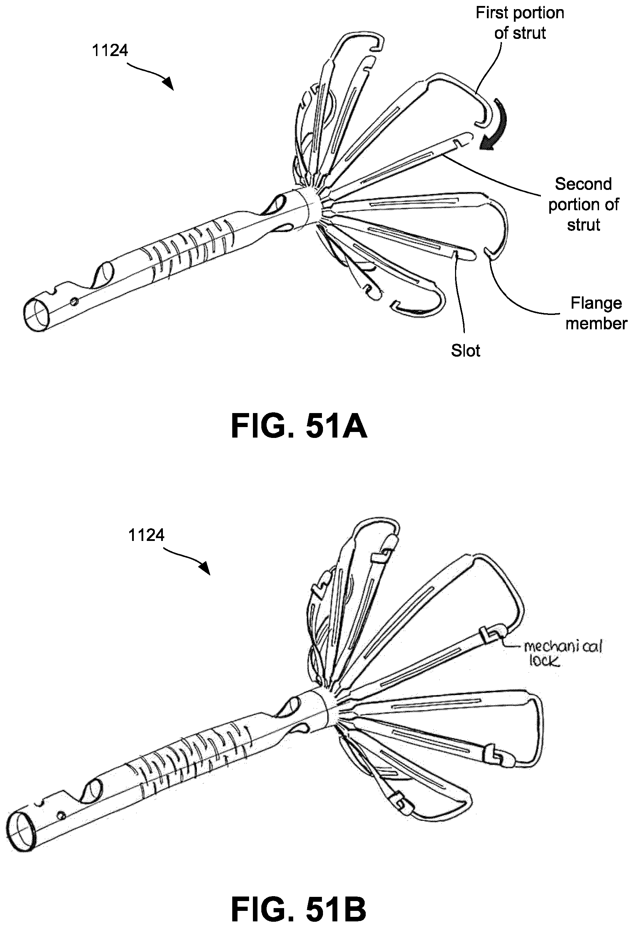

FIGS. 51A and 51B are perspective views of a distal segment including another embodiment of looped struts, each of which comprises two portions that mechanically couple to one another to cooperatively form the looped strut.

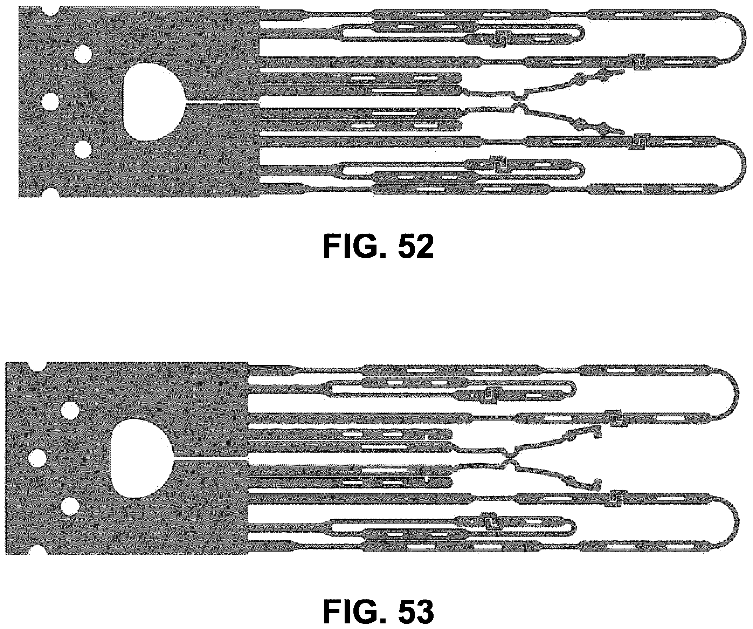

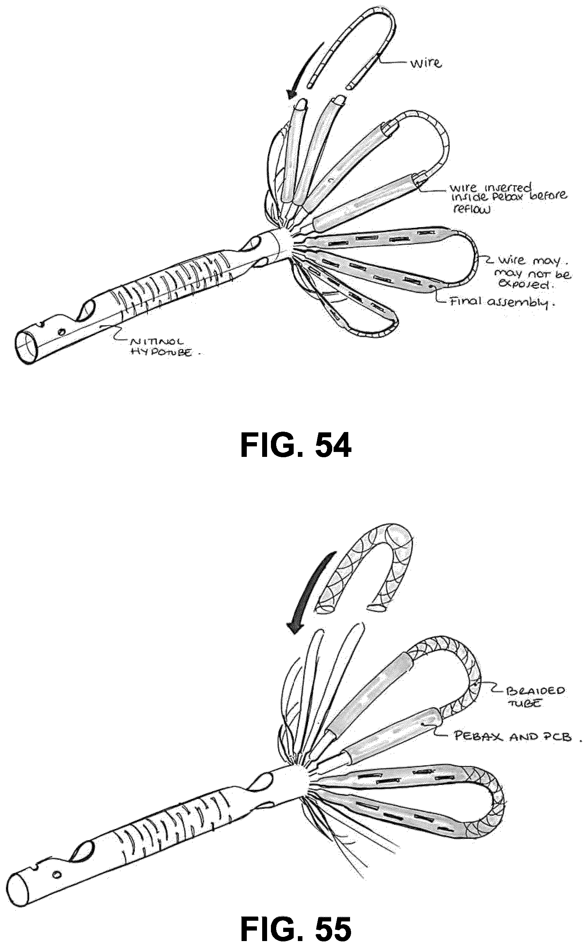

FIGS. 52 and 53 are perspective views of the distal segment of FIG. 28, each figure illustrating placement of polymer tubing to the free, distal ends of the support elements / struts and further joining adjacent struts to one another via placement of a wire or braided tube over respective distal ends of the adjacent struts and fixing the wire or braided tube in place via a polymer reflow process.

### Detailed Description

There are various conditions related to the sino-nasal cavity which may impact breathing and other functions of the nose. One of the more common conditions is rhinitis, which is defined as inflammation of the membranes lining the nose. The symptoms of rhinitis include sino-nasal blockage, obstruction, congestion, sino-nasal discharge (e.g., rhinorrhea and/or posterior sino-nasal drip), facial pain, facial pressure, and/or reduction or complete loss of smell and/or taste. Sinusitis is another common condition, which involves an inflammation or swelling of the tissue lining the sinuses, which can lead to subsequent. Rhinitis and sinusitis are frequently associated with one another, as sinusitis is often preceded by rhinitis. Accordingly, the term rhinosinusitis is often used to describe both conditions.

Depending on the duration and type of systems, rhinosinusitis can fall within different subtypes, including allergic rhinitis, non-allergic rhinitis, chronic rhinitis, acute rhinitis, recurrent rhinitis, chronic sinusitis, acute sinusitis, recurrent sinusitis, and medical resistant rhinitis and/or sinusitis, in addition to combinations of one or more of the preceding conditions. It should be noted that an acute rhinosinusitis condition is one in which symptoms last for less than twelve weeks, whereas a chronic rhinosinusitis condition refers to symptoms lasting longer than twelve weeks.

A recurrent rhinosinusitis condition refers to four or more episodes of an acute rhinosinusitis condition within a twelve-month period, with resolution of symptoms between each episode. There are numerous environmental and biological causes of rhinosinusitis. Non-allergic rhinosinusitis, for example, can be caused by environmental irritants, medications, foods, hormonal changes, and/or sino-nasal septum deviation. Triggers of allergic rhinitis can include exposure to seasonal allergens, perennial allergens that occur any time of year, and/or occupational allergens. Accordingly, rhinosinusitis affects millions of people and is a leading cause for patients to seek medical care.

The invention recognizes that a problem with current surgical procedures is that such procedures are not accurate, cause significant collateral damage, and are limited in scope of treatment. The invention solves that problem by providing a treatment device having a unique end effector configured to complement anatomy a multiple different locations within the sino-nasal cavity. The end effector includes one or more retractable and expandable segments, each of which is comprises a framework of support elements having elastic properties. Once delivered within the sino-nasal cavity, the one or more segments can expand to a specific shape and/or size corresponding to anatomical structures within the sino-nasal cavity and associated with target sites to undergo delivery of therapeutic energy for treatment of a condition (i.e., rhinosinusitis or the like). Each retractable and expandable segment comprises one or more flexible printed circuit board (PCB) members provided thereon. The flexible PCB members are composed of a flexible material capable of moving (e.g, bending, twisting, folding, etc.) between various positions in correspondence with movement of the underlying retractable and expandable segment to which it is attached. Each flexible PCB member further includes one or more energy delivering elements (e.g., electrodes) provided thereon and configured to deliver energy to tissue associated with one or more target sites in the sino-nasal cavity.

The present invention utilizes the many benefits of flexible PCBs, as well as certain manufacturing techniques, to provide an end effector that is capable of highly conforming to anatomical variations within a sino-nasal cavity so that an operator can perform an accurate, minimally invasive, and localized application of energy to one or more target sites within the sino-nasal cavity of the patient to thereby treat a sino-nasal condition.

In particular, the underlying design of the end effector is unique. In a preferred embodiment, the end effector is multi-segmented and includes a proximal segment and a distal segment. The proximal and distal segments are constructed from single, unitary work pieces having elastic properties. More specifically, a single piece of shape memory material, such as nitinol, may be used to construct one or more portions of the proximal segment and further construct the distal segment in its entirety. For example, in one embodiment, the proximal segment is composed of a pair of interlocking members, while the distal segment is composed of a single member. The pair of interlocking members of the proximal segment include a first member providing a first set of support elements and a second member providing a second set of support members. As such, each of the first member and the second member may be constructed from a single workpiece and subsequently interlocked within one another to form the proximal segment, while the distal segment comprises a single component (as opposed to interlocking components), and is thus formed from a single workpiece.

The single workpiece may initially be in the form of a tube or a flat plate and can be laser cut to form the desired framework of support elements of the proximal and distal segments. In addition to reducing time, cost, and complexity, the use of laser machining allows a greater amount of design freedom for the manufacturer, in turn leading to a more tailored geometry and mechanical properties of a given segment of the end effector. For example, laser machining allows greater control over mechanical properties of the support elements, including tailoring the stiffness of a specific one of, or a given group of, support elements for a given segment, thereby allowing for tailoring of the tissue apposition profile when the given segment is in an expanded, deployed configuration. Furthermore, utilizing a stock workpiece in the form of a tube or flat plate results in support elements having a relatively flat surface upon which a corresponding flexible PCB member is affixed, thereby improving apposition of the PCB member to tissue within the sino-nasal cavity.

Furthermore, the use of flexible PCB members results in a greater amount of usable surface area than what is otherwise available with existing end effectors. The increase in surface area allows for a greater number of energy delivery elements to be introduced and utilized in a given procedure and further expands the possible number of patterns of such energy delivery elements. As a result, the contact surface increases substantially, thereby allowing for the end effector of the present invention to deliver treatment to certain areas within the sino-nasal cavity that may have been previously unreachable or untreatable with current treatment devices, or that previously required a surgeon to reposition a given device to reach such areas. The use of flexible PCB members also reduces the overall complexity with regard to manufacturing the end effector of the present invention. In particular, any given flexible PCB member (including an overall PCB assembly, which includes multiple PCB members) is constructed separately from the end effector, which includes constructing the overall electrode design and placement on a given PCB member. Once a PCB assembly is complete, PCB members are then attached to respective portions of a given segment of the end effector as a separate manufacturing step, thereby reducing the complexity that is otherwise associated with placing electrodes directly on the end effector, which is a common practice.

The invention provides improved manufacturing techniques for bonding the PCB members to support elements of a given proximal or distal segment. In particular, the present invention contemplates the use of bonding, thermal, and mechanical processes for joining PCB members to respective support elements, which may include one or more of adhesion, mechanical, lamination, polymer reflow, induction heating, spot welding, and laser welding processes. For example, in one embodiment, attaching a PCB member to a respective support element includes a reflow process in which a flexible PCB member is positioned relative to a respective support element, one or more polymer layers are then disposed around the flexible PCB member, and heat is then applied resulting in the one or more polymer layers encasing the flexible PCB member and subsequently affixing the flexible PCB member to the underlying support element of the end effector segment. In another embodiment, polymer sleeves may be secured to the support elements, thereby providing a substrate upon which a flexible PCB member may be positioned and subsequently attached. It should be noted that the process of securing the polymer sleeve and bonding the flexible PCB member to the support element may occur simultaneously via the application of pressure and heat.

In some embodiments, one or more of the support elements of the underlying proximal and distal segments of the end effector may further include fixation points that facilitate the attachment and alignment of the polymer sleeve and/or flexible PCB member thereto. For example, one or more of the support elements may include a recess, hole, notch, groove, etching, or the like to thereby increase surface area to receive an adhesive or pooling of melted polymer and mechanically secure the sleeve and/or PCB member in place ensuring alignment and simplifying assembly.

In this manner, the present invention provides an end effector that is capable of highly conforming to anatomical variations within a sino-nasal cavity so that an operator can perform an accurate, minimally invasive, and localized application of energy to one or more target sites within the sino-nasal cavity of the patient to thereby treat a sino-nasal condition. In particular, unlike other surgical treatments for rhinitis, the devices of the invention are minimally invasive. Once delivered within the sino-nasal cavity, each segment of the end effector can expand to a specific shape and/or size corresponding to anatomical structures within the sino-nasal cavity and associated with the target sites. More specifically, each of a first segment and a second segment includes a specific geometry when in a deployed configuration to complement anatomy of respective locations within the sino-nasal cavity. A plurality of flexible PCB members attached to the respective first and second segments are able to correspondingly move and transition into the specific geometry of the given segment, such that, once deployed, the flexible PCBs of the first and second segments contact and conform to a shape of the respective locations, including conforming to and complementing shapes of one or more anatomical structures at the respective locations.

In turn, the plurality of flexible PCB members of the first and second segments become accurately positioned within the sino-nasal cavity to subsequently deliver, via one or more electrodes, precise and focused application of energy to targeted tissue at the one or more target sites, to disrupt multiple neural signals to, and/or result in local hypoxia of, mucus producing and/or mucosal engorgement elements, thereby reducing production of mucus and/or mucosal engorgement within a nose of the patient and reducing or eliminate one or more symptoms associated with at least one of rhinitis, congestion, and rhinorrhea.

Accordingly, a handheld device of the present invention provides a user-friendly, non-invasive means of treating rhinosinusitis conditions, including precise and focused application of energy to the intended targeted tissue without causing collateral and unintended damage or disruption to other tissue and/or structures. Thus, the efficacy of a vidian neurectomy procedure can be achieved with the systems and methods of the present invention without the drawbacks discussed above. Most notably, the handheld device provides a surgeon with a user-friendly, non-invasive, and precise means for treating rhinorrhea and other symptoms of rhinosinusitis by targeting only those specific structures associated with such conditions, thereby ensuring that such treatment is effective at treating rhinosinusitis conditions while greatly reducing the risk of causing lateral damage or disruption to other tissue and/or structure thereby reducing the likelihood of unintended complications and side effects.

It should be noted that, although many of the embodiments are described with respect to devices, systems, and methods for therapeutically modulating nerves associated with the peripheral nervous system (PNS) and thus the treatment of peripheral neurological conditions or disorders, other applications and other embodiments in addition to those described herein are within the scope of the present disclosure. For example, at least some embodiments of the present disclosure may be useful for the treatment of other disorders, such as the treatment of disorders associated with the central nervous system.

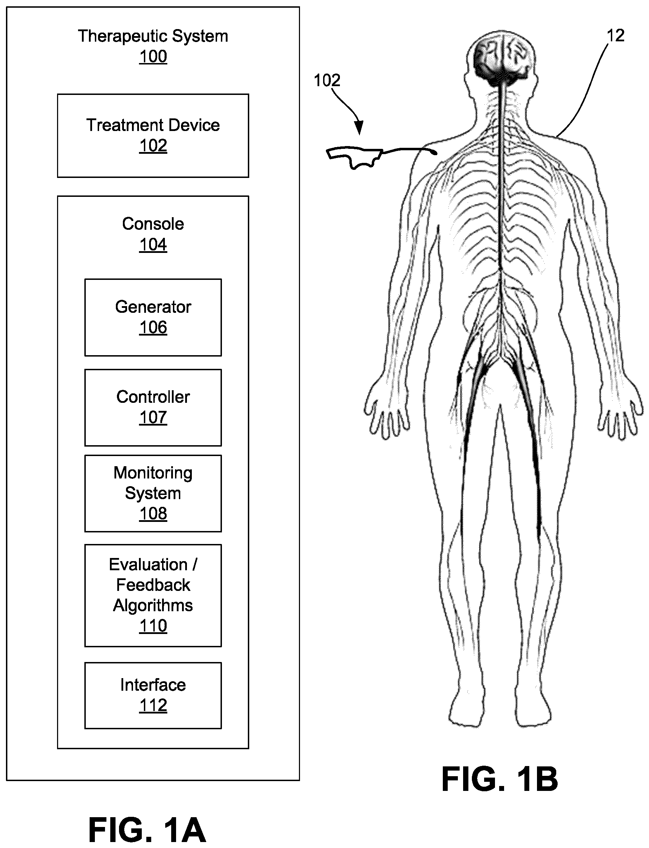

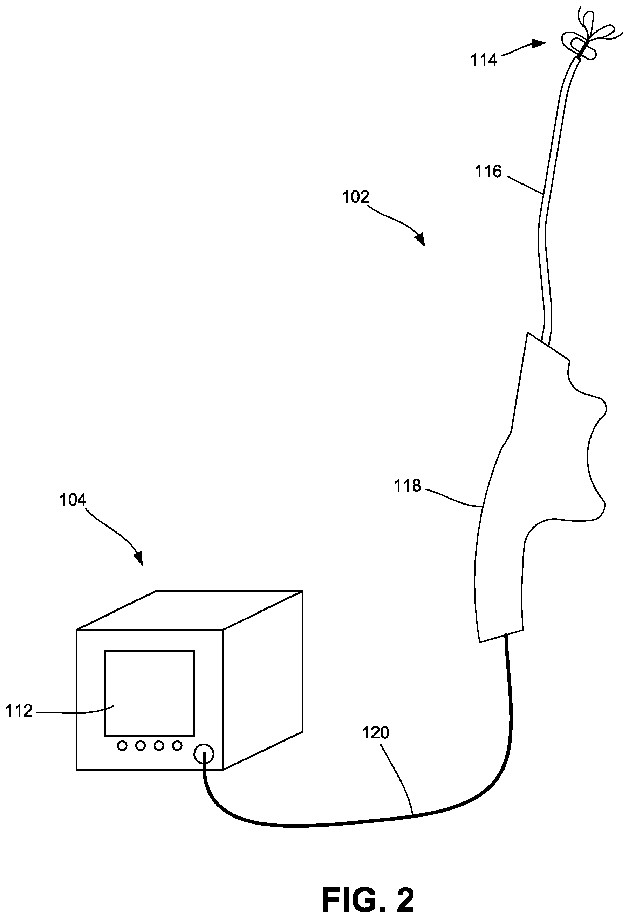

FIGS. 1A and 1B are diagrammatic illustrations of a therapeutic system 100 for treating a condition of a patient using a handheld device 102 according to some embodiments of the present disclosure. The system 100 generally includes a device 102 and a console 104 to which the device 102 is to be connected. FIG. 2 is a diagrammatic illustrations of the console 104 coupled to the handheld device 102 illustrating an exemplary embodiment of an end effector 114 for delivering energy to tissue at the one or more target sites of a patient for the treatment of a condition. As illustrated, the device 102 is a handheld device, which includes end effector 114, a shaft 116 operably associated with the end effector 114, and a handle 118 operably associated with the shaft 116. The end effector 114 may be collapsible/retractable and expandable, thereby allowing for the end effector 114 to be minimally invasive (i.e., in a collapsed or retracted state) upon delivery to one or more target sites within a patient and then expanded once positioned at the target site. It should be noted that the terms "end effector" and "therapeutic assembly" may be used interchangeably throughout this disclosure.

For example, a surgeon or other medical professional performing a procedure can utilize the handle 118 to manipulate and advance the shaft 116 to a desired target site, wherein the shaft 116 is configured to locate at least a distal portion thereof intraluminally at a treatment or target site within a portion of the patient associated with tissue to undergo electrotherapeutic stimulation for subsequent treatment of an associated condition or disorder. In the event that the tissue to be treated is a nerve, such that electrotherapeutic stimulation thereof results in treatment of an associated neurological condition, the target site may generally be associated with peripheral nerve fibers. The target site may be a region, volume, or area in which the target nerves are located and may differ in size and shape depending upon the anatomy of the patient. Once positioned, the end effector 114 may be deployed and subsequently deliver energy to the one or more target sites. The energy delivered may be non-therapeutic stimulating energy at a frequency for locating neural tissue and further sensing one or more properties of the neural tissue. For example, the end effector 114 may include an electrode array, which includes at least a subset of electrodes configured to sense the presence of neural tissue at a respective position of each of the electrodes, as well as morphology of the neural tissue, wherein such data may be used for determining, via the console 104, the type of neural tissue, depth of neural tissue, and location of neural tissue.

Based on the identification of the neural tissue type, the console 104 is configured to determine a specific treatment pattern for controlling delivery of energy from the end effector 114 upon the target site at a specific level for a specific period of time to the tissue of interest (i.e., the targeted tissue) sufficient to ensure successful ablation/modulation of the targeted tissue while minimizing and/or preventing collateral damage to surrounding or adjacent non-targeted tissue at the target site. Accordingly, the end effector 114 is able to therapeutically modulate nerves of interest, particularly nerves associated with a peripheral neurological conditional or disorder so as to treat such condition or disorder, while minimizing and/or preventing collateral damage.

For example, the end effector 114 may include at least one energy delivery element, such as an electrode, configured to delivery energy to the target tissue which may be used for sensing presence and/or specific properties of tissue (such tissue including, but not limited to, muscle, nerves, blood vessels, bones, etc.) for therapeutically modulating tissues of interest, such as neural tissue. For example, one or more electrodes may be provided by one or more portions of the end effector 114, wherein the electrodes may be configured to apply electromagnetic neuromodulation energy (e.g., radiofrequency (RF) energy) to target sites. In other embodiments, the end effector 114 may include other energy delivery elements configured to provide therapeutic neuromodulation using various other modalities, such as cryotherapeutic cooling, ultrasound energy (e.g., high intensity focused ultrasound ("HIFU") energy), microwave energy (e.g., via a microwave antenna), direct heating, high and/or low power laser energy, mechanical vibration, and/or optical power.

In some embodiments, the end effector 114 may include one or more sensors (not shown), such as one or more temperature sensors (e.g., thermocouples, thermistors, etc.), impedance sensors, and/or other sensors. The sensors and/or the electrodes may be connected to one or more wires extending through the shaft 116 and configured to transmit signals to and from the sensors and/or convey energy to the electrodes.

As shown, the device 102 is operatively coupled to the console 104 via a wired connection, such as cable 120. It should be noted, however, that the device 102 and console 104 may be operatively coupled to one another via a wireless connection. The console 104 is configured to provide various functions for the device 102, which may include, but is not limited to, controlling, monitoring, supplying, and/or otherwise supporting operation of the device 102. For example, when the device 102 is configured for electrode-based, heat-element-based, and/or transducer-based treatment, the console 104 may include an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy.

In some embodiments, the console 104 may include a controller 107 communicatively coupled to the device 102. However, in the embodiments described herein, the controller 107 may generally be carried by and provided within the handle 118 of the device 102. The controller 107 is configured to initiate, terminate, and/or adjust operation of one or more electrodes provided by the end effector 114 directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via evaluation/feedback algorithms 110. For example, the evaluation/feedback algorithms 110 can be configured to provide information associated with the location of nerves at the treatment site, the temperature of the tissue at the treatment site, and/or the effect of the therapeutic neuromodulation on the nerves at the treatment site. In certain embodiments, the evaluation/feedback algorithm 110 can include features to confirm efficacy of the treatment and/or enhance the desired performance of the system 100. For example, the evaluation/feedback algorithm 110, in conjunction with the controller 107, can be configured to monitor temperature at the treatment site during therapy and automatically shut off the energy delivery when the temperature reaches a predetermined maximum (e.g., when applying RF energy) or predetermined minimum (e.g., when applying cryotherapy). In other embodiments, the evaluation/feedback algorithm 110, in conjunction with the controller 107, can be configured to automatically terminate treatment after a predetermined maximum time, a predetermined maximum impedance rise of the targeted tissue (i.e., in comparison to a baseline impedance measurement), a predetermined maximum impedance of the targeted tissue, and/or other threshold values for biomarkers associated with autonomic function. This and other information associated with the operation of the system 100 can be communicated to the operator via a graphical user interface (GUI) 112 provided via a display on the console 104 and/or a separate display (not shown) communicatively coupled to the console 104, such as a tablet or monitor, to thereby provide visual and/or audible alerts to the operator. The GUI 112 may generally provide operational instructions for the procedure, such as directing the operator to select which sino-nasal cavity to treat, indicating when the device 102 is primed and ready to perform treatment, and further providing status of therapy during the procedure, including indicating when the treatment is complete.

For example, in some embodiments, the end effector 114and/or other portions of the system 100 can be configured to detect various parameters of the heterogeneous tissue at the target site to determine the anatomy at the target site (e.g., tissue types, tissue locations, vasculature, bone structures, foramen, sinuses, etc.), locate nerves and/or other structures, and allow for neural mapping. For example, the end effector 114 may be configured to detect impedance, dielectric properties, temperature, and/or other properties that indicate the presence of neural fibers in the target region.

As shown in FIG. 1A, the console 104 further includes a monitoring system 108 configured to receive data from the end effector 114 (i.e., detected electrical and/or thermal measurements of tissue at the target site), specifically sensed by appropriate sensors (e.g., temperature sensors and/or impedance sensors, or the like), and process this information to identify the presence of nerves, the location of nerves, neural activity at *the* target site, and/or other properties of the neural tissue, such a physiological properties (e.g., depth), bioelectric properties, and thermal properties. The nerve monitoring system 108 can be operably coupled to the electrodes and/or other features of the end effector 114 via signal wires (e.g., copper wires) that extend through the cable 120 and through the length of the shaft 116. In other embodiments, the end effector 114 can be communicatively coupled to the nerve monitoring system 108 using other suitable communication means.

The nerve monitoring system 108 can determine neural locations and activity before therapeutic neuromodulation to determine precise treatment regions corresponding to the positions of the desired nerves. The nerve monitoring system 108 can further be used during treatment to determine the effect of the therapeutic neuromodulation, and/or after treatment to evaluate whether the therapeutic neuromodulation treated the target nerves to a desired degree. This information can be used to make various determinations related to the nerves proximate to the target site, such as whether the target site is suitable for neuromodulation. In addition, the nerve monitoring system 108 can also compare the detected neural locations and/or activity before and after therapeutic neuromodulation, and compare the change in neural activity to a predetermined threshold to assess whether the application of therapeutic neuromodulation was effective across the treatment site. For example, the nerve monitoring system 108 can further determine electroneurogram (ENG) signals based on recordings of electrical activity of neurons taken by the end effector 114 before and after therapeutic neuromodulation. Statistically meaningful (e.g., measurable or noticeable) decreases in the ENG signal(s) taken after neuromodulation can serve as an indicator that the nerves were sufficiently ablated. Additional features and functions of the nerve monitoring system 108, as well as other functions of the various components of the console 104, including the evaluation/feedback algorithms 110 for providing real-time feedback capabilities for ensuring optimal therapy for a given treatment is administered, are described in at least U.S. Publication No. 2016/0331459 and U.S. Publication No. 2018/0133460, the contents of each of which are incorporated by reference herein in their entireties.

The device 102 provides access to target sites associated with peripheral nerves for the subsequent neuromodulation of such nerves and treatment of a corresponding peripheral neurological condition or disorder. The peripheral nervous system is one of two components that make up the nervous system of bilateral animals, with the other part being the central nervous system (CNS). The PNS consists of the nerves and ganglia outside the brain and spinal cord. The main function of the PNS is to connect the CNS to the limbs and organs, essentially serving as a relay between the brain and spinal cord and the rest of the body. The peripheral nervous system is divided into the somatic nervous system and the autonomic nervous system. In the somatic nervous system, the cranial nerves are part of the PNS with the exception of the optic nerve (cranial nerve II), along with the retina. The second cranial nerve is not a true peripheral nerve but a tract of the diencephalon. Cranial nerve ganglia originated in the CNS. However, the remaining ten cranial nerve axons extend beyond the brain and are therefore considered part of the PNS. The autonomic nervous system exerts involuntary control over smooth muscle and glands. The connection between CNS and organs allows the system to be in two different functional states: sympathetic and parasympathetic. Accordingly, the devices, systems, and methods of the present invention are useful in detecting, identifying, and precision targeting nerves associated with the peripheral nervous system for treatment of corresponding peripheral neurological conditions or disorders.

The peripheral neurological conditions or disorders may include, but are not limited to, chronic pain, movement disorders, epilepsy, psychiatric disorders, cardiovascular disorders, gastrointestinal disorders, genitourinary disorders, to name a few. For example, chronic pain may include headaches, complex regional pain syndrome, neuropathy, peripheral neuralgia, ischemic pain, failed back surgery syndrome, and trigeminal neuralgia. The movement disorders may include spasticity, Parkinson's disease, tremor, dystonia, Tourette syndrome, camptocormia, hemifacial spasm, and Meige syndrome. The psychiatric disorders may include depression, obsessive compulsive disorder, drug addiction, and anorexia/eating disorders. The functional restoration may include restoration of certain functions post traumatic brain injury, hearing impairment, and blindness. The cardiovascular disorders may include angina, heart failure, hypertension, peripheral vascular disorders, and stroke. The gastrointestinal disorders may include dysmotility and obesity. The genitourinary disorders may include painful bladder syndrome, interstitial cystitis, and voiding dysfunction.

For example, the system 100 may be used for the treatment of a cardiovascular disorder, such as arrhythmias or heart rhythm disorders, including, but not limited to, atrial fibrillation (AF or A-fib). Atrial fibrillation is an irregular and often rapid heart rate that can increase one's risk of stroke, heart failure, and other heart-related complications. Atrial fibrillation occurs when regions of cardiac tissue abnormally conduct electric signals to adjacent tissue, thereby disrupting the normal cardiac cycle and causing asynchronous rhythm. Atrial fibrillation symptoms often include heart palpitations, shortness of breath, and weakness. While episodes of atrial fibrillation can come and go, a person may develop atrial fibrillation that doesn't go away and thus will require treatment. Although atrial fibrillation itself usually isn't life-threatening, it is a serious medical condition that sometimes requires emergency treatment, as it may lead to complications. For example, atrial fibrillation is associated with an increased risk of heart failure, dementia, and stroke.

The normal electrical conduction system of the heart allows the impulse that is generated by the sinoatrial node (SA node) of the heart to be propagated to and stimulate the myocardium (muscular layer of the heart). When the myocardium is stimulated, it contracts. It is the ordered stimulation of the myocardium that allows efficient contraction of the heart, thereby allowing blood to be pumped to the body. In AF, the normal regular electrical impulses generated by the sinoatrial node in the right atrium of the heart are overwhelmed by disorganized electrical impulses usually originating in the roots of the pulmonary veins. This leads to irregular conduction of ventricular impulses that generate the heartbeat. In particular, during AF, the heart's two upper chambers (the atria) beat chaotically and irregularly, out of coordination with the two lower chambers (the ventricles) of the heart.

During atrial fibrillation, the regular impulses produced by the sinus node for a normal heartbeat are overwhelmed by rapid electrical discharges produced in the atria and adjacent parts of the pulmonary veins. Sources of these disturbances are either automatic foci, often localized at one of the pulmonary veins, or a small number of localized sources in the form of either a reentrant leading circle, or electrical spiral waves (rotors). These localized sources may be found in the left atrium near the pulmonary veins or in a variety of other locations through both the left or right atrium. There are three fundamental components that favor the establishment of a leading circle or a rotor: 1) slow conduction velocity of cardiac action potential; 2) short refractory period; and 3) small wavelength. Wavelength is the product of velocity and refractory period. If the action potential has fast conduction, with a long refractory period and/or conduction pathway shorter than the wavelength, an AF focus would not be established. In multiple wavelet theory, a wavefront will break into smaller daughter wavelets when encountering an obstacle, through a process called vortex shedding; but under proper conditions, such wavelets can reform and spin around a center, forming an AF focus.

The system 100 provides for the treatment of AF, in which the device 102 may provide access to and provide treatment of one or more target sites associated with nerves that correspond to, or are otherwise associated with, treating AF. For example, the device 102, in conjunction with the console 104, may detect, identify, and precision target cardiac tissue and subsequently deliver energy at a level or frequency sufficient to therapeutically modulate nerves associated with such cardiac tissue. The therapeutic modulation of such nerves is sufficient to disrupt the origin of the signals causing the AF and/or disrupt the conducting pathway for such signals.

Similar to the conduction system of the heart, a neural network exists which surrounds the heart and plays an important role in formation of the substrate of AF and when a trigger is originated, usually from pulmonary vein sleeves, AF occurs. This neural network includes ganglionated plexi (GP) located adjacent to pulmonary vein ostia which are under control of higher centers in normal people. For example, the heart is richly innervated by the autonomic nerves. The ganglion cells of the autonomic nerves are located either outside the heart (extrinsic) or inside the heart (intrinsic). Both extrinsic and intrinsic nervous systems are important for cardiac function and arrhythmogenesis. The vagal nerves include axons that come from various nuclei in the medulla. The extrinsic sympathetic nerves come from the paravertebral ganglia, including the superior cervical ganglion, middle cervical ganglion, the cervicothoracic (stellate) ganglion and the thoracic ganglia. The intrinsic cardiac nerves are found mostly in the atria, and are intimately involved in atrial arrhythmogenesis cardiovascular disorder, such as arrhythmias or heart rhythm disorders, including, but not limited to, atrial fibrillation. When GP become hyperactive owing to loss of inhibition from higher centers (e.g., in elderly), AF can occur.

The system 100 can be used to control hyperactive GP either by stimulating higher centers and their connections, such as vagus nerve stimulation, or simply by ablating GP. Accordingly, the device 102, in conjunction with the console 104, may detect and identify ganglionated plexus (GP) and further determine an energy level sufficient to therapeutically modulate or treat (i.e., ablate) the GP for the treatment of AF (i.e., surgically disrupting the origin of the signals causing the AF and disrupting the conducting pathway for such signals) while minimizing and/or preventing collateral damage to surrounding or adjacent non-neural tissue including bloods vessels and bone and non-targeted neural tissue. It should be noted that other nerves and/or cardiac tissue, or other structures, known to have an impact on or cause AF, may be targeted by the system 100, including, but not limited to, pulmonary veins (e.g., pulmonary vein isolation upon creation of lesions around PV ostia to prevent triggers from reaching atrial substrate).

In addition to treating arrhythmias, the system 100 may also be used for the treatment of other cardiovascular-related conditions, particularly those involving the kidney. The kidneys play a significant role in the progression of CHF, as well as in Chronic Renal Failure (CRF), End-Stage Renal Disease (ESRD), hypertension (pathologically high blood pressure), and other cardio-renal diseases.

The functions of the kidney can be summarized under three broad categories: filtering blood and excreting waste products generated by the body's metabolism; regulating salt, water, electrolyte and acid-base balance; and secreting hormones to maintain vital organ blood flow. Without properly functioning kidneys, a patient will suffer water retention, reduced urine flow and an accumulation of waste toxins in the blood and body. These conditions resulting from reduced renal function or renal failure (kidney failure) are believed to increase the workload of the heart.

For example, in a CHF patient, renal failure will cause the heart to further deteriorate as the water build-up and blood toxins accumulate due to the poorly functioning kidneys and, in turn, cause the heart further harm. CHF is a condition that occurs when the heart becomes damaged and reduces blood flow to the organs of the body. If blood flow decreases sufficiently, kidney function becomes impaired and results in fluid retention, abnormal hormone secretions and increased constriction of blood vessels. These results increase the workload of the heart and further decrease the capacity of the heart to pump blood through the kidney and circulatory system. This reduced capacity further reduces blood flow to the kidney. It is believed that progressively decreasing perfusion of the kidney is a principal non-cardiac cause perpetuating the downward spiral of CHF. Moreover, the fluid overload and associated clinical symptoms resulting from these physiologic changes are predominant causes for excessive hospital admissions, reduced quality of life, and overwhelming costs to the health care system due to CHF.

End-stage renal disease is another condition at least partially controlled by renal neural activity. There has been a dramatic increase in patients with ESRD due to diabetic nephropathy, chronic glomerulonephritis and uncontrolled hypertension. Chronic renal failure (CRF) slowly progresses to ESRD. CRF represents a critical period in the evolution of ESRD. The signs and symptoms of CRF are initially minor, but over the course of 2-5 years, become progressive and irreversible. While some progress has been made in combating the progression to, and complications of, ESRD, the clinical benefits of existing interventions remain limited.

Arterial hypertension is a major health problem worldwide. Treatment-resistant hypertension is defined as the failure to achieve target blood pressure despite the concomitant use of maximally tolerated doses of three different antihypertensive medications, including a diuretic. Treatment-resistant hypertension is associated with considerable morbidity and mortality. Patients with treatment-resistant hypertension have markedly increased cardiovascular morbidity and mortality, facing an increase in the risk of myocardial infarction (MI), stroke, and death compared to patients whose hypertension is adequately controlled.

The autonomic nervous system is recognized as an important pathway for control signals that are responsible for the regulation of body functions critical for maintaining vascular fluid balance and blood pressure. The autonomic nervous system conducts information in the form of signals from the body's biologic sensors such as baroreceptors (responding to pressure and volume of blood) and chemoreceptors (responding to chemical composition of blood) to the central nervous system via its sensory fibers. It also conducts command signals from the central nervous system that control the various innervated components of the vascular system via its motor fibers.

It is known from clinical experience and research that an increase in renal sympathetic nerve activity leads to vasoconstriction of blood vessels supplying the kidney, decreased renal blood flow, decreased removal of water and sodium from the body, and increased renin secretion. It is also known that reduction of sympathetic renal nerve activity, e.g., via denervation, may reverse these processes.

The renal sympathetic nervous system plays a critical influence in the pathophysiology of hypertension. The adventitia of the renal arteries has efferent and afferent sympathetic nerves. Renal sympathetic activation via the efferent nerves initiates a cascade resulting in elevated blood pressure. Efferent sympathetic outflow leads to vasoconstriction with a subsequent reduction in glomerular blood flow, a lowering of the glomerular filtration rate, release of renin by the juxtaglomerular cells, and the subsequent activation of the renin-angiotensin-aldosterone axis leading to increased tubular reabsorption of sodium and water. Decreased glomerular filtration rate also prompts additional systemic sympathetic release of catecholamines. As a consequence, blood pressure increases by a rise in total blood volume and increased peripheral vascular resistance.

The system 100 can be used for the treatment of cardio-renal diseases, including hypertension, by providing renal neuromodulation and/or denervation. For example, the device 102 may be placed at one or more target sites associated with renal nerves other neural fibers that contribute to renal neural function, or other neural features. For example, the device 102, in conjunction with the console 104, may detect, identify, and precision target renal nerve tissue and subsequently deliver energy at a level or frequency sufficient to therapeutically modulate nerves associated with such renal tissue. The therapeutic modulation of such renal nerves and/or renal tissue is sufficient to completely block or denervate the target neural structures and/or disrupt renal nervous activity, while minimizing and/or preventing collateral damage to surrounding or adjacent non-neural tissue including bloods vessels and bone and non-targeted neural tissue.

It should further be noted that the system 100 can be used to determine disease progression. In particular, the present system 100 can obtain measurements at one or more target sites associated with a given disease, disorder, or the like. Such measurements may be based on the active neural parameters (i.e., neuronal firing and active voltage monitoring) and may be used to identify neurons. The active neural parameters (and thus behavior) change with disease progression, thereby allowing the present system to identify such changes and determine a progression of the underlying disease or disorder. Such capabilities are possible based, at least in part, on the fact that the present system 100 is configured to monitor passive electric phenomena (i.e., the present system 100 determines the ohmic conductivity frequency, which remains consistent, while conductivity will be different based on disease or disorder progression).

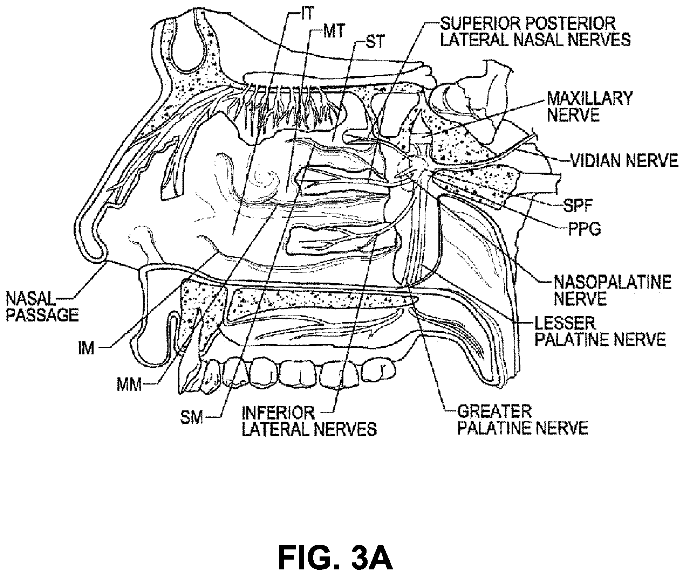

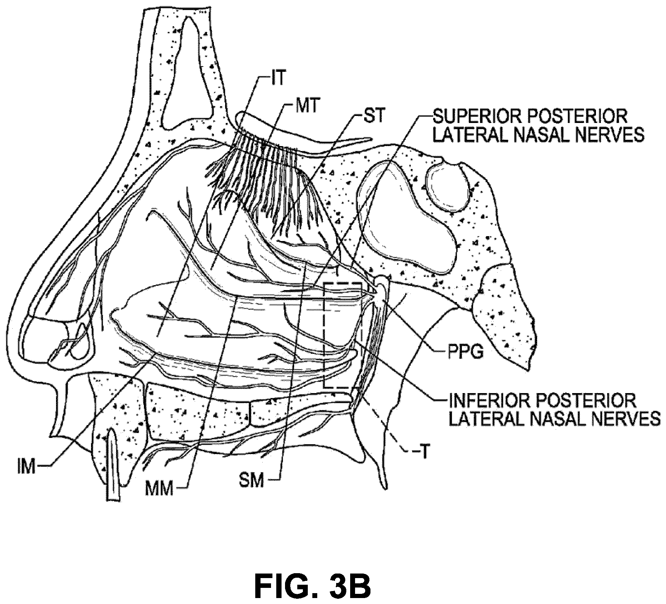

FIG. 3A is a cut-away side view illustrating the anatomy of a lateral sino-nasal wall and FIG. 3B is an enlarged side view of the nerves of the lateral sino-nasal wall of FIG. 1A. The sphenopalatine foramen (SPF) is an opening or conduit defined by the palatine bone and the sphenoid bone through which the sphenopalatine vessels and the posterior superior sino-nasal nerves travel into the sino-nasal cavity. More specifically, the orbital and sphenoidal processes of the perpendicular plate of the palatine bone define the sphenopalatine notch, which is converted into the SPF by the articulation with the surface of the body of the sphenoid bone.

The location of the SPF is highly variable within the posterior region of the lateral sino-nasal cavity, which makes it difficult to visually locate the SPF. Typically, the SPF is located in the middle meatus (MM). However, anatomical variations also result in the SPF being located in the superior meatus (SM) or at the transition of the superior and middle meatuses. In certain individuals, for example, the inferior border of the SPF has been measured at about 19 mm above the horizontal plate of the palatine bone (i.e., the sino-nasal sill), which is about 13 mm above the horizontal lamina of the inferior turbinate (IT) and the average distance from the sino-nasal sill to the SPF is about 64.4 mm, resulting in an angle of approach from the sino-nasal sill to the SPA of about 11.4°. However, studies to measure the precise location of the SPF are of limited practical application due to the wide variation of its location.

The anatomical variations of the SPF are expected to correspond to alterations of the autonomic and vascular pathways traversing into the sino-nasal cavity. In general, it is thought that the posterior sino-nasal nerves (also referred to as lateral posterior superior sino-nasal nerves) branch from the pterygopalatine ganglion (PPG), which is also referred to as the sphenopalatine ganglion, through the SPF to enter the lateral sino-nasal wall of the sino-nasal cavity, and the sphenopalatine artery passes from the pterygopalatine fossa through the SPF on the lateral sino-nasal wall. The sphenopalatine artery branches into two main portions: the posterior lateral sino-nasal branch and the posterior septal branch. The main branch of the posterior lateral sino-nasal artery travels inferiorly into the inferior turbinate IT (e.g., between about 1.0 mm and 1.5 mm from the posterior tip of the inferior turbinate IT), while another branch enters the middle turbinate MT and branches anteriorly and posteriorly.

Beyond the SPF, studies have shown that over 30% of human patients have one or more accessory foramen that also carries arteries and nerves into the sino-nasal cavity. The accessory foramen are typically smaller than the SPF and positioned inferior to the SPF. For example, there can be one, two, three or more branches of the posterior sino-nasal artery and posterior sino-nasal nerves that extend through corresponding accessory foramen. The variability in location, size, and quantity associated with the accessory foramen and the associated branching arteries and nerves that travel through the accessory foramen gives rise to a great deal of uncertainty regarding the positions of the vasculature and nerves of the sphenopalatine region. Furthermore, the natural anatomy extending from the SPF often includes deep inferior and/or superior grooves that carry neural and arterial pathways, which make it difficult to locate arterial and neural branches. For example the grooves can extend more than 5 mm long, more than 2 mm wide, and more than 1 mm deep, thereby creating a path significant enough to carry both arteries and nerves. The variations caused by the grooves and the accessory foramen in the sphenopalatine region make locating and accessing the arteries and nerves (positioned posterior to the arteries) extremely difficult for surgeons.

Recent microanatomic dissection of the pterygopalatine fossa (PPF) have further evidenced the highly variable anatomy of the region surrounding the SPF, showing that a multiplicity of efferent rami that project from the pterygopalatine ganglion (PPG) to innervate the orbit and sino-nasal mucosa via numerous groups of small nerve fascicles, rather than an individual postganglionic autonomic nerves (e.g., the posterior sino-nasal nerve). Studies have shown that at least 87% of humans have microforamina and micro rami in the palatine bone.

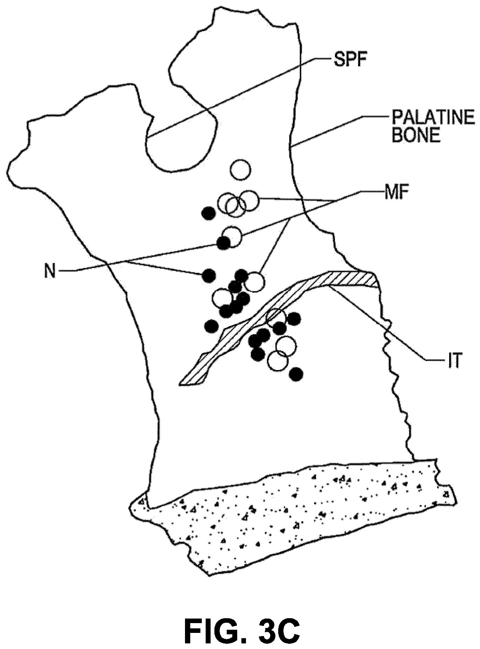

FIG. 3C, for example, is a front view of a left palatine bone illustrating geometry of microforamina and micro rami in a left palatine bone. In FIG. 3C, the solid regions represent nerves traversing directly through the palatine bone, and the open circles represent nerves that were associated with distinct microforamina. As such, FIG. 3C illustrates that a medial portion of the palatine bone can include at least 25 accessory posterolateral nerves.

The respiratory portion of the sino-nasal cavity mucosa is composed of a type of ciliated pseudostratified columnar epithelium with a basement membrane. sino-nasal secretions (e.g., mucus) are secreted by goblet cells, submucosal glands, and transudate from plasma. sino-nasal seromucous glands and blood vessels are highly regulated by parasympathetic innervation deriving from the vidian and other nerves. Parasympathetic (cholinergic) stimulation through acetylcholine and vasoactive intestinal peptide generally results in mucus production. Accordingly, the parasympathetic innervation of the mucosa is primarily responsible submucosal gland activation/hyper activation, venous engorgement (e.g., congestion), and increased blood flow to the blood vessels lining the nose. Accordingly, severing or modulating the parasympathetic pathways that innervate the mucosa are expected to reduce or eliminate the hyper activation of the submucosal glands and engorgement of vessels that cause symptoms associated with rhinosinusitis and other indications.

As previously described herein, postganglionic parasympathetic fibers that innervate the sino-nasal mucosa (i.e., posterior superior sino-nasal nerves) were thought to travel exclusively through the SPF as a sphenopalatine neurovascular bundle. The posterior sino-nasal nerves are branches of the maxillary nerve that innervate the sino-nasal cavity via a number of smaller medial and lateral branches extending through the mucosa of the superior and middle turbinates ST, MT (i.e., sino-nasal conchae) and to the sino-nasal septum. The nasopalatine nerve is generally the largest of the medial posterior superior sino-nasal nerves, and it passes anteroinferiorly in a groove on the vomer to the floor of the sino-nasal cavity. From here, the nasopalatine nerve passes through the incisive fossa of the hard palate and communicates with the greater palatine nerve to supply the mucosa of the hard palate. The posterior superior sino-nasal nerves pass through the pterygopalatine ganglion PPG without synapsing and onto the maxillary nerve via its ganglionic branches.

Based on the understanding that the posterior sino-nasal nerves exclusively traverse the SPF to innervate the sino-nasal mucosa, surgeries have been performed to selectively sever the posterior sino-nasal nerve as it exits the SPF. However, as discussed above, the sinonasal parasympathetic pathway actually comprises individual rami project from the pterygopalatine ganglion (PPG) to innervate the sino-nasal mucosa via multiple small nerve fascicles (i.e., accessory posterolateral nerves), not a single branch extending through the SPF. These rami are transmitted through multiple fissures, accessory foramina, and microforamina throughout the palatine bone and may demonstrate anastomotic loops with both the SPF and other accessory nerves. Thus, if only the parasympathetic nerves traversing the SPF were severed, almost all patients (e.g., 90% of patients or more) would retain intact accessory secretomotor fibers to the posterolateral mucosa, which would result in the persistence of symptoms the neurectomy was meant to relieve.

Accordingly, embodiments of the present disclosure are configured to therapeutically modulate nerves at precise and focused treatment sites corresponding to the sites of rami extending through fissures, accessory foramina, and microforamina throughout the palatine bone (e.g., target region T shown in FIG. 3B). In certain embodiments, the targeted nerves are postganglionic parasympathetic nerves that go on to innervate the sino-nasal mucosa. This selective neural treatment is also expected to decrease the rate of postoperative sino-nasal crusting and dryness because it allows a clinician to titrate the degree of anterior denervation through judicious sparing of the rami orbitonasal. Furthermore, embodiments of the present disclosure are also expected to maintain at least some sympathetic tone by preserving a portion of the sympathetic contributions from the deep petrosal nerve and internal maxillary periarterial plexus, leading to improved outcomes with respect to sino-nasal obstruction. In addition, embodiments of the present disclosure are configured to target a multitude of parasympathetic neural entry locations (e.g., accessory foramen, fissures, and microforamina) to the sino-nasal region to provide for a complete resection of all anastomotic loops, thereby reducing the rate of long-term re-innervation.

FIG. 4 is a side view of one embodiment of a handheld device for providing therapeutic neuromodulation consistent with the present disclosure.

As illustrated, the device 102 includes a multi-segment end effector 114 transformable between a retracted configuration and an expanded deployed configuration, a shaft 116 operably associated with the end effector 114, and a handle 118 operably associated with the shaft 116. The multi-segment end effector 114 includes at least a first segment 122 and a second segment 124 spaced apart from one another. The first segment 122 is generally positioned closer to a distal end of the shaft 116, and is thus sometimes referred to herein as the proximal segment 122, while the second segment 124 is generally positioned further from the distal end of the shaft 116 and is thus sometimes referred to herein as the distal segment 124. Each of the first and second segments 122 and 124 is transformable between a retracted configuration, which includes a low-profile delivery state to facilitate intraluminal delivery of the end effector 114 to a treatment site within the sino-nasal region, and a deployed configuration, which includes an expanded state, as shown in FIG. 4 and further illustrated in FIGS. 5A-5F. The handle 118 includes at least a first mechanism 126 for deployment of the multi-segment end effector 114, notably the first and second segments 122, 124, from the retracted configuration to the deployed configuration and a second mechanism 128, separate from the first mechanism 124, for control of energy output by either of the first and second segments 122, 124 of the end effector 114, specifically electrodes or other energy elements provided by first and/or second segments 122, 124. The handheld device 102 may further include an auxiliary line 121, which may provide a fluid connection between a fluid source, for example, and the shaft 116 such that fluid may be provided to a target site via the distal end of the shaft 116. In some embodiments, the auxiliary line 121 may provide a connection between a vacuum source and the shaft 116, such that the device 102 may include suction capabilities (via the distal end of the shaft 116).

FIGS. 5A, 5B, 5C, 5D, 5E, and 5F are enlarged views of the multi-segment end effector 114, illustrating various views of the first and second segments 122, 124 in greater detail. FIG. 5A is an enlarged, perspective view of the multi-segment end effector 114. FIG. 5B is an exploded, perspective view of the multi-segment end effector 114. FIGS. 5C and 5D are enlarged, top and side views, respectively, of the multi-segment end effector 114. FIG. 5E is an enlarged, front (proximal facing) view of the first segment 122 of the multi-segment end effector 114. FIG. 5F is an enlarged, front (proximal facing) view of the second segment 124 of the multi-segment end effector 114.

As illustrated, the first segment 122 includes at least a first set of flexible support elements, generally in the form of wires, arranged in a first configuration, and the second segment 124 includes a second set of flexible support elements, also in the form of wires, arranged in a second configuration. The first and second sets of flexible support elements include composite wires having conductive and elastic properties. For example, in some embodiments, the composite wires include a shape memory material, such as nitinol. The flexible support elements may further include a highly lubricious coating, which may allow for desirable electrical insulation properties as well as desirable low friction surface finish. Each of the first and second segments 122, 124 is transformable between a retracted configuration and an expanded deployed configuration such that the first and second sets of flexible support elements are configured to position one or more electrodes provided on the respective segments (see electrodes 136 in FIGS. 5E and 5F) into contact with one or more target sites when in the deployed configuration.

As shown, when in the expanded deployed configuration, the first set of support elements of the first segment 122 includes at least a first pair of struts 130a, 130b, each comprising a loop (or leaflet) shape and extending in an upward direction and a second pair of struts 132a, 132b, each comprising a loop (or leaflet) shape and extending in a downward direction, generally in an opposite direction relative to at least the first pair of struts 130a, 130b. It should be noted that the terms upward and downward are used to describe the orientation of the first and second segments 122, 124 relative to one another. More specifically, the first pair of struts 130a, 130b generally extend in an outward inclination in a first direction relative to a longitudinal axis of the multi-segment end effector 114 and are spaced apart from one another. Similarly, the second pair of struts 132a, 132b extend in an outward inclination in a second direction substantially opposite the first direction relative to the longitudinal axis of the multi-segment end effector and spaced apart from one another.

The second set of support elements of the second segment 124, when in the expanded deployed configuration, includes a second set of struts 134(1), 134(2), 134(n) (approximately six struts), each comprising a loop shape extending outward to form an open-ended circumferential shape. As shown, the open-ended circumferential shape generally resembles a blooming flower, wherein each looped strut 134 may generally resemble a flower petal. It should be noted that the second set of struts 134 may include any number of individual struts and is not limited to six, as illustrated. For example, in some embodiments, the second segment 124 may include two, three, four, five, six, seven, eight, nine, ten, or more struts 134.

The first and second segments 122, 124, specifically struts 130, 132, and 134 include one or more energy delivery elements, such as a plurality of electrodes 136. It should be noted that any individual strut may include any number of electrodes 136 and is not limited to one electrode, as shown. In the expanded state, the struts 130, 132, and 134 can position any number of electrodes 136 against tissue at a target site within the sino-nasal region (e.g., proximate to the palatine bone inferior to the SPF). The electrodes 136 can apply bipolar or multi-polar radiofrequency (RF) energy to the target site to therapeutically modulate postganglionic parasympathetic nerves that innervate the sino-nasal mucosa proximate to the target site. In various embodiments, the electrodes 136 can be configured to apply pulsed RF energy with a desired duty cycle (e.g., 1 second on/0.5 seconds off) to regulate the temperature increase in the target tissue.

The first and second segments 122, 124 and the associated struts 130, 132, and 134 can have sufficient rigidity to support the electrodes 136 and position or press the electrodes 136 against tissue at the target site. In addition, each of the expanded first and second segments 122, 124 can press against surrounding anatomical structures proximate to the target site (e.g., the turbinates, the palatine bone, etc.) and the individual struts 130, 132, 134 can at least partially conform to the shape of the adjacent anatomical structures to anchor the end effector 114 In addition, the expansion and conformability of the struts 130, 132, 134 can facilitate placing the electrodes 136 in contact with the surrounding tissue at the target site. The electrodes 136 can be made from platinum, iridium, gold, silver, stainless steel, platinum-iridium, cobalt chromium, iridium oxide, polyethylenedioxythiophene (PEDOT), titanium, titanium nitride, carbon, carbon nanotubes, platinum grey, Drawn Filled Tubing (DFT) with a silver core, and/or other suitable materials for delivery RF energy to target tissue. In some embodiments, such as illustrated in FIG. 6, a strut may include an outer jacket surrounding a conductive wire, wherein portions of the outer jacket are selectively absent along a length of the strut, thereby exposing the underlying conductive wire so as to act as an energy delivering element (i.e., an electrode) and/or sensing element, as described in greater detail herein.

In certain embodiments, each electrode 136 can be operated independently of the other electrodes 136. For example, each electrode can be individually activated and the polarity and amplitude of each electrode can be selected by an operator or a control algorithm (e.g., executed by the controller 107 previously described herein. The selective independent control of the electrodes 136 allows the end effector 114 to deliver RF energy to highly customized regions. For example, a select portion of the electrodes 136 can be activated to target neural fibers in a specific region while the other electrodes 136 remain inactive. In certain embodiments, for example, electrodes 136 may be activated across the portion of the second segment 124 that is adjacent to tissue at the target site, and the electrodes 136 that are not proximate to the target tissue can remain inactive to avoid applying energy to non-target tissue. Such configurations facilitate selective therapeutic modulation of nerves on the lateral sino-nasal wall within one nostril without applying energy to structures in other portions of the sino-nasal cavity.

The electrodes 136 are electrically coupled to an RF generator (e.g., the generator 106 of FIG. 1) via wires (not shown) that extend from the electrodes 136, through the shaft 116, and to the RF generator. When each of the electrodes 136 is independently controlled, each electrode 136 couples to a corresponding wire that extends through the shaft 116. In other embodiments, multiple electrodes 116 can be controlled together and, therefore, multiple electrodes 116 can be electrically coupled to the same wire extending through the shaft 116. As previously described, the RF generator and/or components operably coupled (e.g., a control module) thereto can include custom algorithms to control the activation of the electrodes 136. For example, the RF generator can deliver RF power at about 460-480 kHz (+ or - 5 kHz) to the electrodes 136, and do so while activating the electrodes 136 in a predetermined pattern selected based on the position of the end effector 114 relative to the treatment site and/or the identified locations of the target nerves. The RF generator is able to provide bipolar low power (10 watts with maximum setting of 50 watts) RF energy delivery, and further provide multiplexing capabilities (across a maximum of 30 channels).

Once deployed, the first and second segments 122, 124 contact and conform to a shape of the respective locations, including conforming to and complementing shapes of one or more anatomical structures at the respective locations. In turn, the first and second segments 122, 124 become accurately positioned within the sino-nasal cavity to subsequently deliver, via one or more electrodes 136, precise and focused application of RF thermal energy to the one or more target sites to thereby therapeutically modulate associated neural structures. More specifically, the first and second segments 122, 124 have shapes and sizes when in the expanded configuration that are specifically designed to place portions of the first and second segments 122, 124, and thus one or more electrodes associated therewith 136, into contact with target sites within sino-nasal cavity associated with postganglionic parasympathetic fibers that innervate the sino-nasal mucosa.

For example, the first set of flexible support elements of the first segment 122 conforms to and complements a shape of a first anatomical structure at the first location when the first segment 122 is in the deployed configuration and the second set of flexible support elements of the second segment 124 conforms to and complements a shape of a second anatomical structure at the second location when the second segment is in the deployed configuration. The first and second anatomical structures may include, but are not limited to, inferior turbinate, middle turbinate, superior turbinate, inferior meatus, middle meatus, superior meatus, pterygopalatine region, pterygopalatine fossa, sphenopalatine foramen, accessory sphenopalatine foramen(ae), and sphenopalatine micro-foramen(ae).

In some embodiments, the first segment 122 of the multi-segment end effector 114 is configured in a deployed configuration to fit around at least a portion of a middle turbinate at an anterior position relative to the middle turbinate and the second segment 124 of the multi-segment end effector is configured in a deployed configuration to contact a plurality of tissue locations in a cavity at a posterior position relative to the middle turbinate.

For example, the first set of flexible support elements of the first segment (i.e., struts 130 and 132) conforms to and complements a shape of a lateral attachment and posterior-inferior edge of the middle turbinate when the first segment 122 is in the deployed configuration and the second set of flexible support elements (i.e., struts 134) of the second segment 124 contact a plurality of tissue locations in a cavity at a posterior position relative to the lateral attachment and posterior-inferior edge of middle turbinate when the second segment 124 is in the deployed configuration. Accordingly, when in the deployed configuration, the first and second segments 122, 124 are configured to position one or more associated electrodes 136 at one or more target sites relative to either of the middle turbinate and the plurality of tissue locations in the cavity behind the middle turbinate. In turn, electrodes 136 are configured to deliver RF energy at a level sufficient to therapeutically modulate postganglionic parasympathetic nerves innervating sino-nasal mucosa at an innervation pathway within the sino-nasal cavity of the patient.

As illustrated in FIG. 5E, the first segment 122 comprises a bilateral geometry. In particular, the first segment 122 includes two identical sides, including a first side formed of struts 130a, 132a and a second side formed of struts 130b, 132b. This bilateral geometry allows at least one of the two sides to conform to and accommodate an anatomical structure within the sino-nasal cavity when the first segment 122 is in an expanded state. For example, when in the expanded state, the plurality of struts 130a, 132a contact multiple locations along multiple portions of the anatomical structure and electrodes provided by the struts are configured to emit energy at a level sufficient to create multiple micro-lesions in tissue of the anatomical structure that interrupt neural signals to mucus producing and/or mucosal engorgement elements. In particular, struts 130a, 132a conform to and complement a shape of a lateral attachment and posterior-inferior edge of the middle turbinate when the first segment 122 is in the deployed configuration, thereby allowing for both sides of the anatomical structure to receive energy from the electrodes. By having this independence between first and second side (i.e., right and left side) configurations, the first segment 122 is a true bilateral device. By providing a bilateral geometry, the multi-segment end effector 114 does not require a repeat use configuration to treat the other side of the anatomical structure, as both sides of the structure are accounted at the same time due to the bilateral geometry. The resultant micro-lesion pattern can be repeatable and is predictable in both macro element (depth, volume, shape parameter, surface area) and can be controlled to establish low to high effects of each, as well as micro elements (the thresholding of effects within the range of the macro envelope can be controlled), as well be described in greater detail herein. The systems of the present invention are further able to establish gradients within allowing for control over neural effects without having widespread effect to other cellular bodies, as will be described in greater detail herein.

FIG. 7 is a cross-sectional view of a portion of the shaft 116 of the handheld device taken along lines 7-7 of FIG. 4. As illustrated, the shaft 116 may be constructed from multiple components so as to have the ability to constrain the end effector 114 in the retracted configuration (i.e., the low-profile delivery state) when the end effector 114 is retracted within the shaft 116, and to further provide an atraumatic, low profile and durable means to deliver the end effector 114 to the target site. The shaft 116 includes coaxial tubes which travel from the handle 118 to a distal end of the shaft 116. The shaft 116 assembly is low profile to ensure transnasal delivery of therapy. The shaft 116 includes an outer sheath 138, surrounding a hypotube 140, which is further assembled over electrode wires 129 which surround an inner lumen 142. The outer sheath 138 serves as the interface between the anatomy and the device 102. The outer sheath 138 may generally include a low friction PTFE liner to minimize friction between the outer sheath 138 and the hypotube 140 during deployment and retraction. In particular the outer sheath 138 may generally include an encapsulated braid along a length of the shaft 116 to provide flexibility while retaining kink resistance and further retaining column and/or tensile strength. For example, the outer sheath 138 may include a soft Pebax material, which is atraumatic and enables smooth delivery through the sino-nasal passage. The outer sheath 138 may further include orientation/landmark markings on an exterior surface thereof, generally at the distal end, wherein the markings may provide a visual indication to an operator of the architecture and/or spatial orientation of first and/or second segments 122, 124 of the end effector 114 to assist in positioning and deployment of the end effector 114.

The hypotube 140 is assembled over the electrode wires starting within the handle 118 and travelling to the proximal end of the end effector 114. The hypotube 140 generally acts to protect the wires during delivery and is malleable to enable flexibility without kinking to thereby improve trackability. The hypotube 140 provides stiffness and enables torqueability of the device 102 to ensure accurate placement of the end effector 114. The hypotube 140 also provides a low friction exterior surface which enables low forces when the outer sheath 138 moves relative to the hypotube 140 during deployment and retraction or constraint. The shaft 116 may be pre-shaped in such a manner so as to complement the sino-nasal cavity. For example, the hypotube 140 may be annealed to create a bent shaft 116 with a pre-set curve. The hypotube 140 may include a stainless-steel tubing, for example, which interfaces with a liner in the outer sheath 138 for low friction movement.

The inner lumen 142 may generally provide a channel for fluid extraction during a treatment procedure. For example, the inner lumen 142 extends from the distal end of the shaft 116 through the hypotube 140 and to atmosphere via a fluid line (line 121 of FIG 4). The inner lumen 142 materials are chosen to resist forces of external components acting thereon during a procedure.

FIG. 8A is a side view of the handle of the handheld 118 and FIG. 8B is a side view of the handle 118 illustrating internal components enclosed within. The handle 118 generally includes an ergonomically-designed grip portion which provides ambidextrous use for both left and right handed use and conforms to hand anthropometrics to allow for at least one of an overhand grip style and an underhand grip style during use in a procedure. For example, the handle 118 may include specific contours, including recesses 144, 146, and 148 which are designed to naturally receive one or more of an operator's fingers in either of an overhand grip or underhand grip style and provide a comfortable feel for the operator. For example, in an underhand grip, recess 144 may naturally receive an operator's index finger, recess 146 may naturally receive an operator's middle finger, and recess 148 may naturally receive an operator's ring and little (pinkie or pinky) fingers which wrap around the proximal protrusion 150 and the operator's thumb naturally rests on a top portion of the handle 118 in a location adjacent to the first mechanism 126. In an overhand grip, the operator's index finger may naturally rest on the top portion of the handle 118, adjacent to the first mechanism 126, while recess 144 may naturally receive the operator's middle finger, recess 146 may naturally receive a portion of the operator's middle and/or ring fingers, and recess 148 may naturally receive and rest within the space (sometimes referred to as the purlicue) between the operator's thumb and index finger.

As previously described, the handle includes multiple user-operated mechanisms, including at least a first mechanism 126 for deployment of the end effector from the collapsed/retracted configuration to the expanded deployed configuration and a second mechanism 128 for controlling of energy output by the end effector, notably energy delivery from one or more electrodes. As shown, the user inputs for the first and second mechanisms 126, 128 are positioned a sufficient distance to one another to allow for simultaneous one-handed operation of both user inputs during a procedure. For example, user input for the first mechanism 126 is positioned on a top portion of the handle 118 adjacent the grip portion and user input for the second mechanism 128 is positioned on side portions of the handle 118 adjacent the grip portion. As such, in an underhand grip style, the operator's thumb rests on the top portion of the handle adjacent to the first mechanism 126 and at least their middle finger is positioned adjacent to the second mechanism 128, each of the first and second mechanisms 126, 128 accessible and able to be actuated. In an overhand grip system, the operator's index finger rests on the top portion of the handle adjacent to the first mechanism 126 and at least their thumb is positioned adjacent to the second mechanism 128, each of the first and second mechanisms 126, 128 accessible and able to be actuated. Accordingly, the handle accommodates various styles of grip and provides a degree of comfort for the surgeon, thereby further improving execution of the procedure and overall outcome.

Referring to FIG. 8B, the various components provided within the handle 118 are illustrated. As shown, the first mechanism 126 may generally include a rack and pinion assembly providing movement of end effector between the retracted and deployed configurations in response to input from a user-operated controller. The rack and pinion assembly generally includes a set of gears 152 for receiving input from the user-operated controller and converting the input to linear motion of a rack member 154 operably associated with at least one of the shaft 116 and the end effector. The rack and pinion assembly comprises a gearing ratio sufficient to balance a stroke length and retraction and deployment forces, thereby improving control over the deployment of the end effector. As shown, the rack member 154 may be coupled to a portion of the shaft 116, for example, such that movement of the rack member 154 in a direction towards a proximal end of the handle 118 results in corresponding movement of the shaft 116 while the end effector remains stationary, thereby exposing the end effector and allowing the end effector to transition from the constrained, retracted configuration to the expanded, deployed configuration. Similarly, movement of the rack member 154 in a direction towards a distal end of the handle 118 results in corresponding movement of the shaft 116 while the end effector remains stationary, and thereby encloses the end effector within the shaft 116. It should be noted that, in other embodiments, the rack member 154 may be directly coupled to a portion of the end effector such that movement of the rack member 154 results in corresponding movement of the end effector while the shaft 116 remains stationary, thereby transitioning the end effector between the retracted and deployed configurations.

The user-operated controller associated with the first mechanism 126 may include a slider mechanism operably associated with the rack and pinion rail assembly. Movement of the slider mechanism in a rearward direction towards a proximal end of the handle results in transitioning of the end effector to the deployed configuration and movement of the slider mechanism in a forward direction towards a distal end of the handle results in transitioning of the end effector to the retracted configuration. In other embodiment, the user-operated controller associated with the first mechanism 126 may include a scroll wheel mechanism operably associated with the rack and pinion rail assembly. Rotation of the wheel in a rearward direction towards a proximal end of the handle results in transitioning of the end effector to the deployed configuration and rotation of the wheel in a forward direction towards a distal end of the handle results in transitioning of the end effector to the retracted configuration.

As previously noted, the end effector of the present invention may further include one or more flexible printed circuit board (PCB) members operably associated therewith. Accordingly, as will be described in greater detail herein, the first (proximal) and second (distal) segments of the multi-segment end effector essentially serve as a framework upon which separate respective flexible PCB assemblies are attached, wherein energy delivering elements, such as electrodes, are provided via flexible PCB members.

In this manner, the present invention provides an end effector that is capable of highly conforming to anatomical variations within a sino-nasal cavity so that an operator can perform an accurate, minimally invasive, and localized application of energy to one or more target sites within the sino-nasal cavity of the patient to thereby treat a sino-nasal condition. In particular, unlike other surgical treatments for rhinitis, the devices of the invention are minimally invasive. Once delivered within the sino-nasal cavity, each segment of the end effector can expand to a specific shape and/or size corresponding to anatomical structures within the sino-nasal cavity and associated with the target sites. More specifically, each of a first segment and a second segment includes a specific geometry when in a deployed configuration to complement anatomy of respective locations within the sino-nasal cavity. A plurality of flexible PCB members attached to the respective first and second segments are able to correspondingly move and transition into the specific geometry of the given segment, such that, once deployed, the flexible PCBs of the first and second segments contact and conform to a shape of the respective locations, including conforming to and complementing shapes of one or more anatomical structures at the respective locations.

In turn, the plurality of flexible PCB members of the first and second segments become accurately positioned within the sino-nasal cavity to subsequently deliver, via one or more electrodes, precise and focused application of energy to targeted tissue at the one or more target sites, to disrupt multiple neural signals to, and/or result in local hypoxia of, mucus producing and/or mucosal engorgement elements, thereby reducing production of mucus and/or mucosal engorgement within a nose of the patient and reducing or eliminate one or more symptoms associated with at least one of rhinitis, congestion, and rhinorrhea.

The use of flexible PCBs members results in a greater amount of usable surface area than what is otherwise available with existing end effectors. In particular, the increase in surface area allows for a greater number of energy delivery elements to be introduced and utilized in a given procedure and further expands the possible number of patterns of such energy delivery elements. As a result, the contact surface increases substantially, thereby allowing for the end effector of the present invention to deliver treatment to certain areas within the sino-nasal cavity that may have been previously unreachable or untreatable with current treatment devices, or that previously required a surgeon to reposition a given device to reach such areas. Furthermore, the use of flexible PCB members reduces the overall complexity with regard to manufacturing the end effector of the present invention. In particular, any given flexible PCB member (including an overall PCB assembly, which includes multiple PCB members) is constructed separately from the end effector, which includes constructed the overall electrode design and placement on a given PCB member. Once a PCB assembly is complete, PCB members are then attached to respective portions of a given segment of the end effector as a separate manufacturing step, thereby reducing the complexity that is otherwise associated with placing electrodes directly on the end effector, which is a common practice.

FIGS. 9A and 9B are enlarged perspective views of the second (distal) segment 124 of the multi-segment end effector 114 including a flexible printed circuit board (PCB) assembly operably associated therewith. FIG. 10 is an enlarged perspective view of a single looped strut or support element 134 of the second (distal) segment 124, illustrating an individual PCB member fixed to portions thereof.

As shown, each of the struts or support elements 134(1)-134(6) of the second (distal) segment 134 includes loop-like or leaflet-like shape. Accordingly, the struts or support structures 134 may also be referred to herein as leaflets. Each leaflet 134 includes a pair of flexible PCB members 200 affixed to a portion thereof. For example, a first leaflet 134(1) includes a set of flexible PCB members 200(1) and 200(2) coupled thereto, a second leaflet 134(2) includes a second set of flexible PCB members 200(3) and 200(4) coupled thereto, and so on. Accordingly, in the present embodiment, the flexible PCB assembly includes twelve individual flexible PCB members 200, wherein each of the six leaflets 134 of the distal segment 124 includes a pair of flexible PCB members 200 attached thereto.

Each of the flexible PCB members 200 includes a PCB substrate or base layer composed of a flexible material upon which one or more electronic components are provided, such as, for example, energy delivering elements (i.e., electrodes) and/or sensors. As shown, as a result of a flexible substrate material, each of the flexible PCB members 200 is capable of moving (e.g, bending, twisting, folding, etc.) between various positions in correspondence with movement of the underlying retractable and expandable leaflet 134 to which it is attached. As will be described in greater detail herein, each flexible PCB member 200 further includes one or more energy delivering elements (e.g., electrodes) provided thereon and configured to deliver energy to tissue associated with one or more target sites in the sino-nasal cavity. In this manner, upon deployment of the second (distal) segment 124 to the expanded configuration, each of the plurality of flexible PCB members 200 attached to the distal segment 124 is able to correspondingly move and transition into the specific geometry of leaflets 134, such that, once deployed, the flexible PCB members 200 contact and conform to a shape of the respective location, including conforming to and complementing shapes of one or more anatomical structures at the respective locations. In turn, the plurality of flexible PCB members 200 become accurately positioned within the sino-nasal cavity to subsequently deliver, via one or more electrodes, precise and focused application of energy to targeted tissue at the one or more target sites, to disrupt multiple neural signals to, and/or result in local hypoxia of, mucus producing and/or mucosal engorgement elements, thereby reducing production of mucus and/or mucosal engorgement within a nose of the patient and reducing or eliminate one or more symptoms associated with at least one of rhinitis, congestion, and rhinorrhea.

FIG. 11 is an enlarged plan view of one embodiment of a flexible PCB member 300 consistent with the present disclosure. FIG. 12 is a cross-sectional view of a portion of the flexible PCB member 300 taken along lines 12-12. In some embodiments, the flexible PCB member 300 may include a pair of conductive tracks or traces 304, 306 sandwiched between first and second layers 302(a), 302(b) of a flexible substrate. The flexible PCB member 300 is affixed along a length of a corresponding leaflet 134, specifically a portion of the wire, via an adhesive (e.g., a medical grade epoxy or the like) between the second layer 302(b) of substrate and the wire. Specific portions of a first layer 302(a) of substrate may be removed to thereby expose portions of the conductive track 304, 306 and serve as an energy emitting portion of the flexible PCB member 300.

FIG. 13 is an enlarged plan view of another embodiment of a flexible PCB member 400 consistent with the present disclosure. In this embodiment, as opposed to having exposed portions of electrical track(s) serving as the energy delivery elements, member 400 includes a first set of electrodes 404(1), 404(2), 404(n) and a second set of electrodes 406(1), 406(2), 406(n) disposed on a flexible substrate 402. As illustrated, the electrodes 404, 406 can be deposited directly on the substrate 402 via any known process or technique (e.g., via electrodeposition, ion beam deposition, sputtering, and combinations thereof) or directly mounted with an adhesive such as an insulating glue, then electrically connected to communication paths 405, 407, respectively. The electrodes 404, 406 can be formed from copper, gold, platinum, iridium, stainless steel and/or other conductive materials or elements. The electrodes 404, 406 can optionally be coated with a surface coating, such as iridium oxide, platinum black (Pt black), PEDOT (i.e., poly(3,4-ethylenedioxythiophene)), or carbon nanotubes, as examples. As such, each flexible PCB member may include a thin insulating polymer film having conductive circuit patterns affixed thereto and a thin polymer coating protecting conductor circuits. The substrate material may include polyimide or a similar polymer material. The electrical communication pathways 405, 407 may include traces within or on the substrate formed of copper, gold, platinum, or silver, for example.

FIG. 14 is a plan view of a flexible PCB assembly 500 consistent with the present disclosure, illustrating various portions of the assembly 500. A given flexible PCB assembly generally includes an elongate body including a distal end, defined by a plurality of individual PCB members, and a proximal end, which includes a set of corresponding electrical contacts or connectors in communication with one or more components (e.g., electrodes, sensors, etc.) provided on a given flexible PCB member via electrical communication pathways provided therebetween. Each of the plurality of flexible PCB members 200 extends from a transition portion 202 of an elongate body of the PCB assembly.

The electronic components are provided with accompanying electrical communication pathways (e.g., electrical, optical, or electro-optical communication pathways) and coupled to the console 104, including the various components associated therewith (controller 107, monitoring system 108, evaluation / feedback algorithms 110, interface 112, etc.) by way of the set of corresponding electrical contacts or connectors at the proximal end of the PCB assembly (when the device 102 is coupled to the console 104). Accordingly, electronic components provided on a given flexible PCB member (i.e., electrodes, sensors, or the like) can function in a similar manner as previously described herein and provide similar benefits. For example, electrodes may be configured to deliver therapeutic energy to targeted tissue within a portion of the sino-nasal cavity for the treatment of a sino-nasal condition, such as rhinosinusitis, in a similar manner as previously described herein.

Upon affixing the PCB members to the respective portions of the distal segment 134, for example, the elongate body of the PCB assembly is wrapped around the wires 129 (referring to FIG. 7) and is contained within the shaft 116 such that the proximal end, which includes an electrical connector, extends from the proximal end of the shaft 116 is available to be coupled to a corresponding connector 156 within the handle 118 (referring to FIG. 8B).

FIG. 15 is an enlarged plan view of one embodiment of flexible PCB members 502(1) through 502(n) of a flexible PCB assembly 500 consistent with the present disclosure. The substrate of the PCB assembly 500 can be a single or multi-layer flexible PCB layer made as a single work piece. For example, the substrate 200 could be laser cut from a single piece of flex-PCB material. As such, manufacturing complexity and time, and cost can be reduced. As shown in FIG. 15, for example, the multiple PCB members 502(1)-502(n) can be formed by laser-cutting (or stamping) multiple cuts into a distal end of the assembly body, including cut or stamped reliefs 503 staggered and at the end of every other cut to thereby serve as a coupling between a corresponding pair of each set of PCB members. In other words, for the distal segment 124, six pairs of PCB members 502 is required, as each leaflet 134 of the distal segment 124 includes a pair of PCB members affixed thereto. Accordingly, a relief 503 is formed between each of the six pairs of PCB members 502. A transition portion 504 is also formed in the assembly substrate, which effectively transitions the PCB members down to a smaller width of the elongate body 506 of the PCB assembly.

FIGS. 16A and 16B are enlarged plan views of a distal end and proximal end, respectively, of a flexible PCB assembly 500 consistent with the present disclosure, illustrating PCB members 502 including the arrangement of electrodes 404, 406, and electrical communication pathways 405, 407 shown and previously described with respect to the PCB member of FIG. 13. FIG. 16B illustrates a standard connector at the proximal end of the PCB assembly, which includes multiple electrical contacts coupled to the electrodes 404, 406 via the communication pathways 405, 407.

FIGS. 17A and 17B are plan views of another embodiment of a flexible PCB assembly consistent with the present disclosure, illustrating the interleaving of two separate assemblies 600(a) and 600(b) to form a combined assembly of overlapping flexible PCB members 601 from each assembly 600(a), 600(b). FIG. 17A shows two PCB assemblies separated from one another to illustrate that each assembly includes three pairs of PCB members 600. For example, a first PCB assembly 600(a) includes a first pair 601(1), 601(2), a second pair 601(3), 601(4), and a third pair 601(5), 601(6) of PCB members, while a second PCB assembly 600(b) includes a fourth pair 601(7), 601(8), a fifth pair 601(9), 610(10), and a sixth pair 601(11), 601(12). FIG. 17B shows the overlapping of the two assemblies 600(a) and 600(b), whereby the pairs of PCB members of the first assembly 600(a) are offset relative to the pairs of PCB members of the second assembly 600(b), resulting in even distribution of PCB member when coupled to the respective portions of leaflets. This overlapping, interweaving concept enables relatively simple manufacturing, thereby cutting down on costs.

FIGS. 18A, 18B, and 18C are top and side views, partly in section, of one embodiment of a jig assembly 700 used for attaching one or more flexible PCB members to respective support elements of the second (distal) segment of the end effector. As shown, the jig assembly may include a base 702 and a clamp fixture 704 for applying pressure to a portion of a leaflet or support element, wherein a tensioning pin 706 may be used to apply tension to the leaflet and hold the leaflet in a position that is flat against a flexible PCB substrate for subsequent adhesive deposition via a dispenser 708. By applying tension to the leaflet (i.e., use of dynamic loading), the jig 700 utilizes the elastic properties of the wires to thereby straighten the wires and affix the flexible PCB members thereto. Upon releasing the tension (once the flexible PCB member is affixed), the leaflet can rebound back into the geometric shape.

FIG. 19 is a perspective view of another embodiment of a jig assembly 800 used for attaching one or more flexible PCB members to respective support elements of the second (distal) segment of the end effector. As shown, the jig assembly 800 includes channels 802 corresponding to the geometric shape of the leaflets 134 of the distal segment 124 when it is in the deployed configuration. The channels 802 are shaped and/or sized to receive a length of each strut of a given leaflet, whereby the jig assembly 802 further includes ports 804 within each channel 802 and in communication with a vacuum source. Upon roughly aligning a flexible PCB member with a portion (i.e., a length of a strut) of a leaflet retained within a channel 802, an operator may activate the vacuum source, thereby causing a negative pressure to form at each port 804, thereby effectively drawing the flexible PCB member toward the leaflet via a suction force. The flexible PCB member is effectively held in place against the leaflet, thereby allowing for an operator to affix the flexible PCB member to the leaflet (via an adhesive). Each channel 802 may further include a flange or hook member 806 to further improve alignment of each leaflet within the respective channels 802. By retaining a given leaflet in place, while it is in is deployed configuration and exhibiting its geometric shape associated with the deployed configuration, minimal manipulation is required when affixing flexible PCB members thereto. Accordingly, the flexible PCB members are able to be manufactured (i.e., cut to size and shape) to naturally align with the leaflets without requiring any particular leaflet manipulation (i.e., deformation of the leaflet struts).

FIG. 20 is an enlarged side view of the first (proximal) segment 122 of the multi-segment end effector 114 illustrating placement of a flexible PCB assembly, comprised of multiple flexible PCB members 900(1)-900(n), upon the various struts or support structures 130 of the first (proximal) segment 122. FIG. 21 is an image illustrating a perspective view of the first (proximal) segment 122 including the flexible PCB assembly attached to the support elements. Similar to as previously described with respect to the distal segment 124, the proximal segment 122 includes struts or support elements 130, 132, which include loop-like or leaflet-like shapes. Accordingly, the struts or support structures 130, 132 may also be referred to herein as leaflets. Each leaflet 130, 132 includes a pair of flexible PCB members 900 affixed to a portion thereof. For example, a first leaflet 130 includes a set of flexible PCB members 900(1) and 900(2) coupled thereto, a second leaflet 132 includes a second set of flexible PCB members 900(3) and 900(4) coupled thereto, and so on. The flexible PCB members 900 as similarly configured as the flexible PCB members 200, 300, 400, 500, and 600 previously described herein and thus function in a similar manner.

FIG. 22 is a perspective view of one embodiment of a jig assembly 1000(a) used for attaching one or more flexible PCB members to respective support elements of the first (distal) segment 122 of the end effector 114. FIG. 23 is an enlarged view of the jig assembly 1000(a) of FIG. 22. The jig assembly 1000(a) includes a base 1002 and a clamp fixture 1004 for securing the proximal segment 122 in place while flexible PCB members 900 are affixed to the corresponding portions of the proximal segment 122. The jig assembly 1000(a) further includes channels 1006 corresponding to the geometric shape of the leaflets 130, 132 of the proximal segment 122 when it is in the deployed configuration. The channels 1006 are shaped and/or sized to receive a length of each strut of a given leaflet, whereby the jig assembly 1000(a) further includes ports 1008 within each channel 1006 and in communication with a vacuum source. Again, an operator can simply align a given flexible PCB member with a corresponding portion of a leaflet and activate the vacuum source, which will draw the flexible PCB member toward in into contact with the leaflet, thereby confirming the flexible PCB member to the particular strut of the leaflet for subsequent affixing. The base 1002 of the jig assembly 1000(a) further includes a ridge 110 extending from the base 1002 and having an apex further promotes bending of the flexible PCB member during operation of the vacuum source so that the flexible PCB member better conforms to the void created between leaflets 130 and 132 when the proximal segment 122 is in the deployed configuration. FIG. 24 is an enlarged view of an alternate embodiment of the jig assembly 1000(b) of FIG. 22, which provides for additional features.

It should be noted that the flexible PCB members described herein can by coupled to support elements of any given end effector via any known wafer bonding techniques. For example, a given PCB member can be affixed to the end effector by way of one or more wafer bonding methods, including, but not limited to, direct bonding methods, surface activated bonding methods, plasma activated bonding methods, anodic bonding methods, eutectic bonding methods, glass frit bonding methods, adhesive bonding methods, thermocompression bonding methods, reactive bonding methods, and transient liquid phase diffusion bonding methods. As understood, the applied wafer bonding technology is based, at least in part, on the given material of the substrate(s) and specifications, such as maximum bearable temperature, mechanical pressure, and/or desired gaseous atmosphere.

FIG. 25 is an enlarged perspective view of the multi-stage end effector illustrating flexible PCB members coupled to loop struts or support elements of each of the first (proximal) and second (distal) segments 122, 124, in which the flexible PCB members substantially covers the loop-like or leaflet-like shape when in the deployed configuration.

The use of flexible PCBs members results in a greater amount of usable surface area than what is otherwise available with existing end effectors. In particular, the increase in surface area allows for a greater number of energy delivery elements to be introduced and utilized in a given procedure and further expands the possible number of patterns of such energy delivery elements. As a result, the contact surface increases substantially, thereby allowing for the end effector of the present invention to deliver treatment to certain areas within the sino-nasal cavity that may have been previously unreachable or untreatable with current treatment devices, or that previously required a surgeon to reposition a given device to reach such areas. Furthermore, the use of flexible PCB members reduces the overall complexity with regard to manufacturing the end effector of the present invention. In particular, any given flexible PCB member (including an overall PCB assembly, which includes multiple PCB members) is constructed separately from the end effector, which includes constructed the overall electrode design and placement on a given PCB member. Once a PCB assembly is complete, PCB members are then attached to respective portions of a given segment of the end effector as a separate manufacturing step, thereby reducing the complexity that is otherwise associated with placing electrodes directly on the end effector, which is a common practice.

In this manner, the present invention provides an end effector that is capable of highly conforming to anatomical variations within a sino-nasal cavity so that an operator can perform an accurate, minimally invasive, and localized application of energy to one or more target sites within the sino-nasal cavity of the patient to thereby treat a sino-nasal condition. In particular, unlike other surgical treatments for rhinitis, the devices of the invention are minimally invasive. Once delivered within the sino-nasal cavity, each segment of the end effector can expand to a specific shape and/or size corresponding to anatomical structures within the sino-nasal cavity and associated with the target sites. More specifically, each of a first segment and a second segment includes a specific geometry when in a deployed configuration to complement anatomy of respective locations within the sino-nasal cavity. A plurality of flexible PCB members attached to the respective first and second segments are able to correspondingly move and transition into the specific geometry of the given segment, such that, once deployed, the flexible PCBs of the first and second segments contact and conform to a shape of the respective locations, including conforming to and complementing shapes of one or more anatomical structures at the respective locations.

In turn, the plurality of flexible PCB members of the first and second segments become accurately positioned within the sino-nasal cavity to subsequently deliver, via one or more electrodes, precise and focused application of energy to targeted tissue at the one or more target sites, to disrupt multiple neural signals to, and/or result in local hypoxia of, mucus producing and/or mucosal engorgement elements, thereby reducing production of mucus and/or mucosal engorgement within a nose of the patient and reducing or eliminate one or more symptoms associated with at least one of rhinitis, congestion, and rhinorrhea.

As previously noted, the invention further provides a new and unique end effector that takes advantage of the benefits of flexible printed circuit boards (PCBs), as well as various manufacturing techniques, to provide an improved device for the treatment of a rhinosinusitis condition.

In particular, the underlying design of the end effector is unique. In a preferred embodiment, the end effector is multi-segmented and includes a proximal segment and a distal segment. The proximal and distal segments are constructed from single, unitary work pieces having elastic properties. More specifically, a single piece of shape memory material, such as nitinol, may be used to construct one or more portions of the proximal segment and further construct the distal segment in its entirety. For example, in one embodiment, the proximal segment is composed of a pair of interlocking members, while the distal segment is composed of a single member. The pair of interlocking members of the proximal segment include a first member providing a first set of support elements and a second member providing a second set of support members. As such, each of the first member and the second member may be constructed from a single workpiece and subsequently interlocked within one another to form the proximal segment, while the distal segment comprises a single component (as opposed to interlocking components), and is thus formed from a single workpiece.

The single workpiece may initially be in the form of a tube or a flat plate and can be laser cut to form the desired framework of support elements of the proximal and distal segments. In addition to reducing time, cost, and complexity, the use of laser machining allows a greater amount of design freedom for the manufacturer, in turn leading to a more tailored geometry and mechanical properties of a given segment of the end effector. For example, laser machining allows greater control over mechanical properties of the support elements, including tailoring the stiffness of a specific one of, or a given group of, support elements for a given segment, thereby allowing for tailoring of the tissue apposition profile when the given segment is in an expanded, deployed configuration. Furthermore, utilizing a stock workpiece in the form of a tube or flat plate results in support elements having a relatively flat surface upon which a corresponding flexible PCB member is affixed, thereby improving apposition of the PCB member to tissue within the sino-nasal cavity.

The invention provides improved manufacturing techniques for bonding the PCB members to support elements of a given proximal or distal segment. In particular, the present invention contemplates the use of bonding, thermal, and mechanical processes for joining PCB members to respective support elements, which may include one or more of adhesion, mechanical, lamination, polymer reflow, induction heating, spot welding, and laser welding processes. For example, in one embodiment, attaching a PCB member to a respective support element includes a reflow process in which a flexible PCB member is positioned relative to a respective support element, one or more polymer layers are then disposed around the flexible PCB member, and heat is then applied resulting in the one or more polymer layers encasing the flexible PCB member and subsequently affixing the flexible PCB member to the underlying support element of the end effector segment. In another embodiment, polymer sleeves may be secured to the support elements, thereby providing a substrate upon which a flexible PCB member may be positioned and subsequently attached. It should be noted that the process of securing the polymer sleeve and bonding the flexible PCB member to the support element may occur simultaneously via the application of pressure and heat.

FIG. 26 is a side perspective view of another embodiment of an end effector 1114. The end effector 1114 includes one or more retractable and expandable segments, each of which is comprised of a framework of support elements having elastic properties. Each retractable and expandable segment further includes one or more flexible printed circuit board (PCB) members provided thereon. The flexible PCB members are composed of a flexible material capable of moving (e.g., bending, twisting, folding, etc.) between various positions in correspondence with movement of the underlying retractable and expandable segment to which it is attached. Each flexible PCB member further includes one or more energy delivering elements (e.g., electrodes) provided thereon and configured to deliver energy to tissue associated with one or more target sites in the sino-nasal cavity. Once delivered within the sino-nasal cavity, the one or more segments can expand to a specific shape and/or size corresponding to anatomical structures within the sino-nasal cavity and associated with target sites to undergo delivery of therapeutic energy for treatment of a condition (i.e., rhinosinusitis or the like). As such, once deployed, the flexible PCBs of the first and second segments contact and conform to a shape of the respective locations, including conforming to and complementing shapes of the one or more anatomical structures, thereby accurately positioning the electrodes for focused application of energy to targeted tissue at the one or more target sites.

As illustrated, the end effector 1114 is multi-segmented. It should be noted that the multi-segment end effector 1114 shares similarities with the end effectors (114, etc.) previously described herein and illustrated in the corresponding figures and, as such, like reference numerals generally refer to like parts and components of each.

As shown, the end effector 1114 includes a first segment 1122 and a second segment 1124 generally spaced apart from another. The first segment 1122 is positioned closer to the handle, and thus referred to herein as the "proximal segment 1122", while the second segment 1124 is positioned further from the handle, and thus referred to herein as the "distal segment 1124". As will be described in greater detail herein, the proximal and distal segments 1122 and 1124 are each constructed from single, unitary work pieces having elastic properties. More specifically, a single piece of shape memory material, such as nitinol or the like, may be used to construct one or more portions of the proximal segment 1122 and further construct the distal segment 1124 in its entirety.

For example, as illustrated, the proximal segment 1122 is composed of a pair of interlocking members 1130 and 1132, while the distal segment is composed of a single member. The pair of interlocking members of the proximal segment 1122 include a first member 1130 providing a first set of support elements 1130(a) and 1130(b) and a second member 1132 providing a second set of support members 1132(a)-1132(d). As such, each of the first member 1130 and the second member 1132 may be constructed from a single workpiece and subsequently interlocked within one another to form the proximal segment, as illustrated in FIG. 26, while the distal segment 1124 comprises a single component (as opposed to interlocking components), and is thus formed from a single workpiece.

FIGS. 27A and 27B are perspective views of the first interlocking member 1130 of the proximal segment 1122 and FIGS. 27C and 27D are side and front-facing perspective views of the second interlocking member 1132 of the proximal segment 1122. As illustrated, the first segment 1122 comprises a bilateral geometry. In particular, the first segment 1122 includes two identical sides, including a first side (formed of strut 1130(a) of the first interlocking member and struts 1132(a) and 1132(c) of the second interlocking member) and a second side (formed of strut member 1130(b) of the first interlocking member and struts 1132(b) and 1132(d) of the second interlocking member). This bilateral geometry allows at least one of the two sides to conform to and accommodate an anatomical structure within the sino-nasal cavity when the first segment 1122 is in an expanded state. For example, when in the expanded state, the plurality of struts 1130(a)-1130(b) and 1132(a)-1132(d) may contact multiple locations along multiple portions of the anatomical structure and thereby position flexible PCB members provided by the struts into contact with the desired targets so as to emit energy thereto. In particular, one or more of struts 1130(a)-1130(b) and 1132(a)-1132(d) may conform to and complement a shape of a lateral attachment and posterior-inferior edge of the middle turbinate when the first segment 1122 is in the deployed configuration, thereby allowing for both sides of the anatomical structure to receive energy from the flexible PCB members.

As shown in FIGS. 27A and 27B, the first interlocking member includes a collar member 1126 from which the struts 1130(a) and 1130(b) extend. The collar member is generally annular in shape and configured to connect to a core mandrel or shaft of the treatment device. In the illustrated embodiment, each strut 1130(a) and 1130(b) has ends that are connected to the collar 1126 portion. In other words, neither strut 1130(a) nor 1130(b) has a free end (i.e., an end that is unconnected to anything), which is in contrast to the plurality of struts 1136 of the distal segment 1124, each of which has a free, distal end. As shown the struts 1130(a) and 1130(b) generally extend in a direction orthogonal and away from a longitudinal axis of the device. As further illustrated, each strut 1130(a) and 1130(b) may include one or more articulation sites that improve flexibility of the struts. The one or more articulation sites may generally include an area of reduced material, for example. In the present example, he area of reduced material forms an S-shaped configuration. The inclusion of articulation sites reduces stiffness, thereby allowing a manufacturer to tailor movement of a given strut as desired (i.e., direct movement so as to improve overall tissue apposition when placing the flexible PCB member in place for delivery of treatment energy.

As shown in FIGS. 27C and 27D, the second interlocking member includes a collar member 1128 from which the struts 1132(a)-1132(d) extend. Similar to collar member 1126, the collar member 1128 of the second interlocking member may facilitate fixation of the second interlocking member to a core mandrel or shaft of the device. Furthermore, the collar 1128 may have a slightly smaller outer diameter than an inner diameter of collar 1126, such that the collar 1128 may be received within the collar 1126 of the first interlocking member, concentrically received and secured such that the first and second interlocking members become engaged with one another to thereby create a fully assembled proximal segment 1122.

In the illustrated embodiment, each strut 1132(a) through 1132(d) has ends that are connected to the collar 1128 portion. In other words, none of the struts 1132(a)-1132(d) has a free end (i.e., an end that is unconnected to anything). As shown struts 1132(a) and 1132(b) generally extend in a direction orthogonal and away from a longitudinal axis of the device in a similar direction as struts 1130(a) and 1130(b), while struts 1132(c) and 1132(d) generally extend in a direction orthogonal and away from a longitudinal axis of the device in an opposite direction from struts 1130(a) and 1130(b) and 1132(a) and 1132(b). As further illustrated, each strut 1132(a)-1132(d) may include one or more articulation sites that improve flexibility of the struts.

As will be described in greater detail herein, each of the first and second interlocking members of the proximal segment 1122 are constructed from a single workpiece, which may initially be in the form of a tube or a flat plate and can be laser cut to form the desired framework of struts and collar of the first and second interchangeable members.

FIG. 28 is a perspective view of a distal segment 1124. As shown, the distal segment 1124 includes a plurality of struts 1136(1), 1136(2)... 1136(n) extending from one or more collar portions 1134(a) and 1134(b). In the illustrated embodiment, the distal segment 1124 includes a first collar 1134(a) immediately adjacent to the struts and a second collar 1134(b) positioned a length away from the first collar 1134(a) along a length of a body 1138. At least the second collar 1134(b) and a length of the body 1138 as shaped and/or sized to be received within each of the collar 1126 and collar 1128 of the interlocking members of the proximal segment 1122 (shown in FIG. 26). Accordingly, the second collar 1134(b) may be directed coupled to a core mandrel or shaft of the device, while the body 1138 is sufficient length to allow the struts 1130 and 1132 of the proximal segment 1122 to transition to a fully expanded and deployed configuration unimpeded by the struts 1136 of the distal segment 1124.

As shown, the plurality of struts 1136 of the distal segment 1124 include a free distal end (i.e., a distal end that is unattached). The struts 1136 are deployable into a coaxial configuration with respect to a longitudinal axis of the device. More specifically, when in the deployed configuration, at least a portion of the plurality of individual struts are in a coaxial configuration with respect to a longitudinal axis of the device.

As previously described, each of the proximal and distal segments 1122 and 1124 are constructed from single, unitary work pieces having elastic properties. For example, FIG. 29 is a plan view of a single workpiece of material from which the distal segment 1124, specifically the plurality of support elements / struts 1136, are constructed via a laser machining process. The single workpiece may initially be in the form of a tube or a flat plate and can be laser cut to form the desired framework of support elements of the proximal and distal segments. In addition to reducing time, cost, and complexity, the use of laser machining allows a greater amount of design freedom for the manufacturer, in turn leading to a more tailored geometry and mechanical properties of a given segment of the end effector. For example, laser machining allows greater control over mechanical properties of the support elements, including tailoring the stiffness (e.g., via formation of articulation sites) of a specific one of, or a given group of, support elements for a given segment, thereby allowing for tailoring of the tissue apposition profile when the given segment is in an expanded, deployed configuration. Furthermore, utilizing a stock workpiece in the form of a tube or flat plate results in support elements having a relatively flat surface upon which a corresponding flexible PCB member is affixed, thereby improving apposition of the PCB member to tissue within the sino-nasal cavity.

FIGS. 30A and 30B are perspective and side views of the distal segment 1124 illustrating fixation points 1140 defined on each of the plurality of support elements / struts 1136. As shown, each of the plurality of struts 1136 further includes fixation points 1140(1) and 1140(2) provided thereon. The fixation points may be useful in facilitating the attachment and alignment of a polymer sleeve and/or a flexible PCB member thereto. For example, the fixation points 1140 may include a recess, hole, notch, groove, etching, or the like to thereby increase surface area to receive an adhesive or pooling of melted polymer for mechanically securing a polymer sleeve and/or flexible PCB member in place ensuring alignment and simplifying assembly, as described in greater detail herein.

FIG. 31 is plan view of a single workpiece of material, generally in the form of a flat or substantially planar plate, from which the distal segment 1124 of FIGS. 30A-30B, specifically the plurality of support elements / struts and associated fixation points, are constructed via a laser machining process.

FIGS. 32A and 32B are side views of a single workpiece of material, generally in the form of a tube, from which the distal segment 1124 of FIGS. 30A-30B, specifically the plurality of support elements / struts and associated fixation points, are constructed via a laser machining process.

FIG. 33 is a perspective view of a distal segment illustrating various fixation point designs provided on, or otherwise associated with, one or more of the plurality of struts 1136. As previously described, the fixation points may be useful in facilitating the attachment and alignment of a polymer sleeve and/or a flexible PCB member thereto. For example, the different fixation points 1140(1), 1140(2), 1140(3), 1140(4), and 1140(5) may each be useful in strengthening the bond between a soft polymer material and the strut. As shown, fixation points 1140(1), 1140(3), and 1140(5) each consists of areas of reduced material defined along the length of the strut, which may include fixation point 1140(1) positioned at the distal most end, fixation point 1140(3) positioned mid-length along the strut, and 1140(5) positioned at the proximal most end of the strut. Fixation points may also include holes or apertures, such as fixation points 1140(2) and 1140(4), which include extended slots (1140(2)) and discrete holes (1140(4)) defined along the length of a given strut. It should be noted that each of the plurality of struts of a given distal segment may include the same fixation point design (i.e., all struts have the same fixation point(s) positioned at the same positions) or, in some embodiments, individual struts may of a given distal segment have different fixation point designs, or no fixation points at all.

FIG. 34 is a is a perspective view of a distal segment illustrating various fixation point designs provided on, or otherwise associated with, one or more of the plurality of struts 1130, 1132. As shown, the proximal segment 1122 may include similar fixation point designs as the distal segment 1124, shown in FIG. 33.

FIG. 35 is a perspective view illustrating another process of coupling of flexible PCB members 200 to corresponding support elements / struts 1136 of the distal segment 1124 utilizing the fixation points. As previously noted, the flexible PCB members 200 can be directly bonded to corresponding struts 1136 via an adhesive. In the illustrated embodiment, the fixation points 1140 generally resemble slots for receiving glue, that, once cured, will remain embedded within the fixation point 1140 and adhered to the flexible PCB member 200, thereby maintaining alignment of the PCB member with the strut, while ensuring the two remain bonded together.

FIG. 36 is a perspective view of the distal segment 1124 of FIGS. 30A-30B, including enlarged views illustrating the use of a polymer overlay to join a flexible PCB member to a corresponding support element / strut of the distal segment. In the illustrated embodiment, a polymer material may be used as a substrate for attaching the flexible PCB member to a given strut. In the illustrated example, the polymer may be adhered to a given strut via a thermal and/or mechanical process, such that the surface of the polymer substrate in contact with the strut deforms and the fixation points (shown as a slot and holes) act as locations for a "melt pool" to form during the polymer reflow process. Such an arrangement creates a bond point for the polymer substrate to anchor, at least on one side, if not both sides, of the strut, in which the polymer material may weld together in this pool to form a crosslinked plug. More specifically, the formation of the melt pools (as a result of the fixation points (shown as slots and holes)) occurs post processing (i.e., post polymer reflow process), and in turn, facilitates polymer crosslinking to occur to form a bond, further strengthening adherence of the polymer to a strut.

FIG. 37 is a perspective view of the distal segment 124 of FIGS. 9A-9B, for example, illustrating loading of a polymer sleeve over a wire support element of the distal segment, in which a flexible PCB is joined to the polymer sleeve.

FIG. 38 is a perspective view of the distal segment of FIG. 28, illustrating placement of polymer caps or tubing to the free, distal ends of the support elements / struts. As illustrated, in some embodiments, adjacent struts may be joined to one another via placement of a tube over respective distal ends of the adjacent struts.

As previously described, the invention provides improved manufacturing techniques for bonding the PCB members to support elements of a given proximal or distal segment. In particular, the present invention contemplates the use of bonding, thermal, and mechanical processes for joining PCB members to respective support elements, which may include one or more of adhesion, mechanical, lamination, polymer reflow, induction heating, spot welding, and laser welding processes. For example, in one embodiment, attaching a PCB member to a respective support element includes a reflow process in which a flexible PCB member is positioned relative to a respective support element, one or more polymer layers are then disposed around the flexible PCB member, and heat is then applied resulting in the one or more polymer layers encasing the flexible PCB member and subsequently affixing the flexible PCB member to the underlying support element of the end effector segment. In another embodiment, polymer sleeves may be secured to the support elements, thereby providing a substrate upon which a flexible PCB member may be positioned and subsequently attached. It should be noted that the process of securing the polymer sleeve and bonding the flexible PCB member to the support element may occur simultaneously via the application of pressure and heat.

FIGS. 39 and 40 are an enlarged views illustrating placement of polymer sleeves 1142 over a support element / strut of the distal segment of FIG. 29. FIG. 39 illustrates a sleeve extending along a length of the support element / strut, while FIG. 40 illustrates discrete portions of a polymer sleeve positioned along a length of the support element / strut (generally forming runners 1144) at specific locations of the support element / strut.

FIG. 41 is a perspective view of the distal segment in which each of the plurality of support elements / struts includes a polymer sleeve running a majority of the length and further include a flexible PCB member attached thereto.

FIG. 42 is a perspective view of the distal segment in which each of the plurality of support elements / struts includes polymer runners 1144(1) and 1144(2) provided at discrete locations along a length of each and further include a flexible PCB member attached to the runners on each support element / strut.

FIG. 43 is a perspective view of a distal segment in which each of the support elements / struts has been laser cut and further includes multiple fixation points to which flexible PCB members may be attached.

FIGS. 44A through 44D illustrate a reflow process for positioning and affixing flexible PCB members and polymer tubing to the struts of the proximal segment 1122. In particular, polymer sleeves may be secured to the struts via a polymer reflow process, thereby providing a substrate upon which a flexible PCB member may be positioned and subsequently attached. As shown in FIGS. 44A and 44B, one end of each of the struts 1130 and 1132 may be moveable (temporarily) away from the collar member, thereby allowing for polymer tubing to be positioned over a given strut at one or more discrete locations along the strut. Upon being positioned at a desired location, heat-shrink tubing may then be placed over the polymer tubing (shown in FIG. 44C), upon which heat may be applied resulting in the polymer tubing adhering to and encasing the underlying strut. As previously described, the one or more fixation points on a given strut strengthen the bond between the polymer tubing and the underlying strut. Subsequently, flexible PCB members may then be positioned over the polymer tubing, which now serves as the underlying substrate to which the PCB members will be joined. In particular, as shown in 44D, the flexible PCB members may be positioned over the polymer tubing and joined thereto via a reflow process, in a similar manner as was used to join the polymer tubing to the strut. In particular, heat-shrink tubing may be placed over the flexible PCB member and the polymer tubing (shown in FIG. 44D), upon which heat may be applied resulting in the flexible PCB member adhering to the underlying polymer tubing.

FIGS. 45A and 45B illustrate a reflow process for positioning and affixing flexible PCB members and polymer tubing to the struts of the distal segment 1124. The polymer tubing/sleeve and flexible PCB members are affixed to the plurality of struts 1136 in a similar manner (i.e., reflow process) as described with respect to the reflow process for affixing the polymer tubing/sleeve and flexible PCB members to the struts 1130 and 1132 of the proximal segment 1122.

As previously described herein, at least one of the proximal and distal segments 1122 and 1124 may further include cross members coupling one or more of the plurality of struts to one another. For example, FIG. 46 is a perspective view of a distal segment 1124 in which a plurality of support elements / struts 1136 are connected by cross members in a first configuration, while FIG. 47 is a perspective view of a distal segment 1124 in which a plurality of support elements / struts are connected by cross members in a second configuration.

Referring to FIG. 46, pairs of struts 1136 are coupled to one another via a cross-member 1146 extending therebetween. In particular, the cross members 1146 are arranged in a first configuration with respect to the struts, notably an s-shape or chevron-shape pattern, wherein immediately adjacent support elements / struts are connected to one another via a corresponding strut member. Referring to FIG. 47, the cross members 1146 are arranged in a leap-frog pattern, such that every other one of the plurality of the struts 1136 are connected by a corresponding cross member 1146. For example, struts 1136(1) and 1136(3) are coupled to one another via cross member 1146(1), while struts 1136(2) and 1136(4) are coupled to one another via cross member 1146(2).

The inclusion of cross members 1146 provides notable advantages. For example, inclusion of cross members, particularly with respect to the distal segment 1124, increases the available surface area upon which flexible PCM members may be placed, thereby allowing more options in terms of electrode numbers, placements, and overall design. Furthermore, cross members may be placed at specific locations so at to vary radial stiffness as desired, which may further improve tissue apposition.

FIGS. 48A and 48B are side views illustrating a distal segment 1124 transitioning to an expanded, deployed configuration and corresponding movement of a cross member locking mechanism coupling at least two struts to one another. The cross-member locking mechanism is configured to achieve a locked state when the segment is in a fully deployed configuration (i.e., when the corresponding struts 1136 have reached a certain point of expansion) to thereby inhibit retraction of the plurality of struts unless acted upon by a certain level of force. As illustrated, the cross member locking mechanism includes a runner 1148 that slides along a length of a shaft 1152 extending along a longitudinal axis of the device. A plurality of stretcher members 1150 extend from the runner 1148 and are individually coupled to corresponding struts 1136. Movement of the runner 1148 toward the proximal end of the distal segment 1124 results in expansion of the struts in an outward direction and towards the fully deployed configuration as a result of force placed upon each strut from a corresponding stretcher member 1150. Once the runner 1148 reaches a certain position along the shaft 1152, the cross member locking mechanism achieves a locked position in which the struts 1136 remain in the fully deployed configuration. Movement of the runner 1148 along the shaft 1152 toward a distal tip or end cap 1154 positioned at a distal most end of the shaft 1152 results in transitioning of the distal segment 1124, notably the struts 1136, to a retracted configuration.

FIGS. 49A-49C, 50, and 51A-51B illustrate different embodiments of struts of the distal segment 1124. Similar to the struts illustrated in FIGS. 44A-44C, portions of a given strut illustrated in FIGS 49A-49C, 50, and 51A-51B may moveable so as to allow for positioning and affixing flexible PCB members and polymer tubing thereupon for subsequent polymer reflow process. For example, unlike the struts of FIGS. 44A-44C, in which one end of a given strut is moveable relative to the collar member (i.e., temporarily separatable from the collar member), each of the struts illustrated in FIGS. 49A-49C, 50, and 51A-51B is generally composed of two portions that are temporarily separatable from one another at a mid-portion of the strut.

In particular, as shown in FIG. 49A, each strut may be comprised of two portions (a first portion and a second portion), each of which has a proximal end directly connected to a collar member and an opposing distal free end extending away from the collar member. The distal free end of each of the first and second portions of a given strut may further include an attachment feature allowing for each free end to be mechanically coupled to one another, thereby forming a looped strut. For example, as shown in FIGS. 49B and 49C, each free end of the first and second portions may include a pinhole or other aperture allowing for alignment as well as mechanically locking the free ends to one another. For example, in some embodiments, a pin may be fixed within the pinholes once aligned with one another to thereby couple the free ends to one another. In other embodiments, one free end my include a pin, button, protrusion, or the like shaped and/or sized to be received within a corresponding aperture in the other free end and thereby couple the free ends to one another. The free ends may have any contemplated shape. For example, the free end of the first portion of a strut illustrated in FIGS. 49A-49C may have a distinctive shape, generally in the form of an hourglass. However, the free ends can have any contemplated geometry. For example, as illustrated in FIG. 50, the free end of the first portion of a strut is generally linear.

As shown in FIGS. 51A and 51B, the distal free ends of the first and second portions of struts are shaped so as to cooperatively couple to one another via mechanical engagement. In particular, the free end of a first portion may include a flange member while the free end of the second portion may include a slot for receiving the flange member within and thereby couple the free ends to one another.

An advantage to having a mid-body separation for a given strut, as illustrated in FIGS. 49A-49C, 50, and 51A-51B, allows loading of components (e.g., flexible PCB members and polymer tubing) in both directions along a given strut, as opposed to the embodiments illustrated in FIGS. 44A-44C (in which loading of the components upon a strut is performed at a single, proximal free end of a strut and is completed unidirectionally therefrom). By having the separation of a given strut at a mid-portion, and thus having proximal ends fixed in place to the collar member, attachment of the strut to the collar member is more robust and stronger than the embodiments of FIGS. 44A-44C. Furthermore, loading of components upon the strut may be easier and faster.

It should be noted that, while FIGS. 49A-49C, 50, and 51A-51B illustrate struts of the distal segment, the mid-body separation and various attachment features described herein may be applied to struts of the proximal segment.

FIGS. 52 and 53 are perspective views of the distal segment of FIG. 28, each figure illustrating placement of polymer tubing to the free, distal ends of the support elements / struts and further joining adjacent struts to one another via placement of a wire or braided tube over respective distal ends of the adjacent struts and fixing the wire or braided tube in place via a polymer reflow process. For example, as illustrated in FIG. 52, each end of a metal wire may be placed inside a polymer tube placed over a respective strut and, upon undergoing a polymer reflow process, each end of the wire may be fixed in place, thereby joining two adjacent struts. In some embodiments, the wire may be exposed (i.e., the polymer tube does not completely melt over the surface of the wire), while in other embodiments, the entire wire may be encased within the polymer. FIG. 53 illustrates the use of a braided tube (e.g., metal braided tube) used for joining two adjacent struts (in a similar process as described with reference to FIG. 52).

The following provides a detailed description of the various capabilities of systems and methods of the present invention, including, but not limited to, neuromodulation monitoring, feedback, and mapping capabilities, which, in turn, allowing for detection of anatomical structures and function, neural identification and mapping, and anatomical mapping, for example.

### Neuromodulation Monitoring, Feedback and Mapping Capabilities

As previously described, the system 100 includes a console 104 to which the device 102 is to be connected. The console 104 is configured to provide various functions for the neuromodulation device 102, which may include, but is not limited to, controlling, monitoring, supplying, and/or otherwise supporting operation of the neuromodulation device 102. The console 104 can further be configured to generate a selected form and/or magnitude of energy for delivery to tissue or nerves at the target site via the end effector 114, and therefore the console 104 may have different configurations depending on the treatment modality of the device 102. For example, when device 102 is configured for electrode-based, heat-element-based, and/or transducer-based treatment, the console 104 includes an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy. When the device 102 is configured for cryotherapeutic treatment, the console 104 can include a refrigerant reservoir (not shown), and can be configured to supply the device 102 with refrigerant. Similarly, when the device 102 is configured for chemical-based treatment (e.g., drug infusion), the console 104 can include a chemical reservoir (not shown) and can be configured to supply the device 102 with one or more chemicals.

In some embodiments, the console 104 may include a controller 107 communicatively coupled to the neuromodulation device 102. However, in the embodiments described herein, the controller 107 may generally be carried by and provided within the handle 118 of the neuromodulation device 102. The controller 107 is configured to initiate, terminate, and/or adjust operation of one or more electrodes provided by the end effector 114 directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, perform nerve mapping, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via mapping/evaluation/feedback algorithms 110. For example, the mapping/evaluation/feedback algorithms 110 can be configured to provide information associated with the location of nerves at the treatment site, the location of other anatomical structures (e.g., vessels) at the treatment site, the temperature at the treatment site during monitoring and modulation, and/or the effect of the therapeutic neuromodulation on the nerves at the treatment site. In certain embodiments, the mapping/evaluation/feedback algorithm 110 can include features to confirm efficacy of the treatment and/or enhance the desired performance of the system 100. For example, the mapping/evaluation/feedback algorithm 110, in conjunction with the controller 107 and the end effector 114, can be configured to monitor neural activity and/or temperature at the treatment site during therapy and automatically shut off the energy delivery when the neural activity and/or temperature reaches a predetermined threshold (e.g., a threshold reduction in neural activity, a threshold maximum temperature when applying RF energy, or a threshold minimum temperature when applying cryotherapy). In other embodiments, the mapping/evaluation/feedback algorithm 110, in conjunction with the controller 107, can be configured to automatically terminate treatment after a predetermined maximum time, a predetermined maximum impedance or resistance rise of the targeted tissue (i.e., in comparison to a baseline impedance measurement), a predetermined maximum impedance of the targeted tissue), and/or other threshold values for biomarkers associated with autonomic function. This and other information associated with the operation of the system 100 can be communicated to the operator via a display 112 (e.g., a monitor, touchscreen, user interface, etc.) on the console 104 and/or a separate display (not shown) communicatively coupled to the console 104.

In various embodiments, the end effector 114 and/or other portions of the system 100 can be configured to detect various bioelectric-parameters of the tissue at the target site, and this information can be used by the mapping/evaluation/feedback algorithms 110 to determine the anatomy at the target site (e.g., tissue types, tissue locations, vasculature, bone structures, foramen, sinuses, etc.), locate neural structures, differentiate between different types of neural structures, map the anatomical and/or neural structure at the target site, and/or identify neuromodulation patterns of the end effector 114 with respect to the patient's anatomy. For example, the end effector 114 can be used to detect resistance, complex electrical impedance, dielectric properties, temperature, and/or other properties that indicate the presence of neural fibers and/or other anatomical structures in the target region. In certain embodiments, the end effector 114, together with the mapping/evaluation/feedback algorithms 110, can be used to determine resistance (rather than impedance) of the tissue (i.e., the load) to more accurately identify the characteristics of the tissue. The mapping/evaluation/feedback algorithms 110 can determine resistance of the tissue by detecting the actual power and current of the load (e.g., via the electrodes 136).

In some embodiments, the system 100 provides resistance measurements with a high degree of accuracy and a very high degree of precision, such as precision measurements to the hundredths of an Ohm (e.g., 0.01Ω) for the range of 1-50Q. The high degree of resistance detection accuracy provided by the system 100 allows for the detection sub-microscale structures, including the firing of neural structures, differences between neural structures and other anatomical structures (e.g., blood vessels), and event different types of neural structures. This information can be analyzed by the mapping/evaluation/feedback algorithms and/or the controller 107 and communicated to the operator via a high resolution spatial grid (e.g., on the display 112) and/or other type of display to identify neural structures and other anatomy at the treatment site and/or indicate predicted neuromodulation regions based on the ablation pattern with respect to the mapped anatomy.

As previously described, in certain embodiments, each electrode 136 can be operated independently of the other electrodes 136. For example, each electrode can be individually activated and the polarity and amplitude of each electrode can be selected by an operator or a control algorithm executed by the controller 107. The selective independent control of the electrodes 136allows the end effector 114 to detect information and deliver RF energy to highly customized regions. For example, a select portion of the electrodes 136 can be activated to target specific neural fibers in a specific region while the other electrodes 136 remain inactive. In certain embodiments, for example, electrodes 136 may be activated across the portion of the second segment 124 that is adjacent to tissue at the target site, and the electrodes 136 that are not proximate to the target tissue can remain inactive to avoid applying energy to non-target tissue. In addition, the electrodes 136 can be individually activated to stimulate or therapeutically modulate certain regions in a specific pattern at different times (e.g., via multiplexing), which facilitates detection of anatomical parameters across a zone of interest and/or regulated therapeutic neuromodulation.

The electrodes 136 can be electrically coupled to the energy generator 106 via wires (not shown) that extend from the electrodes 136, through the shaft 116, and to the energy generator 106. When each of the electrodes 136 is independently controlled, each electrode 136 couples to a corresponding wire that extends through the shaft 116. This allows each electrode 136 to be independently activated for stimulation or neuromodulation to provide precise ablation patterns and/or individually detected via the console 104 to provide information specific to each electrode 136 for neural or anatomical detection and mapping. In other embodiments, multiple electrodes 136 can be controlled together and, therefore, multiple electrodes 136 can be electrically coupled to the same wire extending through the shaft 116. The energy generator 16 and/or components (e.g., a control module) operably coupled thereto can include custom algorithms to control the activation of the electrodes 136. For example, the RF generator can deliver RF power at about 200-100 W to the electrodes 136, and do so while activating the electrodes 136 in a predetermined pattern selected based on the position of the end effector 114 relative to the treatment site and/or the identified locations of the target nerves. In other embodiments, the energy generator 106 delivers power at lower levels (e.g., less than 1 W, 1-5 W, 5-15 W, 15-50 W, 50-150 W, etc.) for stimulation and/or higher power levels. For example, the energy generator 106 can be configured to delivery stimulating energy pulses of 1-3 W via the electrodes 136 to stimulate specific targets in the tissue.

As previously described, the end effector 114 can further include one or more temperature sensors disposed on the flexible first and second segments 122, 124 and/or other portions of the end effector 114 and electrically coupled to the console 104 via wires (not shown) that extend through the shaft 116. In various embodiments, the temperature sensors can be positioned proximate to the electrodes 136 to detect the temperature at the interface between tissue at the target site and the electrodes 136. In other embodiments, the temperature sensors can penetrate the tissue at the target site (e.g., a penetrating thermocouple) to detect the temperature at a depth within the tissue. The temperature measurements can provide the operator or the system with feedback regarding the effect of the therapeutic neuromodulation on the tissue. For example, in certain embodiments the operator may wish to prevent or reduce damage to the tissue at the treatment site (e.g., the sino-nasal mucosa), and therefore the temperature sensors can be used to determine if the tissue temperature reaches a predetermined threshold for irreversible tissue damage. Once the threshold is reached, the application of therapeutic neuromodulation energy can be terminated to allow the tissue to remain intact and avoid significant tissue sloughing during wound healing. In certain embodiments, the energy delivery can automatically terminate based on the mapping/evaluation/feedback algorithm 110 stored on the console 104 operably coupled to the temperature sensors.

In certain embodiments, the system 100 can determine the locations and/or morphology of neural structures and/or other anatomical structures before therapy such that the therapeutic neuromodulation can be applied to precise regions including target neural structures, while avoiding negative effects on non-target structures, such as blood vessels. As described in further detail below, the system 100 can detect various bioelectrical parameters in an interest zone (e.g., within in the sino-nasal cavity) to determine the location and morphology of various neural structures (e.g., different types of neural structures, neuronal directionality, etc.) and/or other tissue (e.g., glandular structures, vessels, bony regions, etc.). In some embodiments, the system 100 is configured to measure bioelectric potential. To do so, one or more of the electrodes 136 is placed in contact with an epithelial surface at a region of interest (e.g., a treatment site). Electrical stimuli (e.g., constant or pulsed currents at one or more frequencies) are applied to the tissue by one or more electrodes 136 at or near the treatment site, and the voltage and/or current differences at various different frequencies between various pairs of electrodes 136 of the end effector 114 may be measured to produce a spectral profile or map of the detected bioelectric potential, which can be used to identify different types of tissues (e.g., vessels, neural structures, and/or other types of tissue) in the region of interest. For example, current (i.e., direct or alternating current) can be applied to a pair of electrodes 136 adjacent to each other and the resultant voltages and/or currents between other pairs of adjacent electrodes 136 are measured. It will be appreciated that the current injection electrodes 136 and measurement electrodes 136 need not be adjacent, and that modifying the spacing between the two current injection electrodes 136 can affect the depth of the recorded signals. For example, closely-spaced current injection electrodes 136 provided recorded signals associated with tissue deeper from the surface of the tissue than further spaced apart current injection electrodes 136 that provide recorded signals associated with tissue at shallower depths. Recordings from electrode pairs with different spacings may be merged to provide additional information on depth and localization of anatomical structures.

Further, complex impedance and/or resistance measurements of the tissue at the region of interest can be detected directly from current-voltage data provided by the bioelectric potential measurements while differing levels of frequency currents are applied to the tissue (e.g., via the end effector 114), and this information can be used to map the neural and anatomical structures by the use of frequency differentiation reconstruction. Applying the stimuli at different frequencies will target different stratified layers or cellular bodies or clusters. At high signal frequencies (e.g., electrical injection or stimulation), for example, cell membranes of the neural structures do not impede current flow, and the current passes directly through the cell membranes. In this case, the resultant measurement (e.g., impedance, resistance, capacitance, and/or induction) is a function of the intracellular and extracellular tissue and liquids. At low signal frequencies, the membranes impede current flow to provide different defining characteristics of the tissues, such as the shapes of the cells or cell spacing. The stimulation frequencies can be in the megahertz range, in the kilohertz range (e.g., 400-500 kHz, 450-480 kHz, etc.), and/or other frequencies attuned to the tissue being stimulated and the characteristics of the device being used. The detected complex impedance or resistances levels from the zone of interest can be displayed to the user (e.g., via the display 112) to visualize certain structures based on the stimulus frequency.

Further, the inherent morphology and composition of the anatomical structures in the sino-nasal region react differently to different frequencies and, therefore, specific frequencies can be selected to identify very specific structures. For example, the morphology or composition of targeted structures for anatomical mapping may depend on whether the cells of tissue or other structure are membranonic, stratified, and/or annular. In various embodiments, the applied stimulation signals can have predetermined frequencies attuned to specific neural structures, such as the level of myelination and/or morphology of the myelination. For example, second axonal parasympathetic structures are poorly myelinated than sympathetic nerves or other structures and, therefore, will have a distinguishable response (e.g., complex impedance, resistance, etc.) with respect to a selected frequency than sympathetic nerves. Accordingly, applying signals with different frequencies to the target site can distinguish the targeted parasympathetic nerves from the non-targeted sensory nerves, and therefore provide highly specific target sites for neural mapping before or after therapy and/or neural evaluation post-therapy. In some embodiments, the neural and/or anatomical mapping includes measuring data at a region of interest with at least two different frequencies to identify certain anatomical structures such that the measurements are taken first based on a response to an injection signal having a first frequency and then again based on an injection signal having a second frequency different from the first. For example, there are two frequencies at which hypertrophied (i.e., disease-state characteristics) sub-mucosal targets have a different electrical conductivity or permittivity compared to "normal" (i.e., healthy) tissue. Complex conductivity may be determined based on one or more measured physiological parameters (e.g., complex impedance, resistance, dielectric measurements, dipole measurements, etc.) and/or observance of one or more confidently known attributes or signatures. Furthermore, the system 100 can also apply neuromodulation energy via the electrodes 136 at one or more predetermined frequencies attuned to a target neural structure to provide highly targeted ablation of the selected neural structure associated with the frequency(ies). This highly targeted neuromodulation also reduces the collateral effects of neuromodulation therapy to non-target sites/structures (e.g., blood vessels) because the targeted signal (having a frequency tuned to a target neural structure) will not have the same modulating effects on the non-target structures.

Accordingly, bioelectric properties, such as complex impedance and resistance, can be used by the system 100 before, during, and/or after neuromodulation therapy to guide one or more treatment parameters. For example, before, during, and/or after treatment, impedance or resistance measurements may be used to confirm and/or detect contact between one or more electrodes 136 and the adjacent tissue. The impedance or resistance measurements can also be used to detect whether the electrodes 136 are placed appropriately with respect to the targeted tissue type by determining whether the recorded spectra have a shape consistent with the expected tissue types and/or whether serially collected spectra were reproducible. In some embodiments, impedance or resistance measurements may be used to identify a boundary for the treatment zone (e.g., specific neural structures that are to be disrupted), anatomical landmarks, anatomical structures to avoid (e.g., vascular structures or neural structures that should not be disrupted), and other aspects of delivering energy to tissue.

The bioelectric information can be used to produce a spectral profile or map of the different anatomical features tissues at the target site, and the anatomical mapping can be visualized in a 3D or 2D image via the display 112 and/or other user interface to guide the selection of a suitable treatment site. This neural and anatomical mapping allows the system 100 to accurately detect and therapeutically modulate the postganglionic parasympathetic neural fibers that innervate the mucosa at the numerous neural entrance points into the sino-nasal cavity. Further, because there are not any clear anatomical markers denoting the location of the SPF, accessory foramen, and microforamina, the neural mapping allows the operator to identify and therapeutically modulate nerves that would otherwise be unidentifiable without intricate dissection of the mucosa. In addition, anatomical mapping also allows the clinician to identify certain structures that the clinician may wish to avoid during therapeutic neural modulation (e.g., certain arteries). The neural and anatomical bioelectric properties detected by the system 100 can also be used during and after treatment to determine the real-time effect of the therapeutic neuromodulation on the treatment site. For example, the mapping/evaluation/feedback algorithms 110 can also compare the detected neural locations and/or activity before and after therapeutic neuromodulation, and compare the change in neural activity to a predetermined threshold to assess whether the application of therapeutic neuromodulation was effective across the treatment site.

In various embodiments, the system 100 can also be configured to map the expected therapeutic modulation patterns of the electrodes 136 at specific temperatures and, in certain embodiments, take into account tissue properties based on the anatomical mapping of the target site. For example, the system 100 can be configured to map the ablation pattern of a specific electrode ablation pattern at the 45° C. isotherm, the 55° C. isotherm, the 65° C. isotherm, and/or other temperature/ranges (e.g., temperatures ranging from 45° C. to 70° C. or higher) depending on the target site and/or structure.

The system 100 may provide, via the display 112, three-dimensional views of such projected ablation patterns of the electrodes 136 of the end effector 114. The ablation pattern mapping may define a region of influence that each electrode 136 has on the surrounding tissue. The region of influence may correspond to the region of tissue that would be exposed to therapeutically modulating energy based on a defined electrode activation pattern (i.e., one, two, three, four, or more electrodes on any given strut of the first and second segments 122, 124). In other words, the ablation pattern mapping can be used to illustrate the ablation pattern of any number of electrodes 136, any geometry of the electrode layout, and/or any ablation activation protocol (e.g., pulsed activation, multi-polar/sequential activation, etc.).

In some embodiments, the ablation pattern may be configured such that each electrode 136 has a region of influence surrounding only the individual electrode 136 (i.e., a "dot" pattern). In other embodiments, the ablation pattern may be such that two or more electrodes 136 may link together to form a sub-grouped regions of influence that define peanut-like or linear shapes between two or more electrodes 136. In further embodiments, the ablation pattern can result in a more expansive or contiguous pattern in which the region of influence extends along multiple electrodes 136 (e.g., along each strut). In still further embodiments, the ablation pattern may result in different regions of influence depending upon the electrode activation pattern, phase angle, target temperature, pulse duration, device structure, and/or other treatment parameters. The three-dimensional views of the ablation patterns can be output to the display 112 and/or other user interfaces to allow the clinician to visualize the changing regions of influence based on different durations of energy application, different electrode activation sequences (e.g., multiplexing), different pulse sequences, different temperature isotherms, and/or other treatment parameters. This information can be used to determine the appropriate ablation algorithm for a patient's specific anatomy. In other embodiments, the three-dimensional visualization of the regions of influence can be used to illustrate the regions from which the electrodes 136 detect data when measuring bioelectrical properties for anatomical mapping. In this embodiment, the three dimensional visualization can be used to determine which electrode activation pattern should be used to determine the desired properties (e.g., impedance, resistance, etc.) in the desired area. In certain embodiments, it may be better to use dot assessments, whereas in other embodiments it may be more appropriate to detect information from linear or larger contiguous regions.

In some embodiments, the mapped ablation pattern is superimposed on the anatomical mapping to identify what structures (e.g., neural structures, vessels, etc.) will be therapeutically modulated or otherwise affected by the therapy. An image may be provided to the surgeon which includes a digital illustration of a predicted or planned neuromodulation zone in relation to previously identified anatomical structures in a zone of interest. For example, the illustration may show numerous neural structures and, based on the predicted neuromodulation zone, identifies which neural structures are expected to be therapeutically modulated. The expected therapeutically modulated neural structures may be shaded to differentiate them from the non-affected neural structures. In other embodiments, the expected therapeutically modulated neural structures can be differentiated from the non-affected neural structures using different colors and/or other indicators. In further embodiments, the predicted neuromodulation zone and surrounding anatomy (based on anatomical mapping) can be shown in a three dimensional view (and/or include different visualization features (e.g., color-coding to identify certain anatomical structures, bioelectric properties of the target tissue, etc.). The combined predicted ablation pattern and anatomical mapping can be output to the display 112 and/or other user interfaces to allow the clinician to select the appropriate ablation algorithm for a patient's specific anatomy.

The imaging provided by the system 100 allows the clinician to visualize the ablation pattern before therapy and adjust the ablation pattern to target specific anatomical structures while avoiding others to prevent collateral effects. For example, the clinician can select a treatment pattern to avoid blood vessels, thereby reducing exposure of the vessel to the therapeutic neuromodulation energy. This reduces the risk of damaging or rupturing vessels and, therefore, prevents immediate or latent bleeding. Further, the selective energy application provided by the neural mapping reduces collateral effects of the therapeutic neuromodulation, such as tissue sloughing off during wound healing (e.g., 1-3 weeks post ablation), thereby reducing the aspiration risk associated with the neuromodulation procedure.

The system 100 can be further configured to apply neuromodulation energy (via the electrodes 136) at specific frequencies attuned to the target neural structure and, therefore, specifically target desired neural structures over non-target structures. For example, the specific neuromodulation frequencies can correspond to the frequencies identified as corresponding to the target structure during neural mapping. As described above, the inherent morphology and composition of the anatomical structures react differently to different frequencies. Thus, frequency-tuned neuromodulation energy tailored to a target structure does not have the same modulating effects on non-target structures. More specifically, applying the neuromodulation energy at the target-specific frequency causes ionic agitation in the target neural structure, leading to differentials in osmotic potentials of the targeted neural structures and dynamic changes in neuronal membronic potentials (resulting from the difference in intra-cellular and extra-cellular fluidic pressure). This causes degeneration, possibly resulting in vacuolar degeneration and, eventually, necrosis at the target neural structure, but is not expected to functionally affect at least some non-target structures (e.g., blood vessels). Accordingly, the system 100 can use the neural-structure specific frequencies to both (1) identify the locations of target neural structures to plan electrode ablation configurations (e.g., electrode geometry and/or activation pattern) that specifically focus the neuromodulation on the target neural structure; and (2) apply the neuromodulation energy at the characteristic neural frequencies to selectively ablate the neural structures responsive to the characteristic neural frequencies. For example, the end effector 114 of the system 100 may selectively stimulate and/or modulate parasympathetic fibers, sympathetic fibers, sensory fibers, alpha/beta/delta fibers, C-fibers, anoxic terminals of one or more of the foregoing, insulated over non-insulated fibers (regions with fibers), and/or other neural structures. In some embodiments, the system 100 may also selectively target specific cells or cellular regions during anatomical mapping and/or therapeutic modulation, such as smooth muscle cells, sub-mucosal glands, goblet cells, stratified cellular regions within the sino-nasal mucosa. Therefore, the system 100 provides highly selective neuromodulation therapy specific to targeted neural structures, and reduces the collateral effects of neuromodulation therapy to non-target structures (e.g., blood vessels).

The present disclosure provides a method of anatomical mapping and therapeutic neuromodulation. The method includes expanding an end effector (i.e., end effector 114) at a zone of interest ("interest zone"), such as in a portion of the sino-nasal cavity. For example, the end effector 114 can be expanded such that at least some of the electrodes 136 are placed in contact with mucosal tissue at the interest zone. The expanded device can then take bioelectric measurements via the electrodes 136 and/or other sensors to ensure that the desired electrodes are in proper contact with the tissue at the interest zone. In some embodiments, for example, the system 100 detects the impedance and/or resistance across pairs of the electrodes 136 to confirm that the desired electrodes have appropriate surface contact with the tissue and that all of the electrodes are 136 functioning properly.

The method continues by optionally applying an electrical stimulus to the tissue, and detecting bioelectric properties of the tissue to establish baseline norms of the tissue. For example, the method can include measuring resistance, complex impedance, current, voltage, nerve firing rate, neuromagnetic field, muscular activation, and/or other parameters that are indicative of the location and/or function of neural structures and/or other anatomical structures (e.g., glandular structures, blood vessels, etc.). In some embodiments, the electrodes 136 send one or more stimulation signals (e.g., pulsed signals or constant signals) to the interest zone to stimulate neural activity and initiate action potentials. The stimulation signal can have a frequency attuned to a specific target structure (e.g., a specific neural structure, a glandular structure, a vessel) that allows for identification of the location of the specific target structure. The specific frequency of the stimulation signal is a function of the host permeability and, therefore, applying the unique frequency alters the tissue attenuation and the depth into the tissue the RF energy will penetrate. For example, lower frequencies typically penetrate deeper into the tissue than higher frequencies.

Pairs of the non-stimulating electrodes 136 of the end effector 114 can then detect one or more bioelectric properties of the tissue that occur in response to the stimulus, such as impedance or resistance. For example, an array of electrodes (e.g., the electrodes 136) can be selectively paired together in a desired pattern (e.g., multiplexing the electrodes 136) to detect the bioelectric properties at desired depths and/or across desired regions to provide a high level of spatial awareness at the interest zone. In certain embodiments, the electrodes 136 can be paired together in a time-sequenced manner according to an algorithm (e.g., provided by the mapping/evaluation/feedback algorithms 110). In various embodiments, stimuli can be injected into the tissue at two or more different frequencies, and the resultant bioelectric responses (e.g., action potentials) in response to each of the injected frequencies can be detected via various pairs of the electrodes 136. For example, an anatomical or neural mapping algorithm can cause the end effector 114 to deliver pulsed RF energy at specific frequencies between different pairs of the electrodes 136 and the resultant bioelectric response can be recorded in a time sequenced rotation until the desired interest zone is adequately mapped (i.e., "multiplexing"). For example, the end effector 114 can deliver stimulation energy at a first frequency via adjacent pairs of the electrodes 136 for a predetermined time period (e.g., 1-50 milliseconds), and the resultant bioelectric activity (e.g., resistance) can be detected via one or more other pairs of electrodes 136 (e.g., spaced apart from each other to reach varying depths within the tissue). The end effector 114 can then apply stimulation energy at a second frequency different from the first frequency, and the resultant bioelectric activity can be detected via the other electrodes. This can continue when the interest zone has been adequately mapped at the desired frequencies. As described in further detail below, in some embodiments the baseline tissue bioelectric properties (e.g., nerve firing rate) are detected using static detection methods (without the injection of a stimulation signal).

After detecting the baseline bioelectric properties, the information can be used to map anatomical structures and/or functions at the interest zone. For example, the bioelectric properties detected by the electrodes 136 can be amazed via the mapping/evaluation/feedback algorithms 110, and an anatomical map can be output to a user via the display 112. In some embodiments, complex impedance, dielectric, or resistance measurements can be used to map parasympathetic nerves and, optionally, identify neural structures in a diseased state of hyperactivity. The bioelectric properties can also be used to map other non-target structures and the general anatomy, such as blood vessels, bone, and/or glandular structures. The anatomical locations can be provided to a user (e.g., on the display 112) as a two-dimensional map (e.g., illustrating relative intensities, illustrating specific sites of potential target structures) and/or as a three-dimensional image. This information can be used to differentiate structures on a submicron, cellular level and identify very specific target structures (e.g., hyperactive parasympathetic nerves). The method can also predict the ablation patterns of the end effector 114 based on different electrode neuromodulation protocol and, optionally, superimpose the predicted neuromodulation patterns onto the mapped anatomy to indicate to the user which anatomical structures will be affected by a specific neuromodulation protocol. For example, when the predicted neuromodulation pattern is displayed in relation to the mapped anatomy, a clinician can determine whether target structures will be appropriately ablated and whether non-target structures (e.g., blood vessels) will be undesirably exposed to the therapeutic neuromodulation energy. Thus, the method can be used for planning neuromodulation therapy to locate very specific target structures, avoid non-target structures, and select electrode neuromodulation protocols.

Once the target structure is located and a desired electrode neuromodulation protocol has been selected, the method continues by applying therapeutic neuromodulation to the target structure. The neuromodulation energy can be applied to the tissue in a highly targeted manner that forms micro-lesions to selectively modulate the target structure, while avoiding non-targeted blood vessels and allowing the surrounding tissue structure to remain healthy for effective wound healing. In some embodiments, the neuromodulation energy can be applied in a pulsed manner, allowing the tissue to cool between modulation pulses to ensure appropriate modulation without undesirably affecting non-target tissue. In some embodiments, the neuromodulation algorithm can deliver pulsed RF energy between different pairs of the electrodes 136 in a time sequenced rotation until neuromodulation is predicted to be complete (i.e., "multiplexing"). For example, the end effector 114 can deliver neuromodulation energy (e.g., having a power of 5-10 W (e.g., 7 W, 8 W, 9W) and a current of about 50-100 mA) via adjacent pairs of the electrodes 136 until at least one of the following conditions is met: (a) load resistance reaches a predefined maximum resistance (e.g., 350Ω); (b) a thermocouple temperature associated with the electrode pair reaches a predefined maximum temperature (e.g., 80° C.); or (c) a predetermined time period has elapsed (e.g., 10 seconds). After the predetermined conditions are met, the end effector 114 can move to the next pair of electrodes in the sequence, and the neuromodulation algorithm can terminate when all of the load resistances of the individual pairs of electrodes is at or above a predetermined threshold (e.g., 100Ω). In various embodiments, the RF energy can be applied at a predetermined frequency (e.g., 450-500 kHz) and is expected to initiate ionic agitation of the specific target structure, while avoiding functional disruption of non-target structures.

During and/or after neuromodulation therapy, the method continues by detecting and, optionally, mapping the post-therapy bioelectric properties of the target site. This can be performed in a similar manner as described above. The post-therapy evaluation can indicate if the target structures (e.g., hyperactive parasympathetic nerves) were adequately modulated or ablated. If the target structures are not adequately modulated (i.e., if neural activity is still detected in the target structure and/or the neural activity has not decreased), the method can continue by again applying therapeutic neuromodulation to the target. If the target structures were adequately ablated, the neuromodulation procedure can be completed.

### Detection of Anatomical Structures and Function

Various embodiments of the present technology can include features that measure bioelectric, dielectric, and/or other properties of tissue at target sites to determine the presence, location, and/or activity of neural structures and other anatomical structures and, optionally, map the locations of the detected neural structures and/or other anatomical structures. For example, the present technology can be used to detect glandular structures and, optionally, their mucoserous functions and/or other functions. The present technology can also be configured to detect vascular structures (e.g., arteries) and, optionally, their arterial functions, volumetric pressures, and/or other functions. The mapping features discussed below can be incorporated into any the system 100 and/or any other devices disclosed herein to provide an accurate depiction of nerves at the target site.

Neural and/or anatomical detection can occur (a) before the application of a therapeutic neuromodulation energy to determine the presence or location of neural structures and other anatomical structures (e.g., blood vessels, glands, etc.) at the target site and/or record baseline levels of neural activity; (b) during therapeutic neuromodulation to determine the real-time effect of the energy application on the neural fibers at the treatment site; and/or (c) after therapeutic neuromodulation to confirm the efficacy of the treatment on the targeted structures (e.g., nerves glands, etc.). This allows for the identification of very specific anatomical structures (even to the micro-scale or cellular level) and, therefore, provides for highly targeted neuromodulation. This enhances the efficacy and efficiency of the neuromodulation therapy. In addition, the anatomical mapping reduces the collateral effects of neuromodulation therapy to non-target sites. Accordingly, the targeted neuromodulation inhibits damage or rupture of blood vessels (i.e., inhibits undesired bleeding) and collateral damage to tissue that may be of concern during wound healing (e.g., when damage tissue sloughs off of the wall of the sino-nasal wall).

In certain embodiments, the systems disclosed herein can use bioelectric measurements, such as impedance, resistance, voltage, current density, and/or other parameters (e.g., temperature) to determine the anatomy, in particular the neural, glandular, and vascular anatomy, at the target site. The bioelectric properties can be detected after the transmission of a stimulus (e.g., an electrical stimulus, such as RF energy delivered via the electrodes 136; i.e., "dynamic" detection) and/or without the transmission of a stimulus (i.e., "static" detection).

Dynamic measurements include various embodiments to excite and/or detect primary or secondary effects of neural activation and/or propagation. Such dynamic embodiments involve the heightened states of neural activation and propagation and use this dynamic measurement for nerve location and functional identification relative to the neighboring tissue types. For example, a method of dynamic detection can include: (1) delivering stimulation energy to a treatment site via a treatment device (e.g., the end effector 114) to excite parasympathetic nerves at the treatment site; (2) measuring one or more physiological parameters (e.g., resistance, impedance, etc.) at the treatment site via a measuring/sensing array of the treatment device (e.g., the electrodes 136); (4) based on the measurements, identifying the relative presence and position of parasympathetic nerves at the treatment site; and (5) delivering ablation energy to the identified parasympathetic nerves to block the detected para-sympathetic nerves.

Static measurements include various embodiments associated with specific native properties of the stratified or cellular composition at or near the treatment site. The static embodiments are directed to inherent biologic and electrical properties of tissue types at or near the treatment site, the stratified or cellular compositions at or near the treatment site, and contrasting both foregoing measurements with tissue types adjacent the treatment site (and that are not targeted for neuromodulation). This information can be used to localize specific targets (e.g., parasympathetic fibers) and non-targets (e.g., vessels, sensory nerves, etc.). For example, a method of static detection can include: (1) before ablation, utilizing a measuring/sensing array of a treatment device (e.g., the electrodes 136) to determine one or more baseline physiological parameters; (2) geometrically identifying inherent tissue properties within a region of interest based on the measured physiological parameters (e.g., resistance, impedance, etc.); (3) delivering ablation energy to one or more nerves within the region of via treatment device interest; (4) during the delivery of the ablation energy, determining one or more mid-procedure physiological parameters via the measuring/sensing array; and (5) after the delivery of ablation energy, determining one or more post-procedure physiological parameters via the measurement/sensing array to determine the effectiveness of the delivery of the ablation energy on blocking the nerves that received the ablation energy.

After the initial static and/or dynamic detection of bioelectric properties, the location of anatomical features can be used to determine where the treatment site(s) should be with respect to various anatomical structures for therapeutically effective neuromodulation of the targeted parasympathetic sino-nasal nerves. The bioelectric and other physiological properties described herein can be detected via electrodes (e.g., the electrodes 136 of the end effector 114), and the electrode pairings on a device (e.g., end effector 114) can be selected to obtain the bioelectric data at specific zones or regions and at specific depths of the targeted regions. The specific properties detected at or surrounding target neuromodulation sites and associated methods for obtaining these properties are described below. These specific detection and mapping methods discussed below are described with reference to the system 100 , although the methods can be implemented on other suitable systems and devices that provide for anatomical identification, anatomical mapping and/or neuromodulation therapy.

### Neural Identification and Mapping

In many neuromodulation procedures, it is beneficial to identify the portions of the nerves that fall within a zone and/or region of influence (referred to as the "interest zone") of the energy delivered by a neuromodulation device 102, as well as the relative three-dimensional position of the neural structures relative to the neuromodulation device 102. Characterizing the portions of the neural structures within the interest zone and/or determining the relative positions of the neural structures within the interest zone enables the clinician to (1) selectively activate target neural structures over non-target structures (e.g., blood vessels), and (2) sub-select specific targeted neural structures (e.g., parasympathetic nerves) over non-target neural structures (e.g., sensory nerves, subgroups of neural structures, neural structures having certain compositions or morphologies). The target structures (e.g., parasympathetic nerves) and non-target structures (e.g., blood vessels, sensory nerves, etc.) can be identified based on the inherent signatures of specific structures, which are defined by the unique morphological compositions of the structures and the bioelectrical properties associated with these morphological compositions. For example, unique, discrete frequencies can be associated with morphological compositions and, therefore, be used to identify certain structures. The target and non-target structures can also be identified based on relative bioelectrical activation of the structures to sub-select specific neuronal structures. Further, target and non-target structures can be identified by the differing detected responses of the structures to a tailored injected stimuli. For example, the systems described herein can detect the magnitude of response of structures and the difference in the responses of anatomical structures with respect to differing stimuli (e.g., stimuli injected at different frequencies).

At least for purposes of this disclosure, a nerve can include the following portions that are defined based on their respective orientations relative to the interest zone: terminating neural structures (e.g., terminating axonal structures), branching neural structures (e.g., branching axonal structures), and travelling neural structures (e.g., travelling axonal structures). For example, terminating neural structures enter the zone but do not exit. As such, terminating neural structures are terminal points for neuronal signaling and activation. Branching neural structures are nerves that enter the interest zone and increase number of nerves exiting the interest zone. Branching neural structures are typically associated with a reduction in relative geometry of nerve bundle. Travelling neural structures are nerves that enter the interest zone and exit the zone with no substantially no change in geometry or numerical value.

The system 100 can be used to detect voltage, current, complex impedance, resistance, permittivity, and/or conductivity, which are tied to the compound action potentials of nerves, to determine and/or map the relative positions and proportionalities of nerves in the interest zone. Neuronal cross-sectional area ("CSA") is expected to be due to the increase in axonic structures. Each axon is a standard size. Larger nerves (in cross-sectional dimension) have a larger number of axons than nerves having smaller cross-sectional dimensions. The compound action responses from the larger nerves, in both static and dynamic assessments, are greater than smaller nerves. This is at least in part because the compound action potential is the cumulative action response from each of the axons. When using static analysis, for example, the system 100 can directly measure and map impedance or resistance of nerves and, based on the determined impedance or resistance, determine the location of nerves and/or relative size of the nerves. In dynamic analysis, the system 100 can be used to apply a stimulus to the interest zone and detect the dynamic response of the neural structures to the stimulus. Using this information, the system 100 can determine and/or map impedance or resistance in the interest zone to provide information related to the neural positions or relative nerve sizes. Neural impedance mapping can be illustrated by showing the varying complex impedance levels at a specific location at differing cross-sectional depths. In other embodiments, neural impedance or resistance can be mapped in a three-dimensional display.

Identifying the portions and/or relative positions of the nerves within the interest zone can inform and/or guide selection of one or more treatment parameters (e.g., electrode ablation patterns, electrode activation plans, etc.) of the system 100 for improving treatment efficiency and efficacy. For example, during neural monitoring and mapping, the system 100 can identify the directionality of the nerves based at least in part on the length of the neural structure extending along the interest zone, relative sizing of the neural structures, and/or the direction of the action potentials. This information can then be used by the system 100 or the clinician to automatically or manually adjust treatment parameters (e.g., selective electrode activation, bipolar and/or multipolar activation, and/or electrode positioning) to target specific nerves or regions of nerves. For example, the system 100 can selectively activate specific electrodes 136, electrode combinations (e.g., asymmetric or symmetric), and/or adjust the bi-polar or multi-polar electrode configuration. In some embodiments, the system 100 can adjust or select the waveform, phase angle, and/or other energy delivery parameters based on the nerve portion/position mapping and/or the nerve proportionality mapping. In some embodiments, structure and/or properties of the electrodes 136 themselves (e.g., material, surface roughening, coatings, cross-sectional area, perimeter, penetrating, penetration depth, surface-mounted, etc.) may be selected based on the nerve portion and proportionality mapping.

In various embodiments, treatment parameters and/or energy delivery parameters can be adjusted to target on-axis or near axis travelling neural structures and/or avoid the activation of traveling neural structures that are at least generally perpendicular to the end effector 114. Greater portions of the on-axis or near axis travelling neural structures are exposed and susceptible to the neuromodulation energy provided by the end effector 114 than a perpendicular travelling neural structure, which may only be exposed to therapeutic energy at a discrete cross-section. Therefore, the end effector 114 is more likely to have a greater effect on the on-axis or near axis travelling neural structures. The identification of the neural structure positions (e.g., via complex impedance or resistance mapping) can also allow targeted energy delivery to travelling neural structures rather than branching neural structures (typically downstream of the travelling neural structures) because the travelling neural structures are closer to the nerve origin and, therefore, more of the nerve is affected by therapeutic neuromodulation, thereby resulting in a more efficient treatment and/or a higher efficacy of treatment. Similarly, the identification of neural structure positions can be used to target travelling and branching neural structures over terminal neural structures. In some embodiments, the treatment parameters can be adjusted based on the detected neural positions to provide a selective regional effect. For example, a clinician can target downstream portions of the neural structures if only wanting to influence partial effects on very specific anatomical structures or positions.

In various embodiments, neural locations and/or relative positions of nerves can be determined by detecting the nerve-firing voltage and/or current over time. An array of the electrodes 136 can be positioned in contact with tissue at the interest zone, and the electrodes 136 can measure the voltage and/or current associated with nerve-firing. This information can optionally be mapped (e.g., on a display 112) to identify the location of nerves in a hyper state (i.e., excessive parasympathetic tone). Rhinitis is at least in part the result of over-firing nerves because this hyper state drives the hyper-mucosal production and hyper-mucosal secretion. Therefore, detection of nerve firing rate via voltage and current measurements can be used to locate the portions of the interest region that include hyper-parasympathetic neural function (i.e., nerves in the diseased state). This allows the clinician to locate specific nerves (i.e., nerves with excessive parasympathetic tone) before neuromodulation therapy, rather than simply targeting all parasympathetic nerves (including non-diseased state parasympathetic nerves) to ensure that the correct tissue is treated during neuromodulation therapy. Further, nerve firing rate can be detected during or after neuromodulation therapy so that the clinician can monitor changes in nerve firing rate to validate treatment efficacy. For example, recording decreases or elimination of nerve firing rate after neuromodulation therapy can indicate that the therapy was effective in therapeutically treating the hyper/diseased nerves.

In various embodiments, the system 100 can detect neural activity using dynamic activation by injecting a stimulus signal (i.e., a signal that temporarily activates nerves) via one or more of the electrodes 136 to induce an action potential, and other pairs of electrodes 136 can detect bioelectric properties of the neural response. Detecting neural structures using dynamic activation involves detecting the locations of action potentials within the interest zone by measuring the discharge rate in neurons and the associated processes. The ability to numerically measure, profile, map, and/or image fast neuronal depolarization for generating an accurate index of activity is a factor in measuring the rate of discharge in neurons and their processes. The action potential causes a rapid increase in the voltage across nerve fiber and the electrical impulse then spreads along the fiber. As an action potential occurs, the conductance of a neural cell membrane changes, becoming about 40 times larger than it is when the cell is at rest. During the action potential or neuronal depolarization, the membrane resistance diminishes by about 80 times, thereby allowing an applied current to enter the intracellular space as well. Over a population of neurons, this leads to a net decrease in the resistance during coherent neuronal activity, such as chronic para-sympathetic responses, as the intracellular space will provide additional conductive ions. The magnitude of such fast changes has been estimated to have local resistivity changes with recording near DC is 2.8-3.7% for peripheral nerve bundles (e.g., including the nerves in the sino-nasal cavity).

Detecting neural structures using dynamic activation includes detecting the locations of action potentials within the interest zone by measuring the discharge rate in neurons and the associated processes. The basis of each this discharge is the action potential, during which there is a depolarization of the neuronal membrane of up to 110 mV or more, lasting approximately 2 milliseconds, and due to the transfer of micromolar quantities of ions (e.g., sodium and potassium) across the cellular membrane. The complex impedance or resistance change due to the neuronal membrane falls from 1000 to 25 Ωcm. The introduction of a stimulus and subsequent measurement of the neural response can attenuate noise and improve signal to noise ratios to precisely focus on the response region to improve neural detection, measurement, and mapping.

In some embodiments, the difference in measurements of physiological parameters (e.g., complex impedance, resistance, voltage) over time, which can reduce errors, can be used to create a neural profiles, spectrums, or maps. For example, the sensitivity of the system 100 can be improved because this process provides repeated averaging to a stimulus. As a result, the mapping function outputs can be a unit-less ratio between the reference and test collated data at a single frequency and/or multiple frequencies and/or multiple amplitudes. Additional considerations may include multiple frequency evaluation methods that consequently expand the parameter assessments, such as resistivity, admittivity, center frequency, or ratio of extra- to intracellular resistivity.

In some embodiments, the system 100 may also be configured to indirectly measure the electrical activity of neural structures to quantify the metabolic recovery processes that accompany action potential activity and act to restore ionic gradients to normal. These are related to an accumulation of ions in the extracellular space. The indirect measurement of electrical activity can be approximately a thousand times larger (in the order of millimolar), and thus are easier to measure and can enhance the accuracy of the measured electrical properties used to generate the neural maps.

The system 100 can perform dynamic neural detection by detecting nerve-firing voltage and/or current and, optionally, nerve firing rate over time, in response to an external stimulation of the nerves. For example, an array of the electrodes 136 can be positioned in contact with tissue at the interest zone, one or more of the electrodes 136 can be activated to inject a signal into the tissue that stimulates the nerves, and other electrodes 136 of the electrode array can measure the neural voltage and/or current due to nerve firing in response to the stimulus. This information can optionally be mapped (e.g., on a display 112) to identify the location of nerves and, in certain embodiments, identify parasympathetic nerves in a hyper state (e.g., indicative of Rhinitis or other diseased state). The dynamic detection of neural activity (voltage, current, firing rate, etc.) can be performed before neuromodulation therapy to detect target nerve locations to select the target site and treatment parameters to ensure that the correct tissue is treated during neuromodulation therapy. Further, dynamic detection of neural activity can be performed during or after neuromodulation therapy to allow the clinician to monitor changes in neural activity to validate treatment efficacy. For example, recording decreases or elimination of neural activity after neuromodulation therapy can indicate that the therapy was effective in therapeutically treating the hyper/diseased nerves.

In some embodiments, a stimulating signal can be delivered to the vicinity of the targeted nerve via one or more penetrating electrodes (e.g., microneedles that penetrate tissue) associated with the end effector 114 and/or a separate device. The stimulating signal generates an action potential, which causes smooth muscle cells or other cells to contract. The location and strength of this contraction can be detected via the penetrating electrode(s) and, thereby, indicate to the clinician the distance to the nerve and/or the location of the nerve relative to the stimulating needle electrode. In some embodiments, the stimulating electrical signal may have a voltage of typically 1-2 mA or greater and a pulse width of typically 100-200 microseconds or greater. Shorter pulses of stimulation result in better discrimination of the detected contraction, but may require more current. The greater the distance between the electrode and the targeted nerve, the more energy is required to stimulate. The stimulation and detection of contraction strength and/or location enables identification of how close or far the electrodes are from the nerve, and therefore can be used to localize the nerve spatially. In some embodiments, varying pulse widths may be used to measure the distance to the nerve. As the needle becomes closer to the nerve, the pulse duration required to elicit a response becomes less and less.

To localize nerves via muscle contraction detection, the system 100 can vary pulse-width or amplitude to vary the energy (Energy=pulse-width*amplitude) of the stimulus delivered to the tissue via the penetrating electrode(s). By varying the stimulus energy and monitoring muscle contraction via the penetrating electrodes and/or other type of sensor, the system 100 can estimate the distance to the nerve. If a large amount of energy is required to stimulate the nerve/contract the muscle, the stimulating/penetrating electrode is far from the nerve. As the stimulating/penetrating electrode, moves closer to the nerve, the amount of energy required to induce muscle contraction will drop. For example, an array of penetrating electrodes can be positioned in the tissue at the interest zone and one or more of the electrodes can be activated to apply stimulus at different energy levels until they induce muscle contraction. Using an iterative process, localize the nerve (e.g., via the mapping/evaluation/feedback algorithm 110).

In some embodiments, the system 100 can measure the muscular activation from the nerve stimulus (e.g., via the electrodes 136) to determine neural positioning for neural mapping, without the use of penetrating electrodes. In this embodiment, the treatment device targets the smooth muscle cells' varicosities surrounding the submucosal glands and the vascular supply, and then the compound muscle action potential. This can be used to summate voltage response from the individual muscle fiber action potentials. The shortest latency is the time from stimulus artifact to onset of the response. The corresponding amplitude is measured from baseline to negative peak and measured in millivolts (mV). Nerve latencies (mean±SD) in adults typically range about 2-6 milliseconds, and more typically from about 3.4±0.8 to about 4.0±0.5 milliseconds. A comparative assessment may then be made which compares the outputs at each time interval (especially pre- and post-energy delivery) in addition to a group evaluation using the alternative sino-nasal cavity. This is expected to provide an accurate assessment of the absolute value of the performance of the neural functioning because muscular action/activation may be used to infer neural action/activation and muscle action/activation is a secondary effect or by-product whilst the neural function is the absolute performance measure.

In some embodiments, the system 100 can record a neuromagnetic field outside of the nerves to determine the internal current of the nerves without physical disruption of the nerve membrane. Without being bound by theory, the contribution to the magnetic field from the current inside the membrane is two orders of magnitude larger than that from the external current, and that the contribution from current within the membrane is substantially negligible. Electrical stimulation of the nerve in tandem with measurements of the magnetic compound action fields ("CAFs") can yield sequential positions of the current dipoles such that the location of the conduction change can be estimated (e.g., via the least-squares method). Visual representation (e.g., via the display 112) using magnetic contour maps can show normal or non-normal neural characteristics (e.g., normal can be equated with a characteristic quadrupolar pattern propagating along the nerve), and therefore indicate which nerves are in a diseases, hyperactive state and suitable targets for neuromodulation.

During magnetic field detection, an array of the electrodes 136 can be positioned in contact with tissue at the interest zone and, optionally, one or more of the electrodes 136 can be activated to inject an electrical stimulus into the tissue. As the nerves in the interest zone fire (either in response to a stimulus or in the absence of it), the nerve generates a magnetic field (e.g., similar to a current carrying wire), and therefore changing magnetic fields are indicative of the nerve nerve-firing rate. The changing magnetic field caused by neural firing can induce a current detected by nearby sensor wire (e.g., the sensor 314) and/or wires associated with the nearby electrodes 136. By measuring this current, the magnetic field strength can be determined. The magnetic fields can optionally be mapped (e.g., on a display 112) to identify the location of nerves and select target nerves (nerves with excessive parasympathetic tone) before neuromodulation therapy to ensure that the desired nerves are treated during neuromodulation therapy. Further, the magnetic field information can be used during or after neuromodulation therapy so that the clinician can monitor changes in nerve firing rate to validate treatment efficacy.

In other embodiments, the neuromagnetic field is measured with a Hall Probe or other suitable device, which can be integrated into the end effector 114 and/or part of a separate device delivered to the interest zone. Alternatively, rather than measuring the voltage in the second wire, the changing magnetic field can be measured in the original wire (i.e. the nerve) using a Hall probe. A current going through the Hall probe will be deflected in the semi-conductor. This will cause a voltage difference between the top and bottom portions, which can be measured. In some aspects of this embodiments, three orthogonal planes are utilized.

In some embodiments, the system 100 can be used to induce electromotive force ("EMF") in a wire (i.e., a frequency-selective circuit, such as a tunable/LC circuit) that is tunable to resonant frequency of a nerve. In this embodiment, the nerve can be considered to be a current carrying wire, and the firing action potential is a changing voltage. This causes a changing current which, in turn, causes a changing magnetic flux (i.e., the magnetic field that is perpendicular to the wire). Under Faraday's Law of Induction/Faraday's Principle, the changing magnetic flux induces EMF (including a changing voltage) in a nearby sensor wire (e.g., integrated into the end effector 114, the sensor 314, and/or other structure), and the changing voltage can be measured via the system 100.

In further embodiments, the sensor wire (e.g., the sensor 314) is an inductor and, therefore, provides an increase of the magnetic linkage between the nerve (i.e., first wire) and the sensor wire (i.e., second wire), with more turns for increasing effect. (e.g., V2,rms=V1,rms (N2/N1)). Due to the changing magnetic field, a voltage is induced in the sensor wire, and this voltage can be measured and used to estimate current changes in the nerve. Certain materials can be selected to enhance the efficiency of the EMF detection. For example, the sensor wire can include a soft iron core or other high permeability material for the inductor.

During induced EMF detection, the end effector 114 and/or other device including a sensor wire is positioned in contact with tissue at the interest zone and, optionally, one or more of the electrodes 136 can be activated to inject an electrical stimulus into the tissue. As the nerves in the interest zone fire (either in response to a stimulus or in the absence of it), the nerve generates a magnetic field (e.g., similar to a current carrying wire) that induces a current in the sensor wire (e.g., the sensor 314). This information can be used to determine neural location and/or map the nerves (e.g., on a display 112) to identify the location of nerves and select target nerves (nerves with excessive parasympathetic tone) before neuromodulation therapy to ensure that the desired nerves are treated during neuromodulation therapy. EMF information can also be used during or after neuromodulation therapy so that the clinician can monitor changes in nerve firing rate to validate treatment efficacy.

In some embodiments, the system 100 can detect magnetic fields and/or EMF generated at a selected frequency that corresponds to a particular type of nerve. The frequency and, by extension, the associated nerve type of the detected signal can be selected based on an external resonant circuit. Resonance occurs on the external circuit when it is matched to the frequency of the magnetic field of the particular nerve type and that nerve is firing. In manner, the system 100 can be used to locate a particular sub-group/type of nerves.

In some embodiments, the system 100 can include a variable capacitor frequency-selective circuit to identify the location and/or map specific nerves (e.g., parasympathetic nerve, sensory nerve, nerve fiber type, nerve subgroup, etc.). The variable capacitor frequency-selective circuit can be defined by the sensor 314 and/or other feature of the end effector 114. Nerves have different resonant frequencies based on their function and structure. Accordingly, the system 100 can include a tunable LC circuit with a variable capacitor (C) and/or variable inductor (L) that can be selectively tuned to the resonant frequency of desired nerve types. This allows for the detection of neural activity only associated with the selected nerve type and its associated resonant frequency. Tuning can be achieved by moving the core in and out of the inductor. For example, tunable LC circuits can tune the inductor by: (i) changing the number of coils around the core; (ii) changing the cross-sectional area of the coils around the core; (iii) changing the length of the coil; and/or (iv) changing the permeability of the core material (e.g., changing from air to a core material). Systems including such a tunable LC circuit provide a high degree of dissemination and differentiation not only as to the activation of a nerve signal, but also with respect to the nerve type that is activated and the frequency at which the nerve is firing.

### Anatomical Mapping

In various embodiments, the system 100 is further configured to provide minimally-invasive anatomical mapping that uses focused energy current/voltage stimuli from a spatially localized source (e.g., the electrodes 136) to cause a change in the conductivity of the of the tissue at the interest zone and detect resultant biopotential and/or bioelectrical measurements (e.g., via the electrodes 136). The current density in the tissue changes in response to changes of voltage applied by the electrodes 136, which creates a change in the electric current that can be measured with the end effector 114 and/or other portions of the system 100. The results of the bioelectrical and/or biopotential measurements can be used to predict or estimate relative absorption profilometry to predict or estimate the tissue structures in the interest zone. More specifically, each cellular construct has unique conductivity and absorption profiles that can be indicative of a type of tissue or structure, such as bone, soft tissue, vessels, nerves, types of nerves, and/or certain neural structures. For example, different frequencies decay differently through different types of tissue. Accordingly, by detecting the absorption current in a region, the system 100 can determine the underlying structure and, in some instances, to a sub-microscale, cellular level that allows for highly specialized target localization and mapping. This highly specific target identification and mapping enhances the efficacy and efficiency of neuromodulation therapy, while also enhancing the safety profile of the system 100 to reduce collateral effects on non-target structures.

To detect electrical and dielectric tissue properties (e.g., resistance, complex impedance, conductivity, and/or, permittivity as a function of frequency), the electrodes 136 and/or another electrode array is placed on tissue at an interest region, and an internal or external source (e.g., the generator 106) applies stimuli (current/voltage) to the tissue. The electrical properties of the tissue between the source and the receiver electrodes 136 are measured, as well as the current and/or voltage at the individual receiver electrodes 136. These individual measurements can then be converted into an electrical map/image/profile of the tissue and visualized for the user on the display 112 to identify anatomical features of interest and, in certain embodiments, the location of firing nerves. For example, the anatomical mapping can be provided as a color-coded or gray-scale three-dimensional or two-dimensional map showing differing intensities of certain bioelectric properties (e.g., resistance, impedance, etc.), or the information can be processed to map the actual anatomical structures for the clinician. This information can also be used during neuromodulation therapy to monitor treatment progression with respect to the anatomy, and after neuromodulation therapy to validate successful treatment. In addition, the anatomical mapping provided by the bioelectrical and/or biopotential measurements can be used to track the changes to non-target tissue (e.g., vessels) due to neuromodulation therapy to avoid negative collateral effects. For example, a clinician can identify when the therapy begins to ligate a vessel and/or damage tissue, and modify the therapy to avoid bleeding, detrimental tissue ablation, and/or other negative collateral effects.

Furthermore, the threshold frequency of electric current used to identify specific targets can subsequently be used when applying therapeutic neuromodulation energy. For example, the neuromodulation energy can be applied at the specific threshold frequencies of electric current that are target neuronal-specific and differentiated from other non-targets (e.g., blood vessels, non-target nerves, etc.). Applying ablation energy at the target-specific frequency results in an electric field that creates ionic agitation in the target neural structure, which leads to differentials in osmotic potentials of the targeted neural structures. These osmotic potential differentials cause dynamic changes in neuronal membronic potentials (resulting from the difference in intra-cellular and extra-cellular fluidic pressure) that lead to vacuolar degeneration of the targeted neural structures and, eventually, necrosis. Using the highly targeted threshold neuromodulation energy to initiate the degeneration allows the system 100 to delivery therapeutic neuromodulation to the specific target, while surrounding blood vessels and other non-target structures are functionally maintained.

In some embodiments, the system 100 can further be configured to detect bioelectrical properties of tissue by non-invasively recording resistance changes during neuronal depolarization to map neural activity with electrical impedance, resistance, bio-impedance, conductivity, permittivity, and/or other bioelectrical measurements. Without being bound by theory, when a nerve depolarizes, the cell membrane resistance decreases (e.g., by approximately 80×) so that current will pass through open ion channels and into the intracellular space. Otherwise the current remains in the extracellular space. For non-invasive resistance measurements, tissue can be stimulated by applying a current of less than 100 Hz, such as applying a constant current square wave at 1 Hz with an amplitude less than 25% (e.g., 10%) of the threshold for stimulating neuronal activity, and thereby preventing or reducing the likelihood that the current does not cross into the intracellular space or stimulating at 2 Hz. In either case, the resistance and/or complex impedance is recorded by recording the voltage changes. A complex impedance or resistance map or profile of the area can then be generated.

For impedance/conductivity/permittivity detection, the electrodes 136 and/or another electrode array are placed on tissue at an interest region, and an internal or external source (e.g., the generator 106) applies stimuli to the tissue, and the current and/or voltage at the individual receiver electrodes 136 is measured. The stimuli can be applied at different frequencies to isolate different types of nerves. These individual measurements can then be converted into an electrical map/image/profile of the tissue and visualized for the user on the display 112 to identify anatomical features of interest. The neural mapping can also be used during neuromodulation therapy to select specific nerves for therapy, monitor treatment progression with respect to the nerves and other anatomy, and validate successful treatment.

In some embodiments of the neural and/or anatomical detection methods described above, the procedure can include comparing the mid-procedure physiological parameter(s) to the baseline physiological parameter(s) and/or other, previously-acquired mid-procedure physiological parameter(s) (within the same energy delivery phase). Such a comparison can be used to analyze state changes in the treated tissue. The mid-procedure physiological parameter(s) may also be compared to one or more predetermined thresholds, for example, to indicate when to stop delivering treatment energy. In some embodiments of the present technology, the measured baseline, mid-, and post-procedure parameters include a complex impedance. In some embodiments of the present technology, the post-procedure physiological parameters are measured after a pre-determined time period to allow the dissipation of the electric field effects (ionic agitation and/or thermal thresholds), thus facilitating accurate assessment of the treatment.

In some embodiments, the anatomical mapping methods described above can be used to differentiate the depth of soft tissues within the sino-nasal mucosa. The depth of mucosa on the turbinates is great whilst the depth off the turbinate is shallow and, therefore, identifying the tissue depth in the present technology also identifies positions within the sino-nasal mucosa and where precisely to target. Further, by providing the micro-scale spatial impedance mapping of epithelial tissues as described above, the inherent unique signatures of stratified layers or cellular bodies can be used as identifying the region of interest. For example, different regions have larger or small populations of specific structures, such as submucosal glands, so target regions can be identified via the identification of these structures.

In some embodiments, the system 100 includes additional features that can be used to detect anatomical structures and map anatomical features. For example, the system 100 can include an ultrasound probe for identification of neural structures and/or other anatomical structures. Higher frequency ultrasound provides higher resolution, but less depth of penetration. Accordingly, the frequency can be varied to achieve the appropriate depth and resolution for neural/anatomical localization. Functional identification may rely on the spatial pulse length ("SPL") (wavelength multiplied by number of cycles in a pulse). Axial resolution (SPL/2) may also be determined to locate nerves.

In some embodiments, the system 100 can further be configured to emit stimuli with selective parameters that suppress rather than fully stimulate neural activity. for example, in embodiments where the strength-duration relationship for extracellular neural stimulation is selected and controlled, a state exists where the extracellular current can hyperpolarize cells, resulting in suppression rather than stimulation spiking behavior (i.e., a full action potential is not achieved). Both models of ion channels, HH and RGC, suggest that it is possible to hyperpolarize cells with appropriately designed burst extracellular stimuli, rather than extending the stimuli. This phenomenon could be used to suppress rather than stimulate neural activity during any of the embodiments of neural detection and/or modulation described herein.

In various embodiments, the system 100 could apply the anatomical mapping techniques disclosed herein to locate or detect the targeted vasculature and surrounding anatomy before, during, and/or after treatment

### Incorporation by Reference

References and citations to other documents, such as patents, patent applications, patent publications, journals, books, papers, web contents, have been made throughout this disclosure. All such documents are hereby incorporated herein by reference in their entirety for all purposes.

### Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The terms and expressions which have been employed herein are used as terms of description and not of limitation, and there is no intention, in the use of such terms and expressions, of excluding any equivalents of the features shown and described (or portions thereof), and it is recognized that various modifications are possible within the scope of the claims. Accordingly, the claims are intended to cover all such equivalents.

Certain aspects and/or embodiments of the present invention may be defined by the following numbered clauses:
Clause 1. A device for treating a condition within a sino-nasal cavity of a patient, the device comprising:
   an end effector including one or more flexible printed circuit board (PCB) members for delivering energy to one or more target sites within the sino-nasal cavity of the patient.
Clause 2. The device of clause 1, wherein each of the one or more flexible PCB members comprises a PCB substrate and one or more electrodes configured to deliver energy to tissue at the one or more target sites.
Clause 3. The device of clause 2, wherein each of the one or more flexible PCB members comprises one or more electrical communication paths positioned at least on or within the PCB substrate and selectively coupling the one or more electrodes to a corresponding one or more electrical contacts configured to electrically couple the one or more electrodes to a controller.
Clause 4. The device of clause 2, wherein the end effector comprises one or more retractable and expandable segments to which the one or more flexible PCB members are operably associated with.
Clause 5. The device of clause 4, wherein the end effector comprises a first retractable and expandable segment comprising a plurality of first support structures upon which one or more flexible PCB members are fixedly coupled.
Clause 6. The device of clause 5, wherein, when the first segment is in an expanded configuration, the plurality of first support structures extend in a first direction relative to a shaft and form a first geometry.
Clause 7. The device of clause 6, wherein PCB substrate of the one or more flexible PCB members comprises a flexible material configured to correspondingly transition from a collapsed configuration to a deployed configuration upon movement of the first segment to the expanded configuration.
Clause 8. The device of clause 7, wherein each of the plurality of first support structures includes at least a portion of one or more flexible PCB members fixedly coupled thereto.
Clause 9. The device of clause 8, wherein at least a first one of the plurality of first support structures is configured in a loop-like or leaflet-like shape when the first segment is in an expanded configuration such that a PCB substrate of a flexible PCB member operably associated with the first one of the plurality of first support structures substantially covers the loop-like or leaflet-like shape in a deployed configuration.
Clause 10. The device of clause 6, wherein the first retractable and expandable segment further comprises a plurality of second support structures upon which one or more flexible PCB members are fixedly coupled, wherein, when the first segment is in an expanded configuration, the plurality of second support structures extend in a second, opposing direction relative to the shaft of the device and form a second geometry.
Clause 11. The device of clause 10, wherein each of the first and second geometries comprises a concave shape.
Clause 12. The device of clause 11, wherein the plurality of first support structures comprises at least a first pair of support elements that cooperatively form the concave shape relative to the shaft in the first direction when in the expanded configuration and the plurality of second support structures comprises at least a second pair of support elements the cooperatively form the concave shape relative to the shaft in the second direction when in the expanded configuration.
Clause 13. The device of clause 10, wherein the plurality of first and second support structures comprise deformable wires and/or struts.
Clause 14. The device of clause 13, wherein the deformable wires and/or struts comprise shape memory material.
Clause 15. The device of clause 14, wherein the one or more flexible PCB members are fixedly coupled to one or more deformable wires and/or struts via an adhesive.
Clause 16. The device of clause 6, wherein the end effector comprises a second retractable and expandable segment to which one or more flexible PCB members are operably associated with.
Clause The device of clause 16, wherein the second segment comprises a plurality of third support structures upon which one or more flexible PCB members are fixedly coupled, wherein, when the second segment is in an expanded configuration, the plurality of third support structures extend in a third direction relative to the shaft and cooperatively form an open-ended circumferential shape.
Clause 18. The device of clause 2, wherein the one or more electrodes are configured to deliver radiofrequency (RF) energy.
Clause 19. The device of clause 2, wherein one or more flexible PCB members comprise a first subset of a plurality of elements provided on the PCB substrate and configured to deliver non-therapeutic stimulating energy to tissue at the one or more target sites at a frequency for locating target tissue and non-target tissue.
Clause 20. The device of clause 19, wherein one or more flexible PCB members comprise a second subset of a plurality of elements provided on the PCB substrate and configured to sense properties of at least one of the target tissue and non-target tissue in response to the non-therapeutic stimulating energy.
Clause 21. A method for treating a condition within a sino-nasal cavity of a patient, the method comprising:
   providing a treatment device comprising an end effector including one or more flexible printed circuit board (PCB) members;
   advancing the end effector through a nasal passage and into a sino-nasal cavity of a patient until the one or more flexible PCB members are positioned at one or more target sites; and
   delivering energy, via the one or more flexible PCB members, to tissue at the one or more target sites.
Clause 22. The method of clause 21, wherein each of the one or more flexible PCB members comprises a PCB substrate and one or more electrodes configured to deliver energy to tissue at the one or more target sites.
Clause 23. The method of clause 22, wherein each of the one or more flexible PCB members comprises one or more electrical communication paths positioned at least on or within the PCB substrate and selectively coupling the one or more electrodes to a corresponding one or more electrical contacts configured to electrically couple the one or more electrodes to a controller.
Clause 24. The method of clause 22, wherein the end effector comprises one or more retractable and expandable segments to which the one or more flexible PCB members are operably associated with.
Clause 25. The method of clause 24, wherein the end effector comprises a first retractable and expandable segment comprising a plurality of first support structures upon which one or more flexible PCB members are fixedly coupled.
Clause 26. The method of clause 25, further comprising transitioning the first segment from a retracted configuration to an expanded configuration, thereby extending the plurality of first support structures in a first direction relative to a shaft and forming a first geometry.
Clause 27. The method of clause 26, wherein PCB substrate of the one or more flexible PCB members comprises a flexible material configured to correspondingly transition from a collapsed configuration to a deployed configuration upon movement of the first segment to the expanded configuration.
Clause 28. The method of clause 27, wherein each of the plurality of first support structures includes at least a portion of one or more flexible PCB members fixedly coupled thereto.
Clause 29. The method of clause 28, wherein at least a first one of the plurality of first support structures is configured in a loop-like or leaflet-like shape when the first segment is in an expanded configuration such that a PCB substrate of a flexible PCB member operably associated with the first one of the plurality of first support structures substantially covers the loop-like or leaflet-like shape in a deployed configuration.
Clause 30. The method of clause 26, wherein the first retractable and expandable segment further comprises a plurality of second support structures upon which one or more flexible PCB members are fixedly coupled, wherein, when the first segment is in an expanded configuration, the plurality of second support structures extend in a second, opposing direction relative to the shaft of the device and form a second geometry.
Clause 31. The method of clause 30, wherein each of the first and second geometries comprises a concave shape.
Clause The method of clause 31, wherein the plurality of first support structures comprises at least a first pair of support elements that cooperatively form the concave shape relative to the shaft in the first direction when in the expanded configuration and the plurality of second support structures comprises at least a second pair of support elements the cooperatively form the concave shape relative to the shaft in the second direction when in the expanded configuration.
Clause 33. The method of clause 30, wherein the plurality of first and second support structures comprise deformable wires and/or struts.
Clause 34. The method of clause 33, wherein the deformable wires and/or struts comprise shape memory material.
Clause 35. The method of clause 34, wherein the one or more flexible PCB members are fixedly coupled to one or more deformable wires via an adhesive.
Clause 36. The method of clause 26, wherein the end effector comprises a second retractable and expandable segment to which one or more flexible PCB members are operably associated with.
Clause 37. The method of clause 36, wherein the second segment comprises a plurality of third support structures upon which one or more flexible PCB members are fixedly coupled, wherein, when the second segment is in an expanded configuration, the plurality of third support structures extend in a third direction relative to the shaft and cooperatively form an open-ended circumferential shape.
Clause 38. The method of clause 22, wherein the one or more electrodes are configured to deliver radiofrequency (RF) energy.
Clause 39. The method of clause 32, wherein one or more flexible PCB members comprise a first subset of a plurality of elements provided on the PCB substrate and configured to deliver non-therapeutic stimulating energy to tissue at the one or more target sites at a frequency for locating target tissue and non-target tissue.
Clause 40. The method of clause 39, wherein one or more flexible PCB members comprise a second subset of a plurality of elements provided on the PCB substrate and configured to sense properties of at least one of the target tissue and non-target tissue in response to the non-therapeutic stimulating energy.
Clause 41. A device for treating a condition in a nasal cavity, the device comprising:
   an end effector dimensioned for insertion into a nasal cavity, the end effector comprising at least one segment that is a unitary single piece of material comprising a plurality of individual struts; and
   a flexible printed circuit board (PCB) member attached to at least one of the struts.
Clause 42. The device of clause 41, wherein the end effector comprises at least two segments including a distal segment and a proximal segment.
Clause 43. The device of clause 42, wherein the distal segment comprises a proximal end configured to be received by at least a portion of the proximal segment.
Clause 44. The device of clause 42, wherein the proximal segment comprises at least one pair of struts, each of the struts comprising a first end and a second end that are each connected to a portion of the proximal segment.
Clause 45. The device of clause 42, wherein the distal segment comprises struts that are deployable into a coaxial configuration with respect to a longitudinal axis of the device.
Clause 46. The device of clause 41, wherein the plurality of individual struts are transformable between a retracted configuration and a deployed configuration.
Clause 47. The device of clause 46, wherein, at least when in the deployed configuration, at least a portion of the plurality of individual struts are in a coaxial configuration with respect to a longitudinal axis of the device.
Clause 48. The device of clause 46, wherein, at least when in the deployed configuration, at least a portion of the plurality of individual struts are in an annular configuration with respect to a longitudinal axis of the device.
Clause 49. The device of clause 41, wherein at least a portion of the plurality of individual struts comprises a free distal end.
Clause 50. The device of clause 41, wherein at least a portion of the plurality of individual struts comprise one or more articulation sites that improve flexibility of the struts.
Clause 51. The device of clause 50, wherein the one or more articulation sites comprise an area of reduced material.
Clause 52. The device of clause 51, wherein the area of reduced material forms an S-shaped configuration.
Clause 53. The device of clause 41, wherein a portion of the plurality of individual struts are arranged around a circumference of a distal end of the end effector.
Clause 54. The device of clause 53, wherein at least one of the plurality of individual struts comprises a stiffness that is different than a stiffness of a second strut.
Clause 55. The device of clause 41, wherein the plurality of individual struts comprise at least two substantially flat faces opposite of one another.
Clause 56. The device of clause 41, wherein each of the plurality of struts comprises a corresponding flexible PCB member.
Clause 57. The device of clause 41, wherein, the device further comprises a soft polymer disposed between the flexible PCB member and the at least one of the plurality of individual struts.
Clause 58. The device of clause 41, wherein the at least one segment comprises a distal portion and a proximal portion, wherein each of the distal portion and the proximal portion comprise at least one strut.
Clause 59. The device of clause 41, wherein the flexible PCB member is configured to deliver energy to one or more target sites within the sino-nasal cavity.
Clause 60. The device of clause 41, wherein at least a portion of the plurality of individual struts comprise connectors that connect different ones of the struts.
Clause 61. A method comprising:
   providing an end effector dimensioned to be at least partially deployed inside a sino-nasal cavity of a patient; and
   attaching a flexible printed circuit board (PCB) member to the end effector, the flexible PCB configured to deliver energy to a target site within the sino-nasal cavity.
Clause 62. The method of clause 61, wherein the end effector comprises one or more deployable struts to which the flexible PCB member is attached.
Clause 63. The method of clause 62, wherein at least a portion of the deployable struts comprise a free distal end.
Clause 64. The method of clause 61, wherein attaching involves at least one of a thermal process and mechanical process.
Clause 65. The method of clause 64, wherein the thermal process comprises at least one of a reflow process, an induction heating process, a spot welding process, a lamination process, and a laser welding process.
Clause 66. The method of clause 65, wherein the reflow process comprises polymer reflow.
Clause 67. The method of clause 64, wherein the thermal process comprises:
   positioning the flexible PCB member on the effector;
   disposing one or more polymer layers around the flexible PCB member; and applying heat.
Clause 68. The method of clause 61, wherein, a polymer sleeve is secured, via polymer reform, to a portion of the end effector to thereby provide a substrate onto which the flexible PCB member is attached.
Clause 69. The method of clause 61, wherein the flexible PCB member is machined from a single sheet of flexible PCB material.
Clause 70. The method of clause 61, wherein a portion of the end effector comprises one or more fixation points to facilitate the attachment of at least one of a polymer sleeve and the flexible PCB to the end effector.
Clause 71. The method of clause 70, wherein the one or more fixation points comprise a recess, a hole, a notch, or a groove, etched into the end effector.
Clause 72. The method of clause 70, wherein the one or more fixation points are disposed at a distal portion of the end effector.
Clause 73. The method of clause 61, wherein at least a portion of the flexible PCB member is attached to the end effector by an adhesive.
Clause 74. The method of clause 73, wherein the adhesive is applied to one or more fixation points disposed on the end effector prior to attaching the flexible PCB member.
Clause 75. The method of clause 62, wherein the deployable struts comprise opposing surfaces and the flexible member is attached to the opposing surfaces.
Clause 76. A method comprising:
   providing a single piece of metal; and
   cutting the single piece of metal to form an end effector dimensioned for insertion into a sino-nasal cavity of a subject.
Clause 77. The method of clause 76, wherein the single piece of metal comprises at least one of a tube and a plate.
Clause 78. The method of clause 76, wherein the end effector comprises one or more deployable struts, at least a portion of the deployable struts comprising a free distal end.
Clause 79. The method of clause 78, wherein a proximal portion of the at least a portion of the struts comprises one or more articulation sites that facilitate flexibility.
Clause 80. The method of clause 16, wherein cutting involves laser machining.
Clause 81. A medical device comprising:
   an end effector dimensioned for insertion into a nasal cavity, the end effector comprising a plurality of struts extending from at least a distal end of the effector in a radial configuration, wherein at least two of the struts are connected by a cross member.
Clause 82. The device of clause 81, wherein the cross member comprises a flexible printed circuit board (PCB).
Clause 83. The device of clause 81, wherein the cross member connects two immediately adjacent struts.
Clause 84. The device of clause 81, wherein the plurality of struts are connected by a plurality of cross members.
Clause 85. The device of clause 84, wherein substantially every other one of the plurality of the struts are connected by a corresponding cross member.
Clause 86. The device of clause 81, wherein the plurality of struts are connected by a plurality of cross members in non-uniform locations along a length of the plurality of struts.
Clause 87. The device of clause 81, wherein the cross member comprises one or more electrodes.
Clause 88. The device of clause 81, wherein the plurality of struts and the cross member are transformable between a retracted configuration and a deployed configuration.
Clause 89. The device of clause 88, wherein, when in a deployed configuration, the cross member achieves a locked state inhibiting retraction of the plurality of struts.
Clause 90. The device of clause 81, wherein the cross member comprises an articulation site.
Clause 91. The device of clause 81, wherein the cross member comprises an arcuate shape.
Clause 92. The device of clause 81, wherein the cross member comprises at least one of an S-shape and a chevron shape.
Clause 93. The device of clause 81, wherein the cross member is configured to influence at least one of a radial stiffness and a lateral stiffness of the at least two struts.
Clause 94. The device of clause 81, wherein each one of the plurality of struts comprises flexible PCB member configured to deliver energy to one or more target sites within the nasal cavity.
Clause 95. The device of clause 94, wherein at least two of the flexible PCB members are configured to deliver different energy profiles from each other.

## Claims

1. A system for treating a condition within a sino-nasal cavity of a patient, the system comprising:
a treatment device comprising an end effector dimensioned for insertion into a sino-nasal cavity of the patient, the end effector comprising a retractable and expandable segment arranged at a distal portion of a shaft of the treatment device, the retractable and expandable segment comprising:
a framework of a plurality of loop-shaped support structures transformable between a low-profile delivery state and an expanded state, wherein, when in the expanded state, the plurality of loop-shaped support structures cooperatively form an open-ended circumferential shape, wherein each loop-shaped support structure is defined by a pair of immediately adjacent flexible struts joined to one another via a polymer tubing placed over respective distal ends of each flexible strut; and
a flexible printed circuit board (PCB) member fixedly coupled to each flexible strut of a pair of flexible struts of a loop-shaped support structure, wherein each flexible PCB member comprises a PCB substrate and one or more pairs of bipolar electrodes positioned along a length thereof for delivering energy to one or more target sites within the sino-nasal cavity of the patient; and
a controller operably coupled to the treatment device and configured to control operation thereof.

2. The system of claim 1, wherein each flexible PCB member comprises one or more electrical communication paths positioned at least on or within the PCB substrate and selectively coupling the one or more pairs of bipolar electrodes to a corresponding one or more electrical contacts configured to electrically couple the one or more pairs of bipolar electrodes to the controller.

3. The system of claim 1, wherein the PCB substrate comprises a flexible material configured to correspondingly transition from a collapsed configuration to a deployed configuration upon movement of the segment to the expanded state.

4. The system of claim 1, wherein each flexible strut comprises a deformable wire.

5. The system of claim 4, wherein the deformable wire comprises a shape memory material.

6. The system of claim 1, wherein each flexible PCB member is fixedly coupled to each flexible strut via an adhesive.

7. The system of claim 1, wherein the shaft of the treatment device is bendable.

8. The system of claim 1, wherein the treatment device further comprises a handle with which an operator can manipulate the end effector and control emission of RF energy therefrom.

9. The system of claim 1, wherein the retractable and expandable segment comprises at least four loop-shaped support structures.

10. The system of claim 1, wherein at least one flexible PCB member comprises a first subset of a plurality of elements provided on the PCB substrate and configured to deliver non-therapeutic stimulating energy to tissue at the one or more target sites at a frequency for locating target tissue and non-target tissue.

11. The system of claim 10, wherein the at least one flexible PCB member comprises a second subset of a plurality of elements provided on the PCB substrate and configured to sense properties of at least one of the target tissue and non-target tissue in response to the non-therapeutic stimulating energy.

12. The system of claim 1, wherein the controller is configured to:
control emission of RF energy from at least one pair of bipolar electrodes for the detection of one or more properties of tissue at the target site; and
control delivery of a plurality of treatment applications comprising emission of RF energy from at least one pair of bipolar electrodes for altering transmission of signals through the target tissue at the one or more target sites.

13. The system of claim 1, further comprising a console unit operably coupled to the treatment device via the controller.

14. The system of claim 13, wherein the console unit comprises an energy generator configured to generate RF energy to be delivered by at least one pair of bipolar electrodes.

15. The system of claim 13, wherein the console unit comprises a user interface to control, monitor, and regulate RF energy delivery to tissue by the treatment device.
